# EUROPEAN PATENT APPLICATION

(11) **EP 4 406 966 A1**
(43) Date of publication of application: **31.07.2024**
(21) Application number: 22873219.4
(22) Date of filing: 23.09.2022
(51) Int. Cl.: C07K 14/705, C07K 14/54, C12N 15/62, A61K 38/00, A61P 35/00

(54) **FUSION PROTEIN DIMER INCLUDING PD-1 AND IL-21, AND USE THEREOF**

(30) Priority: 24.09.2021 KR 20210126052
(71) Applicant: Bionsystems Inc, Uiwang-si, Gyeonggi-do 16006 (KR)
(72) Inventor: SUH, Jung-Keun, Daejeon 35213 (KR)
(74) Representative: HGF
(86) International application number: PCT/KR2022/014297
(87) International publication number: WO 2023/048516

(57) **Abstract**

The present invention relates to a fusion protein comprising a PD-1 protein and an IL-21 protein; and a use thereof. In one embodiment, a fusion protein comprising PD-1, IL-21, and prolonged serum persistent Fc may activate immune cells such as NK cells, and effectively increase *in vivo* half-life. Therefore, a pharmaceutical composition comprising the fusion protein as an active ingredient enhances immune activity in the body and thus may be used as an anti-cancer agent for recurrent patients and patients who do not respond to immune checkpoint inhibitor treatment.

## Description

### Technical Field

The present invention relates to a fusion protein comprising a PD-1 protein and an IL-21 protein; and a use thereof. Specifically, the present invention relates to a novel fusion protein dimer comprising PD-1, IL-21, and prolonged serum persistent Fc, which has an increased half-life in the blood and immune-boosting efficacy, and a use thereof for cancer treatment.

### Background Art

PD-1 (programmed death receptor-1) is an immune checkpoint that has recently been in the spotlight. It is mainly involved in controlling the activation of T cells and can regulate the intensity and duration of immune responses. Under normal circumstances, PD-1 mediates and maintains autoimmune tolerance of the body's tissues and prevents the immune system from being overactivated and damaging autologous tissues during the inflammatory response, thereby preventing the development of autoimmune diseases to have a positive effect. However, in pathological situations, PD-1 is known to be involved in the process of tumor immunity and the occurrence and development of various autoimmune diseases (Sara Pilotto et al., Anti-cancer Agents Med Chem., 15(3):307-313, 2015).

PD-1 is mainly expressed on the surface of activated T cells, but is also expressed on B cells, NK cells, mononuclear cells, and dendritic cells (DC). PD-L1 and PD-L2, which are ligands of PD-1, are expressed on tumor cells, activated B and T cells, dendritic cells, and macrophages. PD-1 binds to these ligands and induces T cell apoptosis, thereby weakening the immune response of the cell. Accordingly, blocking the PD-1/PD-L1 or PD-L2 pathway is a promising therapeutic approach that is being explored in research on many types of cancer (Miguel F. Sanmamed et al., Cancer J., 20(4):256-261, 2014).

Meanwhile, IL-21 (Interleukin-21) is a cytokine secreted by activated CD4+ T cells, and has been reported to induce the maturation of NK cell precursors from the bone marrow, and especially increase effector functions of NK cells, such as the cytokine production ability and apoptosis ability. In addition, it is known to promote the anti-cancer response of the immune system by increasing the effector functions of CD8+ T cells.

Through the interaction of IL-21 and its receptor, IL-21R, the Jak/STAT signaling pathway is activated, thereby inducing strong and continuous activation of STAT3, which is important for T cell differentiation (Wenjun Ouyang et al., Immunity, 28(4):454-467, 2008).

Meanwhile, the CH2-CH3 portion of immunoglobulin Fc has an FcRn (protection receptor) binding site that prolongs the half-life of the antibody (Korean Patent No. 10-1957431). FcRn is an MHC class I-related protein expressed in vascular endothelial cells and binds to IgG and albumin. An antibody fragment without Fc, that is, without an FcRn binding site, has the half-life in the human body of about 2 to 3 hours, but IgG1, IgG2, and IgG4 with an FcRn binding site have the half-life in the human body of on average 3 weeks, which is longer than that of other proteins.

### Detailed Description of Invention

### Technical Problem

Accordingly, the present inventor has studied to develop an anti-cancer therapeutic agent with increased *in vivo* half-life and excellent immune-boosting efficacy. As a result, the present inventors have discovered that a novel fusion protein comprising PD-1, IL-21, and prolonged serum persistent Fc may activate immune cells and and increase half-life without affecting anti-cancer activity, thereby completing the present invention.

### Solution to Problem

In order to achieve the above object, in one aspect of the present invention, there is provided a fusion protein comprising a PD-1 protein and an IL-21 protein.

In another aspect of the present invention, there is provided a fusion protein dimer in which the two fusion proteins are attached to each other.

In yet another aspect of the present invention, there is provided a polynucleotide encoding the fusion protein.

In still yet another aspect of the present invention, there is provided a vector comprising the polynucleotide.

In still yet another aspect of the present invention, there is provided a host cell transformed with the vector.

In still yet another aspect of the present invention, there is provided a pharmaceutical composition for preventing or treating cancer, comprising the fusion protein or the fusion protein dimer as an active ingredient.

In still yet another aspect of the present invention, there is provided a use of the fusion protein for treatment of cancer.

### Effects of Invention

A fusion protein comprising PD-1, IL-21, and prolonged serum persistent Fc can not only activate immune cells by PD-1 and IL-21, but can also maximize *in vivo* half-life by prolonged serum persistent Fc. Therefore, the fusion protein may efficiently attack cancer cells and thus may be usefully used in the treatment of cancers.

### Brief Description of Drawings

Figure 1 illustrates a result obtained by identifying the obtained fusion protein (BNS001; PD-1 D-Fc(29)-IL-21, PD-1 D-Fc(41)-IL-21, and PD-1 D-Fc(wt)-IL-21) by SDS-PAGE.
Figure 2 illustrates a result obtained by analyzing the purity of the obtained fusion protein (BNS001) by SEC-HPLC analysis.
Figure 3 illustrates a result obtained by identifying the concentration of the obtained fusion protein (BNS001) by a UV spectrophotometer.
Figure 4 illustrates a result obtained by identifying the thermodynamic stability of the obtained fusion protein (BNS001) by fluorescence and SLS (static light scattering) detectors.
Figure 5 illustrates a result obtained by identifying the structural stability of the obtained fusion protein (BNS001) by a FT-IR (Fourier-transform infrared spectroscopy) spectrophotometer.
Figure 6 illustrates a result obtained by identifying the molecular weight of the obtained fusion protein (BNS001) by MALDI-TOF mass spectrometry.
Figure 7 illustrates a result obtained by identifying the binding affinity between the obtained fusion protein (BNS001) and hPD-L1 (human programmed cell death ligand-1).
Figure 8 illustrates a result obtained by identifying the binding affinity between a hPD-1 protein (human programmed cell death protein-1) and hPD-L1.
Figure 9 illustrates a result obtained by identifying the binding affinity between the obtained fusion protein (BNS001) and hPD-L2 (human programmed cell death ligand-2).
Figure 10 illustrates a result obtained by identifying the binding affinity between a hPD-1 protein and hPD-L2.
Figure 11 illustrates a result obtained by identifying the binding affinity between the obtained fusion protein (BNS001) and a hIL-21 receptor (human Interleukin-21 receptor).
Figure 12 illustrates a result obtained by identifying the binding affinity between a hIL-21 protein and a hIL-21 receptor.
Figure 13 illustrates a result obtained by identifying the binding affinity between the obtained fusion protein (BNS001) and a hFcRn receptor (human neonatal Fc receptor).
Figure 14 illustrates a result obtained by identifying the binding affinity between the obtained fusion protein (BNS001) and PD-L1 through ELISA analysis.
Figure 15 illustrates a result obtained by identifying the binding affinity between the obtained fusion protein (BNS001) and PD-L2 through ELISA analysis.
Figure 16 illustrates a result obtained by identifying the binding affinity between the obtained fusion protein (BNS001) and an IL-21 receptor through ELISA analysis.
Figure 17 illustrates a result obtained by identifying the binding affinity between the obtained fusion protein (BNS001) and an FcRn receptor through LUMIT^{™} FcRn binding immunoassay analysis.
Figure 18 illustrates a result obtained by identifying the binding affinity between the obtained fusion protein (BNS001) and an IL-21 receptor through PATHHUNTER^{®} eXpress Dimerization Assay analysis.
Figure 19 illustrates a result obtained by measuring the amount of IFN-γ secreted from PBMC containing immune cells through ELISA analysis when the peripheral blood mononuclear cells (PBMC) were subjected to treatment with the obtained fusion protein (BNS001) and IL-21, respectively, and incubation is performed.
Figure 20 illustrates a result obtained by identifying the effect of the obtained fusion protein (BNS001) and an IL-21 protein on the proliferation of CD8+ T cells through FACS analysis.
Figure 21 illustrates a result obtained by identifying the effect of the obtained fusion protein (BNS001) and an IL-21 protein on the proliferation of CD4+ T cells through FACS analysis.
Figure 22a illustrates a result obtained by identifying the effect of the obtained fusion protein (BNS001) and an IL-21 protein on the proliferation of NK cells through FACS analysis.
Figure 22b is a graph showing a result obtained by identifying the effect of the obtained fusion protein (BNS001) and an IL-21 protein on the proliferation of NK cells through FACS analysis.
Figure 23 illustrates a result obtained by identifying the effect of the obtained fusion protein (BNS001) on effector T cells through PD-L1/PD-1 blockade assay.
Figure 24 illustrates a result obtained by identifying the effect of the obtained fusion protein (BNS001) on effector T cells through PD-L2/PD-1 blockade assay.
Figure 25 illustrates a schematic diagram of the the lentivirus vector map used to construct cell lines expressing firefly luciferase and GFP (Green fluorescent protein). Here, CMV is a promoter derived from cytomegalovirus, and RSV is a promoter derived from respiratory syncytial virus.
Figure 26 illustrates a result obtained by measuring the luminescence signal (left) and fluorescence signal (right) of the human melanoma cell line (A375-Luc-GFP) expressing firefly luciferase and GFP established in the present invention.
Figure 27 illustrates a result obtained by identifying the expression pattern of PD-L1 and PD-L2 genes specifically expressed in the human melanoma cell line (A375-Luc-GFP) according to one embodiment of the present invention (left); and the expression pattern of the PD-1 gene specifically expressed in PBMC containing immune cells (right) through reverse transcription PCR.
Figure 28 illustrates a result obtained by measuring the amount of IFN-γ secreted from cells through ELISA analysis when the co-cultured human melanoma cell line (A375-Luc-GFP) and peripheral blood mononuclear cells (PBMC) were subjected to treatment with the obtained fusion protein (BNS001) and IL-21 at respective concentrations, respectively, and incubation is performed.
Figure 29 illustrates a result obtained by analyzing the cancer cell-killing effect by treatment time through measurement of luciferase activity when the co-cultured human melanoma cell line (A375-Luc-GFP) and peripheral blood mononuclear cells (PBMC) were subjected to treatment with the obtained fusion protein (BNS001).
Figure 30 illustrates a result obtained by identifying the cancer cell-killing effect through measurement of luciferase activity when the co-cultured human melanoma cell line (A375-Luc-GFP) and peripheral blood mononuclear cells (PBMC) were subjected to treatment with the obtained fusion protein (BNS001) for 48 hours.
Figure 31 illustrates a result obtained by analyzing the cancer cell-killing effect for each treatment concentration through measurement of luciferase activity when the co-cultured human melanoma cell line (A375-Luc-GFP) and peripheral blood mononuclear cells (PBMC) were subjected to treatment with the obtained fusion protein (BNS001) at respective concentrations.
Figure 32 illustrates a result obtained by identifying the antibody dependent cellular cytotoxicity (ADCC) activity of the obtained fusion protein (BNS001) specific to a cancer cell line (A375-Luc-GFP).
Figure 33 illustrates a result obtained by measuring the blood concentration of the obtained fusion protein (BNS001) through ELISA analysis.
Figure 34 illustrates a result obtained by identifying the blood half-life of the fusion protein (BNS001) in a normal BALB/c mouse animal model.
Figure 35 illustrates a result obtained by identifying the blood half-life of the fusion protein (BNS001) in a human FcRn TG mouse animal model.
Figure 36 illustrates a schematic diagram of the administration and experiment schedule for identifying the anti-cancer effect of the fusion protein (BNS001) in mice transplanted with mouse-derived colorectal cancer cells.
Figure 37 illustrates a result obtained by identifying the tumor inhibitory effect by the fusion protein (BNS001) in mice transplanted with mouse-derived colorectal cancer cells.
Figure 38 illustrates a result obtained by identifying the statistical significance of the tumor inhibitory effect by the fusion protein (BNS001) in mice transplanted with mouse-derived colorectal cancer cells.
Figure 39 illustrates a result obtained by identifying the level of immune cell activity in cancer tissue by the obtained fusion protein (BNS001). Specifically, it is a graph showing a result obtained by analyzing the level of activity of macrophages, dendritic cells (DC), CD4+ cells, CD8+ cells, and NK cells in the cancer tissue extracted on day 35 after treating the mice transplanted with mouse-derived colorectal cancer cells with the fusion protein (BNS001) by FACS.
Figure 40 illustrates a schematic diagram of the administration and experiment schedule for identifying the anti-cancer effect of the fusion protein (BNS001) in humanized mice transplanted with human-derived lung cancer cells (H460).
Figure 41 illustrates a result obtained by identifying the tumor inhibitory effect by administration of the fusion protein (BNS001) in humanized mice transplanted with human-derived lung cancer cells (H460).
Figure 42 illustrates a result obtained by identifying the statistical significance of the tumor inhibitory effect by administration of the fusion protein (BNS001) in humanized mice transplanted with human-derived lung cancer cells (H460).
Figure 43 illustrates a schematic diagram of the administration and experiment schedule for identifying the anti-cancer effect of the fusion protein (mBNS001) in mice allografted with mouse-derived colorectal cancer cells (MC38).
Figure 44 illustrates a result obtained by identifying the concentration-dependent tumor inhibitory effect by administration of the fusion protein (mBNS001) in mice allografted with mouse-derived colorectal cancer cells (MC38).
Figure 45 illustrates the concentration-dependent tumor inhibitory effect by administration of the fusion protein (mBNS001) in mice allografted with mouse-derived colorectal cancer cells (MC38), calculated as tumor growth inhibition rate (%).
Figure 46 illustrates a schematic diagram of the administration and experiment schedule for evaluating the tumor growth inhibition effectiveness of the fusion protein (mBNS001) in mice allografted with mouse-derived colorectal cancer cells (CT-26).
Figure 47 illustrates a result obtained by evaluating the tumor growth inhibition effectiveness of the fusion protein (mBNS001) in mice allografted with mouse-derived colorectal cancer cells (CT-26) in the form of a plot for each group (G1 to G8) and each subject (indicated by subject number).
Figure 48 illustrates a result obtained by evaluating the tumor growth inhibition effectiveness by administration of the fusion protein (mBNS001) in mice allografted with mouse-derived colorectal cancer cells (CT-26), which shows a result obtained by measuring the tumor volume for each group (G1 to G8).
Figure 49 illustrates a result obtained by evaluating the tumor growth inhibition effectiveness by administration of the fusion protein (mBNS001) in mice allografted with mouse-derived colorectal cancer cells (CT-26), which shows the statistical significance between BNS001 (mPD1-Fc-IL-21) and commercially available antibodies anti-PD1 Ab (Keytruda, MSD) and anti-PD-L1 Ab (Tecentriq, Roche). Here, * is p ≤ 0.05, and *** is p ≤ 0.001.
Figure 50 illustrates a result obtained by evaluating the tumor growth inhibition effectiveness by administration of the fusion protein (mBNS001) in mice allografted with mouse-derived colorectal cancer cells (CT-26), which shows the statistical significance between BNS001 (mPD1-Fc-IL-21) and the control (PD1-Fc, Fc-IL21, PD1-Fc + Fc-IL21). Here, * is p ≤ 0.05, and *** is p ≤ 0.001.
Figure 51 illustrates a schematic diagram of the administration and experiment schedule for evaluating the tumor growth inhibition effectiveness of the fusion protein (mBNS001) in mice allografted with mouse-derived breast cancer cells (EMT-6).
Figure 52 illustrates a result obtained by evaluating the tumor growth inhibition effectiveness of the fusion protein (mBNS001) in mice allografted with mouse-derived breast cancer cells (EMT-6) in the form of a plot for each group (G1 to G9) and each subject (indicated by subject number).
Figure 53 illustrates a result obtained by evaluating the tumor growth inhibition effectiveness by administration of the fusion protein (mBNS001) in mice allografted with mouse-derived breast cancer cells (EMT-6), which shows a result obtained by measuring the tumor volume for each group (G1 to G9).
Figure 54 illustrates a result obtained by evaluating the tumor growth inhibition effectiveness by administration of the fusion protein (mBNS001; mPD1-Fc-mIL-21) and commercially available antibodies anti-PD1 Ab (Keytruda, MSD) and anti-PD-L1 Ab (Tecentriq, Roche) in mice allografted with mouse-derived breast cancer cells (EMT-6).
Figure 55 illustrates a result obtained by evaluating the tumor growth inhibition effectiveness by administration of the fusion protein (mBNS001; mPD1-Fc-mIL-21) and the control (PD1-Fc, Fc-IL21, PD1-Fc + Fc-IL21) sample in mice allografted with mouse-derived breast cancer cells (EMT-6).
Figure 56 illustrates a result obtained by measuring the level of IFN-γ secretion induced by BNS001 treatment through ELISA analysis when human PBMCs obtained from a healthy donor (donor 1) were stimulated by treatment with PHA.
Figure 57 illustrates a result obtained by measuring the level of IFN-γ secretion induced by BNS001 treatment for each concentration through ELISA analysis when human PBMCs obtained from a healthy donor (donor 1) were stimulated by treatment with PHA.
Figure 58 illustrates a result obtained by measuring the level of IFN-γ secretion induced by BNS001 treatment for each concentration through ELISA analysis when human PBMCs obtained from a healthy donor (donor 2) were stimulated by treatment with PHA.
Figure 59 illustrates a result obtained by measuring the level of IFN-γ secretion induced by BNS001 treatment for each concentration through ELISA analysis when human PBMCs obtained from a healthy donor (donor 3) were stimulated by treatment with PHA.
Figure 60 illustrates the level of activation of NK cells induced by BNS001 treatment through FACS analysis for the expression levels of the immune activation markers CD69, CD38, and 4-1BB when human PBMCs obtained from a healthy donor (donor 1) were stimulated by treatment with PHA.
Figure 61 illustrates the level of activation of CD8+ T cells induced by BNS001 treatment through FACS analysis for the expression levels of the immune activation markers CD69 and 2B4 when human PBMCs obtained from a healthy donor (donor 1) were stimulated by treatment with PHA.
Figure 62 illustrates the level of activation of CD4+ T cells induced by BNS001 treatment through FACS analysis for the expression level of the immune activation marker CD69 when human PBMCs obtained from a healthy donor (donor 1) were stimulated by treatment with PHA.
Figure 63 illustrates the level of activation of B cells induced by BNS001 treatment through FACS analysis for the expression level of the immune activation marker CD69 when human PBMCs obtained from a healthy donor (donor 1) were stimulated by treatment with PHA.
Figure 64 illustrates the level of activation of NK cells, CD8+ T cells, and CD4+ T cells each induced by BNS001 treatment for each concentration through FACS analysis for the expression levels of the immune activation markers CD69 and CD38 when human PBMCs obtained from a healthy donor (donor 1) were stimulated by treatment with PHA.
Figures 65a and 65b illustrate the level of activation of NK cells, CD8+ T cells, CD4+ T cells, and B cells each induced by BNS001 treatment for each concentration through FACS analysis for the expression levels of the immune activation markers CD69, 2B4, and OX40 when human PBMCs obtained from a healthy donor (donor 2) were stimulated by treatment with PHA.
Figure 66 illustrates the level of activation of NK cells, CD8+ T cells, CD4+ T cells, and B cells each induced by BNS001 treatment for each concentration through FACS analysis for the expression level of the immune activation marker CD69 when human PBMCs obtained from a healthy donor (donor 3) were stimulated by treatment with PHA.
Figure 67 illustrates a result obtained by identifying the level of proliferation of CD8+ T cells by the BNS001 fusion protein treatment in PBMCs through FACS analysis.
Figure 68 illustrates a result obtained by identifying the level of proliferation of CD8+ T cells by the BNS001 fusion protein treatment for each concentration in PBMCs through FACS analysis.
Figure 69 illustrates a schematic diagram of the administration and experiment schedule for identifying the anti-cancer effect of the fusion protein (BNS001) in humanized mice transplanted with human-derived colorectal cancer cells (HCT116).
Figure 70 illustrates a result obtained by identifying the concentration-dependent tumor inhibitory effect by administration of the fusion protein (BNS001) in humanized mice transplanted with human-derived colorectal cancer cells (HCT116).
Figure 71 illustrates a result obtained by identifying the degree of body weight change according to administration of the fusion protein (BNS001) for each concentration in humanized mice transplanted with human-derived colorectal cancer cells (HCT116).
Figure 72 illustrates a schematic diagram of the administration and experiment schedule for identifying the anti-cancer effect between the fusion protein (BNS001) and the control drug in humanized mice transplanted with human-derived colorectal cancer cells (HCT116).
Figure 73 illustrates a result obtained by comparing the tumor inhibitory effect by administration of the fusion protein (BNS001; PD-1 D-Fc-IL-21) with the tumor inhibitory effect by administration of the BNS001D (PD-1 D-Fc) fusion protein and the BNS001I (Fc-IL-21) fusion protein, which are comparative controls, alone or in combination in humanized mice transplanted with human-derived colorectal cancer cells (HCT116).
Figure 74 illustrates a result obtained by comparing the degree of body weight change by administration of the fusion protein (BNS001; PD-1 D-Fc-IL-21) with the degree of body weight change by administration of the BNS001D (PD-1 D-Fc) fusion protein and the BNS001I (Fc-IL-21) fusion protein, which are comparative controls, alone or in combination in humanized mice transplanted with human-derived colorectal cancer cells (HCT116).
Figure 75 illustrates a result obtained by comparing the tumor inhibitory effect by administration of the fusion protein (BNS001) with the tumor inhibitory effect by administration of atezolizumab, pembrolizumab, and avelumab, which are positive controls, in humanized mice transplanted with human-derived colorectal cancer cells (HCT116).
Figure 76 illustrates a result obtained by comparing the degree of body weight change by administration of the fusion protein (BNS001) with the degree of body weight change by administration of atezolizumab, pembrolizumab, and avelumab, which are positive controls, in humanized mice transplanted with human-derived colorectal cancer cells (HCT116).
Figure 77 illustrates a schematic diagram of the administration and experiment schedule for identifying the anti-cancer effect between the fusion protein (BNS001) and the control drug in humanized mice transplanted with human-derived lung cancer cells (A549).
Figure 78 illustrates a result obtained by comparing the tumor inhibitory effect by administration of the fusion protein (BNS001) with the tumor inhibitory effect by administration of atezolizumab, pembrolizumab, and avelumab, which are positive controls, in humanized mice transplanted with human-derived lung cancer cells (A549).
Figure 79 illustrates a result obtained by comparing the degree of body weight change by administration of the fusion protein (BNS001) with the degree of body weight change by administration of atezolizumab, pembrolizumab, and avelumab, which are positive controls, in humanized mice transplanted with human-derived lung cancer cells (A549).
Figure 80 illustrates a schematic diagram of the administration and experiment schedule for identifying the anti-cancer effect between the fusion protein (BNS001) and the control drug in humanized mice transplanted with human-derived breast cancer cells (MDA-MB-23 1).
Figure 81 illustrates a result obtained by comparing the tumor inhibitory effect by administration of the fusion protein (BNS001) with the tumor inhibitory effect by administration of atezolizumab, pembrolizumab, and avelumab, which are positive controls, in humanized mice transplanted with human-derived breast cancer cells (MDA-MB-231).
Figure 82 illustrates a result obtained by comparing the degree of body weight change by administration of the fusion protein (BNS001) with the degree of body weight change by administration of atezolizumab, pembrolizumab, and avelumab, which are positive controls, in humanized mice transplanted with human-derived breast cancer cells (MDA-MB-231).
Figure 83 illustrates a schematic diagram of the administration and experiment schedule for identifying the anti-cancer effect between the fusion protein (BNS001) and the control drug in humanized mice transplanted with human-derived lung cancer cells (NCI-H1975).
Figure 84 illustrates a result obtained by comparing the tumor inhibitory effect by administration of the fusion protein (BNS001) with the tumor inhibitory effect by administration of bevacizumab and ramucirumab, which are positive controls, in humanized mice transplanted with human-derived lung cancer cells (NCI-H1975).
Figure 85 illustrates a result obtained by comparing the degree of body weight change by administration of the fusion protein (BNS001) with the degree of body weight change by administration of bevacizumab and ramucirumab, which are positive controls, in humanized mice transplanted with human-derived lung cancer cells (NCI-H1975).
Figure 86 illustrates a result obtained by comparing the tumor inhibitory effect by administration of the fusion protein (BNS001) with the tumor inhibitory effect by administration of cetuximab and amivantamab, which are positive controls, in humanized mice transplanted with human-derived lung cancer cells (NCI-H1975).
Figure 87 illustrates a result obtained by comparing the degree of body weight change by administration of the fusion protein (BNS001) with the degree of body weight change by administration of cetuximab and amivantamab, which are positive controls, in humanized mice transplanted with human-derived lung cancer cells (NCI-H1975).

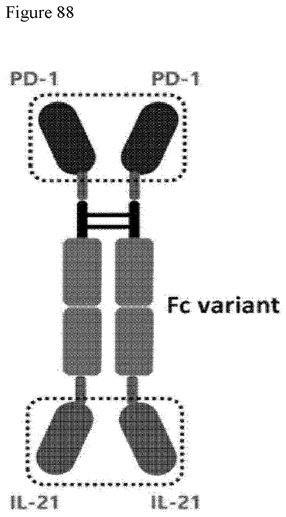

Figure 88 illustrates a schematic diagram of one embodiment of the fusion protein (BNS001) dimer.

### Best Mode for Carrying out the Invention

### Fusion protein comprising PD-1 protein and IL-21 protein

In one aspect of the present invention, there is provided a fusion protein comprising a PD-1 protein and an IL-21 protein.

As used herein, the term "PD-1 (programmed cell death protein 1 or programmed death receptor-1)" is a type of mutual inhibitory receptor belonging to the B7-CD28 family, and is an immune checkpoint protein, also known as CD279. PD-1 is widely expressed on the surface of activated T cells, natural killer T cells, B cells, and macrophages. When PD-1 binds to its ligands, PD-L1 (programmed cell death ligand-1) or PD-L2 (programmed cell death ligand-2), it induces intracellular signaling that inhibits CD3- and CD28-mediated T cell activation. Downregulation of the T cell activation results in a decrease in T cell proliferation, IFN-γ secretion, IL-2 secretion, and the like. The interaction between PD-1 and its ligands, PD-L1 or PD-L2, known to be expressed on various types of cancer cells (e.g., melanoma, liver cancer, lung cancer, ovarian cancer, cervical cancer, bladder cancer, kidney cancer, pancreatic cancer, breast cancer, lymphoma, glioma, and the like), inactivates T cells and acts as a mechanism for cancer cells to avoid attack by the immune system. Therefore, blocking the interaction between PD-1 and, PD-L1 or PD-L2 activates T cells within the tumor microenvironment to eliminate tumor cells.

As used herein, the term "PD-1", "programmed cell death receptor-1", or "programmed cell death protein-1", unless otherwise stated, refers to any wild type PD-1 obtained from any vertebrate source, including mammals, for example, primates (such as humans) and rodents (such as mice and rats). PD-1 may be obtained from animal cells, and also includes one obtained from recombinant cells capable of producing PD-1. In addition, PD-1 may be wild type PD-1 or a fragment thereof.

PD-1 is a type I membrane protein consisting of 288 amino acids, and its structure consists of an extracellular IgV-like domain, a transmembrane domain, and a cytoplasmic domain. The amino acid sequence of PD-1 may be a sequence described in GenBank accession No. NP_005009.2 or UniProtkB No. Q15116 (for the amino acid sequence of mouse PD-1, see Genbank accession No. NP_032824.1). In addition, the PDCD1 (programmed cell death 1) gene as a sequence encoding the PD-1 may be a nucleotide sequence corresponding to a CDS (coding sequence) among the sequences described in GenBank accession NO. NM_005018.3 (for the mouse sequence, see GenBank accession No. NM_008798.3).

As used herein, the term "PD-1 protein" refers to the full-length PD-1 or a PD-1 fragment. In the present specification, PD-1 or a fragment thereof may be collectively expressed by the term "PD-1 protein". PD-1, a PD-1 protein, and a PD-1 fragment specifically bind to, for example, PD-L1 or PD-L2, which is a ligand of PD-1. This specific binding may be identified by methods known to those of ordinary skill in the art.

PD-L1 and PD-L2, the ligands of PD-l, are proteins expressed on the surface of cancer cells. PD-L1 is also known as B7-H1 or CD274, and PD-L2 is also known as B7-DC or CD273. When PD-L1 or PD-L2 binds to PD-1 present in T cells, it changes immune checkpoint function to suppress T cell function. In other words, T cell function is suppressed by the interaction between PD-1 and PD-L1 or PD-L2, and ultimately cancer cells avoid attack by immune cells.

As used herein, the term "PD-1 fragment" refers to a truncated form of PD-1. In addition, the PD-1 fragment may be an extracellular domain of PD-1. One embodiment of a PD-1 fragment may be one in which the 1st to 24th amino acids from the N-terminus, which is the signal sequence of PD-1, are excluded. Specifically, one embodiment of the PD-1 fragment may be a protein consisting of the 25th to 288th amino acids of SEQ ID NO: 18. In addition, one embodiment of the PD-1 fragment may be a protein consisting of the 25th to 170th amino acids of SEQ ID NO: 18. In addition, one embodiment of the PD-1 fragment may be a protein consisting of the 25th to 150th amino acids of SEQ ID NO: 18. In addition, one embodiment of the PD-1 fragment may be a protein consisting of the 25th to 144th amino acids of SEQ ID NO: 18. In the above example, the PD-1 fragment may have the amino acid sequence of SEQ ID NO: 2 or 34.

In addition, one embodiment of the PD-1 fragment may be a protein consisting of the 25th to 169th amino acids of SEQ ID NO: 29. In the above example, the PD-1 fragment may have the amino acid sequence of SEQ ID NO: 21.

As used herein, the term "IL-21" or "interleukin-21" is a cytokine produced primarily by activated CD4+ and NK T cells. IL-21 belongs to the common γ-chain cytokine family and is involved in lymphocyte activation, proliferation, differentiation and survival. In addition, IL-21 activates the Jak/STAT signaling pathway through interaction with a heterodimeric receptor consisting of its specific receptor, IL-21R (interleukin-21 receptor), and a common γ-chain receptor. Signaling induced by the interaction between IL-21 and its receptor, IL-21R, induces an immune-inflammatory response. IL-21 induces the maturation of NK cell precursors from the bone marrow, and especially increases the effector functions of NK cells, such as the cytokine production ability and apoptosis ability. In addition, IL-21 not only induces cytotoxic activity by increasing degranulation and secretion of inflammatory cytokines, but also promotes expansion of the number of antigen-specific CD8+ T cells and effector functions, resulting in tumor regression.

As used herein, the term "IL-21" or "interleukin-21", unless otherwise stated, refers to any wild type IL-21 obtained from any vertebrate source, including mammals, for example, primates (such as humans) and rodents (such as mice and rats). IL-21 may be obtained from animal cells, and also includes one obtained from recombinant cells capable of producing IL-21. In addition, IL-21 may be wild type IL-21 or a variant thereof.

IL-21 consists of 162 amino acids, and specifically consists of a secretory signal sequence of amino acid residues 1 (Met) to 29 (Ser), and a mature polypeptide of amino acid residues 30 (Gln) to 162 (Ser). The amino acid sequence of IL-21 may be a sequence described in GenBank accession NO. NP_068575.1 or UniProtkB No. Q9HBE4 (for the amino acid sequence of mouse IL-21, see Genbank accession No. NP_068554.1). In addition, the IL-21 gene as a sequence encoding the IL-21 may be a nucleotide sequence corresponding to a CDS (coding sequence) among the sequences described in GenBank accession NO. NM_021803.4 (for the mouse sequence, see GenBank accession No. NM_021782.3).

As used herein, the term "IL-21 protein" refers to the full-length IL-21, an IL-21 fragment, or an IL-21 variant. In the present specification, IL-21, an IL-21 fragment, or a variant thereof may be collectively expressed by the term "IL-21 protein" or "IL-21 polypeptide". IL-21, an IL-21 protein, an IL-21 polypeptide, and an IL-21 variant specifically bind to, for example, an an IL-21 receptor (IL-21R). This specific binding may be identified by methods known to those of ordinary skill in the art.

IL-21R, the receptor for IL-21, is a transmembrane IL-21-binding protein belonging to the Class I cytokine receptor family. Signaling induced by the interaction of IL-21R and IL-21 induces the proliferation and activation of NK cells and T cells, promotes the secretion of INF-γ, and ultimately induces anti-cancer effects.

One embodiment of the term IL-21, as used herein, may have the amino acid sequence of SEQ ID NO: 19 or 30. Here, IL-21 may also be in a mature form. Specifically, the mature IL-21 may not comprise a signal sequence, and may have the amino acid sequence of SEQ ID NO: 6 or 22. Here, IL-21 may be used under a concept encompassing a fragment of wild type IL-21 in which a portion of N-terminus or C-terminus of the wild type IL-21 is truncated.

In addition, the IL-21 fragment may be in a form in which 1, 2, 3, 4, 5, 6, 7, 8, 9, 10, 11, 12, 13, 14, 15, 16, 17, 18, 19, 20, 21, 22, 23, or 24 contiguous amino acids are truncated from the N-terminus of a protein having the amino acid sequence of SEQ ID NO: 19 or 30. In addition, the IL-21 fragment may be in a form in which 1, 2, 3, 4, 5, 6, 7, 8, 9, 10, 11, 12, 13, 14, 15, 16, 17, 18, 19, 20, 21, 22, 23, or 24 contiguous amino acids are truncated from the C-terminus of a protein having the amino acid sequence of SEQ ID NO: 19 or 30.

As used herein, the term "IL-21 variant" refers to a form in which a portion of amino acids in the full-length IL-21 or the above-described IL-21 fragment is substituted. That is, an IL-21 variant may have an amino acid sequence different from wild type IL-21 or a fragment thereof. However, the IL-21 variant may have activity equivalent or similar to the wild type IL-21. Here, "IL-21 activity" may, for example, refer to specific binding to an an IL-21 receptor, which specific binding may be measured by methods known to those of ordinary skill in the art.

Specifically, the IL-21 variant may be obtained by deletion and/or substitution of a portion of amino acids in the wild type IL-21. One embodiment of an IL-21 variant obtained by amino acid deletion and/or substitution may be obtained by deletion of the secretory signal sequence of 1 (Met) to 29 (Ser) in the amino acid sequence of SEQ ID NO: 19, and deletion and/or substitution of amino acids in the 66 (Ile) to 98 (Ser) regions of the mature 133 amino acid peptide, as disclosed in U.S. Patent No. 8,034,326. The IL-2 variant may be characterized by maintained or improved IL-21 activity compared to the wild type IL-21.

In addition, a PD-1 protein and an IL-21 protein may be attached to each other via a linker or a carrier. Specifically, the PD-1 or a fragment thereof and the IL-21 or a variant thereof may be attached to each other via a linker or a carrier. In the present specification, the linker and the carrier may be used interchangeably.

The linker links two proteins. An embodiment of the linker may include 1 to 50 amino acids, albumin or a fragment thereof, an Fc domain of an immunoglobulin, or the like. Here, the Fc domain of an immunoglobulin refers to a protein that comprises heavy chain constant region 2 (CH2) and heavy chain constant region 3 (CH3) of an immunoglobulin, and does not comprise heavy and light chain variable regions and light chain constant region 1 (CH1) of an immunoglobulin. The immunoglobulin may be IgG, IgA, IgE, IgD, or IgM, and may preferably be IgG1. Here, an Fc domain of wild type immunoglobulin G1 may have the amino acid sequence of SEQ ID NO: 14.

In addition, the Fc domain of an immunoglobulin may be an Fc domain variant as well as a wild type Fc domain. In addition, as used herein, the term "Fc domain variant" may refer to a form which is different from the wild type Fc domain in terms of glycosylation pattern, has a high glycosylation as compared with the wild type Fc domain, or has a low glycosylation as compared with the wild type Fc domain, or a deglycosylated form. In addition, an aglycosylated Fc domain is included therein. The Fc domain or a variant thereof may be adapted to have an adjusted number of sialic acids, fucosylations, or glycosylations, through culture conditions or genetic engineering of a host.

In addition, glycosylation of the Fc domain of an immunoglobulin may be modified by conventional methods such as chemical methods, enzymatic methods, and genetic engineering methods using microorganisms. In addition, the Fc domain variant may be in a mixed form of respective Fc regions of immunoglobulin IgG, IgA, IgE, IgD or IgM. In addition, the Fc domain variant may be in a form in which some amino acids of the Fc domain are substituted with other amino acids. One embodiment of the Fc domain variant may be obtained by a substitution of Q81R, M198L, L79G, T136W, Q81R/M198L, L79G/M198L or Q81R/T136W/M198L in the amino acid sequence of SEQ ID NO: 14. In addition, the Fc domain may be further modified to prevent cleavage by IdeS protease. For example, it may be obtained by substitution of E3P, L4V or L5A or deletion of the amino acid of G6 in the amino acid sequence of SEQ ID NO: 14.

Specifically, one embodiment of the Fc domain variant may have the amino acid sequence of SEQ ID NO: 4, 10, 50, 87, 95, 99, 104, 108, 112, or 116.

One embodiment of the Fc domain variant by amino acid substitution may be an Fc domain variant comprising an amino acid substitution that modulates association and dissociation to FcRn (neonatal Fc receptor), as disclosed in Korean Patent No. 10-1957431. This may include, in particular, an Fc variant that shows increased binding affinity for FcRn at low pH and does not show substantially altered binding affinity at high pH, or a functional variant thereof.

The Fc domain variant may have a binding affinity for FcRn at pH 5.6 to 6.2 (preferably pH 5.8 to 6.0) that is increased by 10% or more, 20% or more, 30% or more, 40% or more, 50% or more, 60% or more, 70% or more, 80% or more, 90% or more, or 100% or more compared to the wild type Fc domain, or may have a binding affinity for FcRn at pH 5.6 to 6.2 (preferably pH 5.8 to 6.0) that is increased by 2-fold or more, 3-fold or more, 4-fold or more, 5-fold or more, 6-fold or more, 7-fold or more, 8-fold or more, 9-fold or more, 10-fold or more, 20-fold or more, 30-fold or more, 40-fold or more, 50-fold or more, 60-fold or more, 70-fold or more, 80-fold or more, 90-fold or more or 100-fold or more to the wild type Fc domain.

The degree of dissociation from FcRn (neonatal Fc receptor) of the Fc domain variant at pH 7.0 to 7.8 (preferably pH 7.2 to 7.6) may be the same or may not be substantially changed compared to the wild type Fc domain.

In addition, the Fc domain variant may be characterized by an increased half-life compared to the wild type. Specifically, the Fc domain variant may have a half-life that is increased by 10% or more, 20% or more, 30% or more, 40% or more, 50% or more, 60% or more, 70% or more, 80% or more, 90% or more, or 100% or more compared to the wild type Fc domain, or may have a half-life that is increased by 2-fold or more, 3-fold or more, 4-fold or more, 5-fold or more, 6-fold or more, 7-fold or more, 8-fold or more, 9-fold or more, or 10-fold or more compared to the wild type Fc domain.

As used herein, the term "FcRn (neonatal Fc receptor)" or "neonatal Fc receptor" is an MHC class I-related protein expressed in vascular endothelial cells and binds to IgG and albumin. A characteristic feature is that the binding between IgG and FcRn is strong when the pH is slightly acidic, and there is no binding force at neutral pH. Therefore, IgG, which enters cells through pinocytosis or endocytosis, may avoid the degradative lysosomal pathway by binding strongly to FcRn, a type of Fc gamma receptor (FcyR), in endosomes under pH 6.0 conditions. Once circulated back to the cell membrane, IgG rapidly dissociates from FcRn in the bloodstream at pH 7.4. FcRn is known to effectively block the degradation of IgG in lysosomes and extend the half-life of IgG through this receptor mediated recycling mechanism.

As used herein, the term "Fc gamma receptor (FcyR)" refers to an Fc receptor for IgG, and there are three major types: FcyRI (CD64), FcyRII (CD32), and FcyRIII (CD16). The Fc receptors are molecules that bind to immunoglobulins and allow the bound antibody to perform various biological functions independently of the binding site with the antigen, and are distributed on the surface of various cells and tissues. The Fc receptors are involved in cytotoxicity, antibody-dependent cell-mediated cytotoxicity (ADCC), secretion of mediators such as cytokines, phagocytosis, initiation of oxidation, and regulation of antibody production.

The fusion protein may have a structure in which, using an Fc domain as a linker (or carrier), PD-1 and an IL-21 protein, or IL-21 and PD-1 are linked to the N-terminus and C-terminus of the linker or carrier, respectively. Linkage between the N-terminus or C-terminus of the Fc domain and PD-1 or IL-21 may optionally be achieved by a linker peptide.

Specifically, the fusion protein may consist of the following structural formula I or II:

N'-A-[Li]n-Fc domain-[L₂]m-B-C' I

N'-B-[L₁]n-Fc domain-[L₂]m-A-C' II

wherein, in the structural formulas I and II,
N' is the N-terminus of the fusion protein,
C' is the C-terminus of the fusion protein,
A is the PD-1 protein or a fragment thereof,
B is the IL-21 protein or a variant thereof,
L₁ and L₂ are peptide linkers, and
n and m are each independently 0 or 1.

Preferably, the fusion protein may consist of the structural formula I. The PD-1 protein and an IL-21 protein are each as described above. According to one embodiment, the PD-1 protein may be a fragment truncated up to about 24 contiguous amino acid residues from the N-terminus or C-terminus of the wild type PD-1. The IL-21 protein may be a fragment truncated up to about 24 contiguous amino acid residues from the N-terminus or C-terminus of the wild type IL-21. Alternatively, the IL-21 protein may be an IL-21 variant obtained by deletion and/or substitution of a portion of amino acids in an IL-21 fragment.

Specifically, the fusion protein may have the amino acid sequence of SEQ ID NO: 8, 12, 16, 24, 27, 32, 36, 41, 44, 52, 55, 76, 79, 82, 89, 92, 97, 101, 106, 110, 114, or 118. According to another embodiment, the fusion protein includes a polypeptide having a sequence identity of 85%, 86%, 87%, 88%, 89%, 90%, 91%, 92%, 93%, 94%, 95%, 96%, 97%, 98%, 99%, or 100% to the amino acid sequence of SEQ ID NO: 8, 12, 16, 24, 27, 32, 36, 41, 44, 52, 55, 76, 79, 82, 89, 92, 97, 101, 106, 110, 114, or 118. Here, the identity is, for example, percent homology, and may be determined through homology comparison software such as BlastN software of the National Center of Biotechnology Information (NCBI).

The peptide linker Li may be included between the PD-1 protein and the Fc domain. The peptide linker Li may consist of 5 to 80 contiguous amino acids, 20 to 60 contiguous amino acids, 25 to 50 contiguous amino acids, or 30 to 40 contiguous amino acids. In one embodiment, the peptide linker Li may consist of 30 amino acids. In addition, the peptide linker Li may comprise at least one cysteine. Specifically, the peptide linker Li may comprise one, two, or three cysteines. In addition, the peptide linker Li may be derived from the hinge of an immunoglobulin. In one embodiment, the peptide linker Li may be a peptide linker consisting of the amino acid sequence of SEQ ID NO: 3 or 38.

The peptide linker L₂ may consist of 1 to 50 contiguous amino acids, 3 to 30 contiguous amino acids, or 5 to 15 contiguous amino acids. In one embodiment, the peptide linker L₂ may be (G₄S)n (where n is an integer of 1 to 10). Here, in (G₄S)n, n may be 1, 2, 3, 4, 5, 6, 7, 8, 9, or 10. In one embodiment, the peptide linker L₂ may be a peptide linker consisting of the amino acid sequence of SEQ ID NO: 5, 39, or 57.

In another aspect of the present invention, there is provided a fusion protein dimer in which the two fusion proteins are attached to each other comprising a PD-1 protein and an IL-21 protein. The fusion protein comprising PD-1 or a fragment thereof and IL-21 or a variant thereof is as described above.

Here, the binding between the fusion proteins constituting the dimer may be achieved by, but is not limited to, a disulfide bond formed by cysteines present in the linker. The fusion proteins constituting the dimer may be the same or different fusion proteins from each other. Preferably, the dimer may be a homodimer. One embodiment of the fusion protein constituting the dimer may be a protein having the amino acid sequence of SEQ ID NO: 8, 12, 16, 24, 27, 32, 36, 41, 44, 52, 55, 76, 79, 82, 89, 92, 97, 101, 106, 110, 114, or 118.

### Polynucleotide encoding fusion protein

In another aspect of the present invention, there is provided a polynucleotide encoding a fusion protein comprising a PD-1 protein and an IL-21 protein. Specifically, the polynucleotide may comprise the nucleotide sequence of SEQ ID NO: 9, 13, 17, 25, 28, 33, 37, 42, 45, 53, 56, 77, 80, 83, 90, 93, 98, 102, 107, 111, 115, or 119. The fusion protein comprising a PD-1 protein and an IL-21 protein is as described above. In the polynucleotide, one or more nucleotides may be mutated by substitution, deletion, insertion, or a combination thereof. When a nucleotide sequence is prepared by chemical synthesis, synthetic methods well known in the art may be used, such as those described in Engels and Uhlmann (Angew Chem IntEd Engl., 37:73-127, 1988). Such methods may include triester, phosphite, phosphoramidite and H-phosphate methods, PCR and other autoprimer methods, oligonucleotide syntheses on solid supports, and the like.

According to one embodiment, the polynucleotide may comprise a nucleic acid sequence having an identity of at least about 70%, at least about 75%, at least about 80%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, or at least about 100% to SEQ ID NO: 9, 13, 17, 25, 28, 33, 37, 42, 45, 53, 56, 77, 80, 83, 90, 93, 98, 102, 107, 111, 115, or 119.

The polynucleotide may further comprise a nucleic acid encoding a signal sequence or a leader sequence. As used herein, the term "signal sequence" refers to a signal peptide that directs secretion of a target protein. The signal peptide is translated and then cleaved in a host cell. Specifically, the signal sequence is an amino acid sequence that initiates migration of a protein across the endoplasmic reticulum (ER) membrane. In one embodiment, the signal sequence may have the amino acid sequence of SEQ ID NO: 1, 20, or 94.

Signal sequences are well known in the art for their characteristics. Such signal sequences typically comprise 16 to 30 amino acid residues, and may comprise more or fewer amino acid residues than such amino acid residues. A typical signal peptide is composed of three regions, that is, a basic N-terminal region, a central hydrophobic region, and a more polar C-terminal region. The central hydrophobic region comprises 4 to 12 hydrophobic residues that cause the signal sequence to be immobilized during migration of an immature polypeptide through the membrane lipid bilayer.

After initiation, signal sequences are cleaved in the lumen of ER by cellular enzymes, commonly known as signal peptidases. Here, the signal sequence may be a secretory signal sequence of tPa (tissue plasminogen activator), HSV gDs (signal sequence of Herpes simplex virus glycoprotein D), or a growth hormone. Preferably, a secretory signal sequence used in higher eukaryotic cells including mammals and the like may be used. In addition, a signal sequence included in the wild type PD-1 and/or IL-21 may be used, or a signal sequence that has been substituted with a codon having high expression frequency in a host cell may be used.

### Vector with polynucleotide encoding fusion protein

In another aspect of the present invention, there is provided a vector comprising the polynucleotide.

The vector may be introduced into a host cell to be recombined with and inserted into the genome of the host cell. Alternatively, the vector is understood as nucleic acid means comprising a polynucleotide sequence which is autonomously replicable as an episome. The vectors include linear nucleic acids, plasmids, phagemids, cosmids, RNA vectors, viral vectors, and analogs thereof. Examples of the viral vector include, but are not limited to, retroviruses, adenoviruses, and adeno-associated viruses.

Specifically, the vector may include plasmid DNA, phage DNA, and the like; and commercially developed plasmids (pUC18, pBAD, pIDTSAMRT-AMP, and the like), *E. coli-*derived plasmids (pYG601BR322, pBR325, pUC118, pUC119, and the like), *Bacillus subtilis-*derived plasmids (pUB110, pTP5, and the like), yeast-derived plasmids (YEp13, YEp24, YCp50, and the like), phage DNA (Charon4A, Charon21A, EMBL3, EMBL4, λgt10, λgt11, λZAP, and the like), animal viral vectors (retroviruses, adenoviruses, vaccinia viruses, and the like), insect viral vectors (baculoviruses and the like). Since the vector exhibits different expression levels and modification of a protein depending on a host cell, it is preferred to select and use a host cell which is most suitable for the purpose.

As used herein, the term "gene expression" or "expression" of a target protein is understood to mean transcription of DNA sequences, translation of mRNA transcripts, and secretion of fusion protein products or fragments thereof. A useful expression vector may be RcCMV (Invitrogen, Carlsbad) or a variant thereof. The expression vector may comprise human cytomegalovirus (CMV) promoter for promoting continuous transcription of a target gene in mammalian cells, and a bovine growth hormone polyadenylation signal sequence for increasing the stability level of RNA after transcription.

### Transformed cell expressing fusion protein

In another aspect of the present invention, there is provided a transformed host cell into which the vector has been introduced.

Host cells for the transformed cell may include, but are not limited to, prokaryotic cells, eukaryotic cells, and cells of mammalian, plant, insect, fungal, or cellular origin. As an example of the prokaryotic cells, *E. coli* may be used. In addition, as an example of the eukaryotic cells, yeast may be used. In addition, for the mammalian cells, CHO cells, F2N cells, CSO cells, BHK cells, Bowes melanoma cells, HeLa cells, 911 cells, AT1080 cells, A549 cells, HEK 293 cells, HEK293T cells, or the like may be used. However, the mammalian cells are not limited thereto, and any cells which are known to those of ordinary skill in the art to be usable as mammalian host cells may be used.

In addition, for the introduction of an expression vector into the host cell, CaCl₂ precipitation, Hanahan method whose efficiency has been increased efficiency by using a reducing agent such as dimethyl sulfoxide (DMSO) in CaCl₂ precipitation, electroporation, calcium phosphate precipitation, protoplast fusion, agitation using silicon carbide fiber, Agrobacterium-mediated transformation, transformation using PEG, dextran sulfate-, Lipofectamine-, and dry/inhibition-mediated transformation, and the like may be used.

As described above, for optimization of properties of a fusion protein as a therapeutic agent or for any other purpose, glycosylation pattern of the fusion protein (for example, sialic acids, fucosylations, glycosylations) may be adjusted by engineering, through methods known to those of ordinary skill in the art, glycosylation-related genes possessed by host cells.

### Method for producing a fusion protein

In another aspect of the present invention, there is provided a method for producing a fusion protein comprising a PD-1 protein and an IL-21 protein, the method comprising culturing the transformed cells. Specifically, the production method may comprise: i) culturing the transformed cells to obtain a culture product; and ii) collecting the fusion protein from the culture product.

Culturing the transformed cells may be carried out using methods well known in the art. Specifically, the culture may be carried out in a batch process, or carried out continuously in a fed batch or repeated fed batch process.

### Use of fusion protein or dimer thereof

In another aspect of the present invention, there is provided a pharmaceutical composition for preventing or treating cancer, and/or for increasing therapeutic effect (efficacy), the pharmaceutical composition comprising a fusion protein comprising a PD-1 protein and an IL-21 protein or a fusion protein dimer in which the two fusion proteins are attached to each other as an active ingredient.

The fusion protein comprising a PD-1 protein and an IL-21 protein, or the fusion protein dimer in which the two fusion proteins are attached to each other is as described above.

The cancer may be selected from the group consisting of colorectal cancer, melanoma, gastric cancer, liver cancer, lung cancer, breast cancer, prostate cancer, ovarian cancer, pancreatic cancer, cervical cancer, bladder cancer, kidney cancer, gallbladder cancer, thyroid cancer, laryngeal cancer, acute myeloid leukemia, brain tumor, neuroblastoma, retinoblastoma, head and neck cancer, salivary gland cancer, and lymphoma.

A preferred dosage of the pharmaceutical composition varies depending on the patient's condition and body weight, severity of disease, form of drug, route and duration of administration and may be appropriately selected by those of ordinary skill in the art. In the pharmaceutical composition for treating or preventing cancer of the present invention, the active ingredient may be contained in any amount (effective amount) depending on usage, dosage form, blending purpose, and the like, as long as the active ingredient may exhibit anti-cancer activity. A conventional effective amount thereof will be determined within a range of 0.001% to 20.0% by weight, based on the total weight of the composition. Here, the term "effective amount" refers to an amount of an active ingredient capable of inducing an anti-cancer effect. Such an effective amount may be experimentally determined within the scope of common knowledge of those of ordinary skill in the art.

As used herein, the term "treatment" may be used to mean both therapeutic and prophylactic treatment. Here, prophylaxis may be used to mean that a pathological condition or disease of a subject is alleviated or mitigated. In one embodiment, the term "treatment" includes both application or any form of administration for treating a disease in a mammal, including a human. In addition, the term includes inhibiting or slowing down a disease or disease progression; and includes meanings of restoring or repairing impaired or lost function so that a disease is partially or completely alleviated; stimulating inefficient processes; or alleviating a serious disease.

As used herein, the term "efficacy" refers to capacity that may be determined by one or more parameters, for example, survival or disease-free survival over a certain period of time such as one year, five years, or ten years. In addition, the parameter may include inhibition of size of at least one tumor in a subject.

Pharmacokinetic parameters such as bioavailability and underlying parameters such as clearance rate may also affect efficacy. Therefore, "enhanced efficacy" (for example, improvement in efficacy) may be due to enhanced pharmacokinetic parameters and enhanced efficacy, which may be measured by comparing clearance rate and tumor growth in test animals or human subjects, or by comparing parameters such as survival, recurrence, or disease-free survival.

As used herein, the term "therapeutically effective amount" or "pharmaceutically effective amount" refers to an amount of a compound or composition effective to prevent or treat the disease in question, which is sufficient to treat the disease at a reasonable benefit/risk ratio applicable to medical treatment and does not cause adverse effects. A level of the effective amount may be determined depending on factors including the patient's health condition, type and severity of disease, activity of drug, the patient's sensitivity to drug, mode of administration, time of administration, route of administration and excretion rate, duration of treatment, combined or simultaneously used drugs, and other factors well known in the medical field. In one embodiment, the therapeutically effective amount means an amount of drug effective to treat cancer.

Here, the pharmaceutical composition may further comprise a pharmaceutically acceptable carrier. The pharmaceutically acceptable carrier may be any carrier as long as the carrier is a non-toxic substance suitable for delivery to a patient. Distilled water, alcohol, fat, wax, and inert solid may be contained as the carrier. A pharmaceutically acceptable adjuvant (buffer, dispersant) may also be contained in the pharmaceutical composition.

Specifically, by including a pharmaceutically acceptable carrier in addition to the active ingredient, the pharmaceutical composition may be prepared into a parenteral formulation depending on its route of administration using conventional methods known in the art. Here, the term "pharmaceutically acceptable" means that the carrier does not have more toxicity than the subject to be applied (prescribed) may adapt while not inhibiting activity of the active ingredient.

When the pharmaceutical composition is prepared into a parenteral formulation, it may be made into preparations in the form of injections, transdermal patches, nasal inhalants, or suppositories with suitable carriers according to methods known in the art. In a case of being made into injections, sterile water, ethanol, polyol such as glycerol or propylene glycol, or a mixture thereof may be used as a suitable carrier; and an isotonic solution, such as Ringer's solution, phosphate buffered saline (PBS) or sterile water containing triethanol amine for injection, and 5% dextrose, or the like may preferably be used. Formulation of pharmaceutical compositions is known in the art, and reference may specifically be made to Remington's Pharmaceutical Sciences (19th ed., 1995) and the like. This document is considered part of the present specification.

A preferred dosage of the pharmaceutical composition may range from 0.01 µg/kg to 10 g/kg, or 0.01 mg/kg to 1 g/kg, per day, depending on the patient's condition, body weight, sex, age, severity of the patient, and route of administration. The dosage may be administered once a day or may be divided into several times a day. Such a dosage should not be construed as limiting the scope of the present invention in any aspect.

Subjects to which the pharmaceutical composition may be applied (prescribed) are mammals and humans, with humans being particularly preferred. In addition to the active ingredient, the pharmaceutical composition of the present invention may further comprise any compound or natural extract, which has already been validated for safety and is known to have a therapeutic effect on anti-cancer activity, so as to boost or reinforce anti-cancer activity.

In another aspect of the present invention, there is provided a use of a fusion protein comprising a PD-1 protein and an IL-21 protein for treating cancer.

In another aspect of the present invention, there is provided a use of a fusion protein comprising a PD-1 protein and an IL-21 protein for enhancing a therapeutic effect on cancer.

In another aspect of the present invention, there is provided a use of a fusion protein comprising a PD-1 protein and an IL-21 protein for manufacture of a medicament for treating cancer.

In another aspect of the present invention, there is provided a method for treating cancer, and/or a method for enhancing a therapeutic effect on cancer, comprising administering a fusion protein comprising a PD-1 protein and an IL-21 protein or a fusion protein dimer in which the two fusion proteins are attached to each other to a subject.

The subject may be an individual suffering from cancer. In addition, the subject may be a mammal, preferably a human. The fusion protein comprising a PD-1 protein and an IL-21 protein, or the fusion protein dimer in which the two fusion proteins are attached to each other is as described above.

Route of administration, dosage, and frequency of administration of the fusion protein or the fusion protein dimer may vary depending on the patient's condition and the presence or absence of side effects, and thus the fusion protein or the fusion protein dimer may be administered to a subject in various ways and amounts. The optimal administration method, dosage, and frequency of administration may be selected in an appropriate range by those of ordinary skill in the art. In addition, the fusion protein or the fusion protein dimer may be administered in combination with other drugs or physiologically active substances whose therapeutic effect is known with respect to a disease to be treated, or may be formulated in the form of combination preparations with other drugs.

### Cell culture medium comprising fusion protein or dimer thereof

In another aspect of the present invention, there is provided a cell culture medium comprising a fusion protein comprising a PD-1 protein and an IL-21 protein or a fusion protein dimer in which the two fusion proteins are attached to each other.

Here, the cells may be T cells or natural killer cells. The cell culture medium may be a cell culture medium supplemented with a fusion protein comprising a PD-1 protein and an IL-21 protein or a fusion protein dimer in which the two fusion proteins are attached to each other. The cell culture medium may comprise any one selected from the group consisting of amino acids, sugars, inorganic salts, and vitamins. Preferably, the cell culture medium may comprise all amino acids, sugars, inorganic salts, and vitamins.

As used herein, the term "cell culture medium" refers to a medium used to culture cells, specifically refers to a medium for culturing immune cells, more specifically CD4+ or CD8+ T cells; or NK cells. The medium contains ingredients that cells need for cell growth and survival *in vitro,* or contains ingredients that help cell growth and survival. Specifically, the ingredients may be vitamins, essential or non-essential amino acids, and trace elements. The medium may be a medium used for culturing cells, preferably eukaryotic cells, more preferably T cells or NK cells.

### Mode for Carrying out the Invention

Hereinafter, the present invention will be described in more detail by way of the following examples. However, the following examples are only for illustrating the present invention, and the scope of the present invention is not limited only to these examples.

### I. Preparation of fusion protein: BNS001

### Preparation Example 1. Preparation of hPD-1 D-Fc(29)-hIL-21

In order to produce a fusion protein comprising a human PD-1 fragment, a persistent Fc(29) domain, and IL-21, a polynucleotide was synthesized through the Gene Synthesis service of GenScript. Specifically, the polynucleotide comprises a nucleotide sequence (SEQ ID NO: 9) which encodes a fusion protein (SEQ ID NO: 7) that comprises a signal peptide (SEQ ID NO: 1), a PD-1 fragment (SEQ ID NO: 2), an Ig hinge (SEQ ID NO: 3) to which a linker is bound, an Fc(29) domain (SEQ ID NO: 4), a linker (SEQ ID NO: 5), and IL-21 (SEQ ID NO: 6), in this order, from the N-terminus. The polynucleotide was introduced into pcDNA3.4 TOPO vector (Thermo Fisher Scientific).

The expression vector was introduced into CHO cells (EXPICHO-S^{™}, Thermo Fisher Scientific) to express the fusion protein. After the CHO cells were transfected with the expression vector and cultured for 11 days using the manufacturer's Max Protocol, the culture solution was collected and the fusion protein was purified therefrom. The purified fusion protein (SEQ ID NO: 8) was designated "hPD-1 D-Fc(29)-hIL-21" or "BNS001(29)", and as such, the fusion protein dimer comprising PD-1 and IL-21 was collectively designated "BNS001".

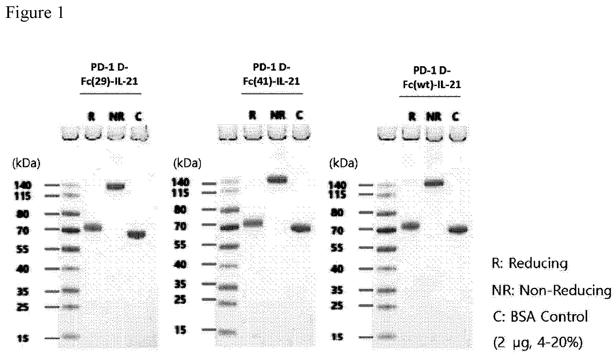

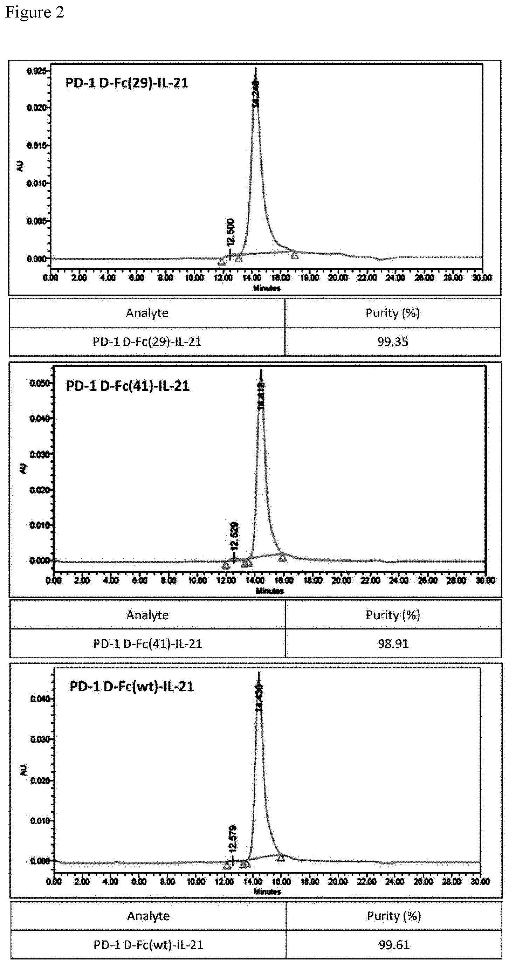

The fusion protein in the collected culture solution was subjected to first purification using affinity chromatography containing Protein A resin (KANEKA). The bound fusion protein and resin were washed with DPBS buffer, and then only the bound fusion protein was eluted with 0.1 M glycine buffer (pH 3.3). The eluted fusion protein was dialyzed against DPBS buffer for one day to exchange the buffer, and then was subjected to second purification by performing size exclusion chromatography using a HILOAD^{®} 16/600 SUPERDEX^{®} 200 pg column (Cytiva). The isolated and purified fusion protein was quantified using NanoDrop, and purity thereof was analyzed by SDS-PAGE and SEC-HPLC analysis (Figures 1 and 2).

### Preparation Example 2. Preparation of hPD-1 D-Fc(41)-hIL-21

In order to produce a fusion protein comprising a human PD-1 fragment, a persistent Fc(41) domain, and IL-21, a polynucleotide was synthesized through the Gene Synthesis service of GenScript. Specifically, the polynucleotide comprises a nucleotide sequence (SEQ ID NO: 13) which encodes a fusion protein (SEQ ID NO: 11) that comprises a signal peptide (SEQ ID NO: 1), a PD-1 fragment (SEQ ID NO: 2), an Ig hinge (SEQ ID NO: 3) to which a linker is bound, an Fc(41) domain (SEQ ID NO: 10), a linker (SEQ ID NO: 5), and IL-21 (SEQ ID NO: 6), in this order, from the N-terminus. The polynucleotide was introduced into pcDNA3.4 TOPO vector (Thermo Fisher Scientific).

The expression vector was introduced into CHO cells (EXPICHO-S^{™}, Thermo Fisher Scientific) to express the fusion protein. After the CHO cells were transfected with the expression vector and cultured for 11 days using the manufacturer's Max Protocol, the culture solution was collected and the fusion protein was purified therefrom. The purified fusion protein (SEQ ID NO: 12) was designated "hPD-1 D-Fc(41)-hIL-21" or "BNS001(41)", and as such, the fusion protein dimer comprising PD-1 and IL-21 was collectively designated "BNS001".

The purification and collection of the fusion protein were carried out in the same manner as in Preparation Example 1 above. The isolated and purified fusion protein was quantified using NanoDrop, and purity thereof was analyzed by SDS-PAGE and SEC-HPLC analysis (Figures 1 and 2).

### Preparation Example 3. Preparation of hPD-1 D-Fc(wt)-hIL-21

In order to produce a fusion protein comprising a human PD-1 fragment, a persistent Fc(wt) domain, and IL-21, a polynucleotide was synthesized through the Gene Synthesis service of GenScript. Specifically, the polynucleotide comprises a nucleotide sequence (SEQ ID NO: 17) which encodes a fusion protein (SEQ ID NO: 15) that comprises a signal peptide (SEQ ID NO: 1), a PD-1 fragment (SEQ ID NO: 2), an Ig hinge (SEQ ID NO: 3) to which a linker is bound, an Fc(wt) domain (SEQ ID NO: 14), a linker (SEQ ID NO: 5), and IL-21 (SEQ ID NO: 6), in this order, from the N-terminus. The polynucleotide was introduced into pcDNA3.4 TOPO vector (Thermo Fisher Scientific).

The expression vector was introduced into CHO cells (EXPICHO-S^{™}, Thermo Fisher Scientific) to express the fusion protein. After the CHO cells were transfected with the expression vector and cultured for 11 days using the manufacturer's Max Protocol, the culture solution was collected and the fusion protein was purified therefrom. The purified fusion protein (SEQ ID NO: 16) was designated "hPD-1 D-Fc(wt)-hIL-21" or "BNS001(wt)", and as such, the fusion protein dimer comprising PD-1 and IL-21 was collectively designated "BNS001".

The purification and collection of the fusion protein were carried out in the same manner as in Preparation Example 1 above. The isolated and purified fusion protein was quantified using NanoDrop, and purity thereof was analyzed by SDS-PAGE and SEC-HPLC analysis (Figures 1 and 2).

### Preparation Example 4. Preparation of mPD-1 D-Fc(29)-mIL-21

In order to produce a fusion protein comprising a mouse PD-1 fragment, a persistent Fc(29) domain, and IL-21, a polynucleotide was synthesized through the Gene Synthesis service of GenScript. Specifically, the polynucleotide comprises a nucleotide sequence (SEQ ID NO: 25) which encodes a fusion protein (SEQ ID NO: 23) that comprises a signal peptide (SEQ ID NO: 20), a PD-1 fragment (SEQ ID NO: 21), an Ig hinge (SEQ ID NO: 3) to which a linker is bound, an Fc(29) domain (SEQ ID NO: 4), a linker (SEQ ID NO: 5), and IL-21 (SEQ ID NO: 22), in this order, from the N-terminus. The polynucleotide was introduced into pcDNA3.4 TOPO vector (Thermo Fisher Scientific).

The expression vector was introduced into CHO cells (EXPICHO-S^{™}, Thermo Fisher Scientific) to express the fusion protein. After the CHO cells were transfected with the expression vector and cultured for 11 days using the manufacturer's Max Protocol, the culture solution was collected and the fusion protein was purified therefrom. The purified fusion protein (SEQ ID NO: 24) was designated "mPD-1 D-Fc(29)-mIL-21" or "mBNS001(29)", and as such, the fusion protein dimer comprising mPD-1 and mIL-21 was collectively designated "BNS001". The purification and collection of the fusion protein were carried out in the same manner as in Preparation Example 1 above.

### Preparation Example 5. Preparation of mPD-1 D-Fc(wt)-mIL-21

In order to produce a fusion protein comprising a mouse PD-1 fragment, a persistent Fc(wt) domain, and IL-21, a polynucleotide was synthesized through the Gene Synthesis service of GenScript. Specifically, the polynucleotide comprises a nucleotide sequence (SEQ ID NO: 28) which encodes a fusion protein (SEQ ID NO: 26) that comprises a signal peptide (SEQ ID NO: 20), a PD-1 fragment (SEQ ID NO: 21), an Ig hinge (SEQ ID NO: 3) to which a linker is bound, an Fc(wt) domain (SEQ ID NO: 14), a linker (SEQ ID NO: 5), and IL-21 (SEQ ID NO: 22), in this order, from the N-terminus. The polynucleotide was introduced into pcDNA3.4 TOPO vector (Thermo Fisher Scientific).

The expression vector was introduced into CHO cells (EXPICHO-S^{™}, Thermo Fisher Scientific) to express the fusion protein. After the CHO cells were transfected with the expression vector and cultured for 11 days using the manufacturer's Max Protocol, the culture solution was collected and the fusion protein was purified therefrom. The purified fusion protein (SEQ ID NO: 27) was designated "mPD-1 D-Fc(wt)-mIL-21" or "mBNS001(wt)", and as such, the fusion protein dimer comprising PD-1 and IL-21 was collectively designated "BNS001". The purification and collection of the fusion protein were carried out in the same manner as in Preparation Example 1 above.

### Preparation Example 6. Preparation of hPD-1 D-Fc(29)-mIL-21

In order to produce a fusion protein comprising a human PD-1 fragment, a persistent Fc(29) domain, and mouse IL-21, a polynucleotide was synthesized through the Gene Synthesis service of GenScript. Specifically, the polynucleotide comprises a nucleotide sequence (SEQ ID NO: 33) which encodes a fusion protein (SEQ ID NO: 31) that comprises a signal peptide (SEQ ID NO: 1), a PD-1 fragment (SEQ ID NO: 2), an Ig hinge (SEQ ID NO: 3) to which a linker is bound, an Fc(29) domain (SEQ ID NO: 4), a linker (SEQ ID NO: 5), and mouse IL-21 (SEQ ID NO: 22), in this order, from the N-terminus. The polynucleotide was introduced into pcDNA3.4 TOPO vector (Thermo Fisher Scientific).

The expression vector was introduced into CHO cells (EXPICHO-S^{™}, Thermo Fisher Scientific) to express the fusion protein. After the CHO cells were transfected with the expression vector and cultured for 11 days using the manufacturer's Max Protocol, the culture solution was collected and the fusion protein was purified therefrom. The purified fusion protein (SEQ ID NO: 32) was designated "hPD-1 D-Fc(29)-mIL-21", and as such, the fusion protein dimer comprising PD-1 and IL-21 was collectively designated "BNS001". The purification and collection of the fusion protein were carried out in the same manner as in Preparation Example 1 above.

### Preparation Example 7. Preparation of hPD-1 ECD-Fc(29)-hIL-21

In order to produce a fusion protein comprising a human PD-1 fragment, a persistent Fc(29) domain, and IL-21, a polynucleotide was synthesized through the Gene Synthesis service of GenScript. Specifically, the polynucleotide comprises a nucleotide sequence (SEQ ID NO: 37) which encodes a fusion protein (SEQ ID NO: 35) that comprises a signal peptide (SEQ ID NO: 1), a PD-1 fragment (SEQ ID NO: 34), an Ig hinge (SEQ ID NO: 3) to which a linker is bound, an Fc(29) domain (SEQ ID NO: 4), a linker (SEQ ID NO: 5), and IL-21 (SEQ ID NO: 6), in this order, from the N-terminus. The polynucleotide was introduced into pcDNA3.4 TOPO vector (Thermo Fisher Scientific).

The expression vector was introduced into CHO cells (EXPICHO-S^{™}, Thermo Fisher Scientific) to express the fusion protein. After the CHO cells were transfected with the expression vector and cultured for 11 days using the manufacturer's Max Protocol, the culture solution was collected and the fusion protein was purified therefrom. The purified fusion protein (SEQ ID NO: 36) was designated "hPD-1 ECD-Fc(29)-hIL-21", and as such, the fusion protein dimer comprising PD-1 and IL-21 was collectively designated "BNS001". The purification and collection of the fusion protein were carried out in the same manner as in Preparation Example 1 above.

### Preparation Example 8. Preparation of hIL-21-Fc(29)-hPD-1 D

In order to produce a fusion protein comprising a human IL-21 fragment, a persistent Fc(29) domain, and a PD-1 fragment, a polynucleotide was synthesized through the Gene Synthesis service of GenScript. Specifically, the polynucleotide comprises a nucleotide sequence (SEQ ID NO: 42) which encodes a fusion protein (SEQ ID NO: 40) that comprises a signal peptide (SEQ ID NO: 20), IL-21 (SEQ ID NO: 6), an Ig hinge (SEQ ID NO: 38) to which a linker is bound, an Fc(29) domain (SEQ ID NO: 4), a linker (SEQ ID NO: 39), and a PD-1 fragment (SEQ ID NO: 2), in this order, from the N-terminus. The polynucleotide was introduced into pcDNA3.4 TOPO vector (Thermo Fisher Scientific).

The expression vector was introduced into CHO cells (EXPICHO-S^{™}, Thermo Fisher Scientific) to express the fusion protein. After the CHO cells were transfected with the expression vector and cultured for 11 days using the manufacturer's Max Protocol, the culture solution was collected and the fusion protein was purified therefrom. The purified fusion protein (SEQ ID NO: 41) was designated "hII,-21-Fc(29)-hPD-1 D", and as such, the fusion protein dimer comprising PD-1 and IL-21 was collectively designated "BNS001". The purification and collection of the fusion protein were carried out in the same manner as in Preparation Example 1 above.

### Preparation Example 9. Preparation of hIL-21-Fc(29)-hPD-1 ECD

In order to produce a fusion protein comprising a human IL-21 fragment, a persistent Fc(29) domain, and a PD-1 fragment, a polynucleotide was synthesized through the Gene Synthesis service of GenScript. Specifically, the polynucleotide comprises a nucleotide sequence (SEQ ID NO: 45) which encodes a fusion protein (SEQ ID NO: 43) that comprises a signal peptide (SEQ ID NO: 20), IL-21 (SEQ ID NO: 6), an Ig hinge (SEQ ID NO: 38) to which a linker is bound, an Fc(29) domain (SEQ ID NO: 4), a linker (SEQ ID NO: 39), and a PD-1 fragment (SEQ ID NO: 34), in this order, from the N-terminus. The polynucleotide was introduced into pcDNA3.4 TOPO vector (Thermo Fisher Scientific).

The expression vector was introduced into CHO cells (EXPICHO-S^{™}, Thermo Fisher Scientific) to express the fusion protein. After the CHO cells were transfected with the expression vector and cultured for 11 days using the manufacturer's Max Protocol, the culture solution was collected and the fusion protein was purified therefrom. The purified fusion protein (SEQ ID NO: 44) was designated "hII,-21-Fc(29)-hPD-1 ECD", and as such, the fusion protein dimer comprising PD-1 and IL-21 was collectively designated "BNS001". The purification and collection of the fusion protein were carried out in the same manner as in Preparation Example 1 above.

### Preparation Example 10. Preparation of hPD-1 D-Fc(29, K)-hIL-21

In order to produce a fusion protein comprising a human PD-1 fragment, a persistent Fc(29, K) domain, and IL-21, a polynucleotide was synthesized through the Gene Synthesis service of GenScript. Specifically, the polynucleotide comprises a nucleotide sequence (SEQ ID NO: 53) which encodes a fusion protein (SEQ ID NO: 51) that comprises a signal peptide (SEQ ID NO: 1), a PD-1 fragment (SEQ ID NO: 2), an Ig hinge (SEQ ID NO: 3) to which a linker is bound, an Fc(29, K) domain (SEQ ID NO: 50), a linker (SEQ ID NO: 5), and IL-21 (SEQ ID NO: 6), in this order, from the N-terminus. The polynucleotide was introduced into pcDNA3.4 TOPO vector (Thermo Fisher Scientific).

The expression vector was introduced into CHO cells (EXPICHO-S^{™}, Thermo Fisher Scientific) to express the fusion protein. After the CHO cells were transfected with the expression vector and cultured for 11 days using the manufacturer's Max Protocol, the culture solution was collected and the fusion protein was purified therefrom. The purified fusion protein (SEQ ID NO: 52) was designated "hPD-1 D-Fc(29, K)-hII,-21", and as such, the fusion protein dimer comprising PD-1 and IL-21 was collectively designated "BNS001". The purification and collection of the fusion protein were carried out in the same manner as in Preparation Example 1 above.

### Preparation Example 11. Preparation of hPD-1 D-Fc(29, K)-mIL-21

In order to produce a fusion protein comprising a human PD-1 fragment, a persistent Fc(29, K) domain, and a mouse IL-21 fragment, a polynucleotide was synthesized through the Gene Synthesis service of GenScript. Specifically, the polynucleotide comprises a nucleotide sequence (SEQ ID NO: 56) which encodes a fusion protein (SEQ ID NO: 54) that comprises a signal peptide (SEQ ID NO: 1), a PD-1 fragment (SEQ ID NO: 2), an Ig hinge (SEQ ID NO: 3) to which a linker is bound, an Fc(29, K) domain (SEQ ID NO: 50), a linker (SEQ ID NO: 5), and IL-21 (SEQ ID NO: 22), in this order, from the N-terminus. The polynucleotide was introduced into pcDNA3.4 TOPO vector (Thermo Fisher Scientific).

The expression vector was introduced into CHO cells (EXPICHO-S^{™}, Thermo Fisher Scientific) to express the fusion protein. After the CHO cells were transfected with the expression vector and cultured for 11 days using the manufacturer's Max Protocol, the culture solution was collected and the fusion protein was purified therefrom. The purified fusion protein (SEQ ID NO: 55) was designated "hPD-1 D-Fc(29, K)-mIL-21", and as such, the fusion protein dimer comprising PD-1 and IL-21 was collectively designated "BNS001". The purification and collection of the fusion protein were carried out in the same manner as in Preparation Example 1 above.

### Preparation Example 12. Preparation of hPD-1 ECD-Fc(wt)-hIL-21

In order to produce a fusion protein comprising a human PD-1 fragment, a persistent Fc(wt) domain, and IL-21, a polynucleotide was synthesized through the Gene Synthesis service of GenScript. Specifically, the polynucleotide comprises a nucleotide sequence (SEQ ID NO: 77) which encodes a fusion protein (SEQ ID NO: 75) that comprises a signal peptide (SEQ ID NO: 1), a PD-1 fragment (SEQ ID NO: 34), an Ig hinge (SEQ ID NO: 3) to which a linker is bound, an Fc(wt) domain (SEQ ID NO: 14), a linker (SEQ ID NO: 5), and IL-21 (SEQ ID NO: 6), in this order, from the N-terminus. The polynucleotide was introduced into pcDNA3.4 TOPO vector (Thermo Fisher Scientific).

The expression vector was introduced into CHO cells (EXPICHO-S^{™}, Thermo Fisher Scientific) to express the fusion protein. After the CHO cells were transfected with the expression vector and cultured for 11 days using the manufacturer's Max Protocol, the culture solution was collected and the fusion protein was purified therefrom. The purified fusion protein (SEQ ID NO: 76) was designated "hPD-1 ECD-Fc(wt)-hIL-21", and as such, the fusion protein dimer comprising PD-1 and IL-21 was collectively designated "BNS001". The purification and collection of the fusion protein were carried out in the same manner as in Preparation Example 1 above.

### Preparation Example 13. Preparation of hIL-21-Fc(wt)-hPD-1 D

In order to produce a fusion protein comprising a human IL-21 fragment, a persistent Fc(wt) domain, and a PD-1 fragment, a polynucleotide was synthesized through the Gene Synthesis service of GenScript. Specifically, the polynucleotide comprises a nucleotide sequence (SEQ ID NO: 80) which encodes a fusion protein (SEQ ID NO: 78) that comprises a signal peptide (SEQ ID NO: 20), IL-21 (SEQ ID NO: 6), an Ig hinge (SEQ ID NO: 38) to which a linker is bound, an Fc(wt) domain (SEQ ID NO: 14), a linker (SEQ ID NO: 39), and a PD-1 fragment (SEQ ID NO: 2), in this order, from the N-terminus. The polynucleotide was introduced into pcDNA3.4 TOPO vector (Thermo Fisher Scientific).

The expression vector was introduced into CHO cells (EXPICHO-S^{™}, Thermo Fisher Scientific) to express the fusion protein. After the CHO cells were transfected with the expression vector and cultured for 11 days using the manufacturer's Max Protocol, the culture solution was collected and the fusion protein was purified therefrom. The purified fusion protein (SEQ ID NO: 79) was designated "hII,-21-Fc(wt)-hPD-1 D", and as such, the fusion protein dimer comprising PD-1 and IL-21 was collectively designated "BNS001". The purification and collection of the fusion protein were carried out in the same manner as in Preparation Example 1 above.

### Preparation Example 14. Preparation of hIL-21-Fc(wt)-hPD-1 ECD

In order to produce a fusion protein comprising a human IL-21 fragment, a persistent Fc(wt) domain, and a PD-1 fragment, a polynucleotide was synthesized through the Gene Synthesis service of GenScript. Specifically, the polynucleotide comprises a nucleotide sequence (SEQ ID NO: 83) which encodes a fusion protein (SEQ ID NO: 81) that comprises a signal peptide (SEQ ID NO: 20), IL-21 (SEQ ID NO: 6), an Ig hinge (SEQ ID NO: 38) to which a linker is bound, an Fc(wt) domain (SEQ ID NO: 14), a linker (SEQ ID NO: 39), and a PD-1 fragment (SEQ ID NO: 34), in this order, from the N-terminus. The polynucleotide was introduced into pcDNA3.4 TOPO vector (Thermo Fisher Scientific).

The expression vector was introduced into CHO cells (EXPICHO-S^{™}, Thermo Fisher Scientific) to express the fusion protein. After the CHO cells were transfected with the expression vector and cultured for 11 days using the manufacturer's Max Protocol, the culture solution was collected and the fusion protein was purified therefrom. The purified fusion protein (SEQ ID NO: 82) was designated "hII,-21-Fc(wt)-hPD-1 ECD", and as such, the fusion protein dimer comprising PD-1 and IL-21 was collectively designated "BNS001". The purification and collection of the fusion protein were carried out in the same manner as in Preparation Example 1 above.

### Preparation Example 15. Preparation of hPD-1 D-Fc(wt, K)-hIL-21

In order to produce a fusion protein comprising a human PD-1 fragment, a persistent Fc(wt, K) domain, and IL-21, a polynucleotide was synthesized through the Gene Synthesis service of GenScript. Specifically, the polynucleotide comprises a nucleotide sequence (SEQ ID NO: 90) which encodes a fusion protein (SEQ ID NO: 88) that comprises a signal peptide (SEQ ID NO: 1), a PD-1 fragment (SEQ ID NO: 2), an Ig hinge (SEQ ID NO: 3) to which a linker is bound, an Fc(wt, K) domain (SEQ ID NO: 87), a linker (SEQ ID NO: 5), and IL-21 (SEQ ID NO: 6), in this order, from the N-terminus. The polynucleotide was introduced into pcDNA3.4 TOPO vector (Thermo Fisher Scientific).

The expression vector was introduced into CHO cells (EXPICHO-S^{™}, Thermo Fisher Scientific) to express the fusion protein. After the CHO cells were transfected with the expression vector and cultured for 11 days using the manufacturer's Max Protocol, the culture solution was collected and the fusion protein was purified therefrom. The purified fusion protein (SEQ ID NO: 89) was designated "hPD-1 D-Fc(wt, K)-hIL-21", and as such, the fusion protein dimer comprising PD-1 and IL-21 was collectively designated "BNS001". The purification and collection of the fusion protein were carried out in the same manner as in Preparation Example 1 above.

### Preparation Example 16. Preparation of hPD-1 D-Fc(wt, K)-mIL-21

In order to produce a fusion protein comprising a human PD-1 fragment, a persistent Fc(wt, K) domain, and a mouse IL-21 fragment, a polynucleotide was synthesized through the Gene Synthesis service of GenScript. Specifically, the polynucleotide comprises a nucleotide sequence (SEQ ID NO: 93) which encodes a fusion protein (SEQ ID NO: 91) that comprises a signal peptide (SEQ ID NO: 1), a PD-1 fragment (SEQ ID NO: 2), an Ig hinge (SEQ ID NO: 3) to which a linker is bound, an Fc(wt, K) domain (SEQ ID NO: 87), a linker (SEQ ID NO: 5), and a mouse IL-21 fragment (SEQ ID NO: 22), in this order, from the N-terminus. The polynucleotide was introduced into pcDNA3.4 TOPO vector (Thermo Fisher Scientific).

The expression vector was introduced into CHO cells (EXPICHO-S^{™}, Thermo Fisher Scientific) to express the fusion protein. After the CHO cells were transfected with the expression vector and cultured for 11 days using the manufacturer's Max Protocol, the culture solution was collected and the fusion protein was purified therefrom. The purified fusion protein (SEQ ID NO: 92) was designated "hPD-1 D-Fc(wt, K)-mIL-21", and as such, the fusion protein dimer comprising PD-1 and IL-21 was collectively designated "BNS001". The purification and collection of the fusion protein were carried out in the same manner as in Preparation Example 1 above.

### Preparation Example 17. Preparation of hPD-1 D-Fc(29)R-hIL-21

In order to produce a fusion protein comprising a human PD-1 fragment, a persistent Fc(29)R domain, and a human IL-21 fragment, a polynucleotide was synthesized through the Gene Cloning service of Cosmogenetech Co, Ltd. Specifically, the polynucleotide comprises a nucleotide sequence (SEQ ID NO: 98) which encodes a fusion protein (SEQ ID NO: 96) that comprises a signal peptide (SEQ ID NO: 94), a PD-1 fragment (SEQ ID NO: 2), an Ig hinge (SEQ ID NO: 3) to which a linker is bound, an Fc(29)R domain (SEQ ID NO: 95), a linker (SEQ ID NO: 5), and an IL-21 fragment (SEQ ID NO: 6), in this order, from the N-terminus. The polynucleotide was introduced into UCOE-GS vector (Merck KGaA).

The expression vector was introduced into CHO cells (EXPICHO-S^{™}, Thermo Fisher Scientific) to express the fusion protein. After the CHO cells were transfected with the expression vector and cultured for 7 days using the manufacturer's Max Protocol, the culture solution was collected and the fusion protein was purified therefrom. The purified fusion protein (SEQ ID NO: 97) was designated "hPD-1 D-Fc(29)R-hIL-21", and as such, the fusion protein dimer comprising PD-1 and IL-21 was collectively designated "BNS001R".

The fusion protein in the collected culture solution was subjected to first purification using affinity chromatography containing Protein A resin (Cytiva). The bound fusion protein and resin were washed with DPBS buffer, and then only the bound fusion protein was eluted with 0.1 M glycine buffer (pH 3.3). The eluted fusion protein was dialyzed against DPBS buffer for one day to exchange the buffer, and then quantified using NanoDrop, and SDS-PAGE analysis was performed.

### Preparation Example 18. Preparation of hPD-1 D-Fc(29)RE-hIL-21

In order to produce a fusion protein comprising a human PD-1 fragment, a persistent Fc(29)RE domain, and a human IL-21 fragment, a polynucleotide was synthesized through the Gene Cloning service of Cosmogenetech Co, Ltd. Specifically, the polynucleotide comprises a nucleotide sequence (SEQ ID NO: 102) which encodes a fusion protein (SEQ ID NO: 100) that comprises a signal peptide (SEQ ID NO: 94), a PD-1 fragment (SEQ ID NO: 2), an Ig hinge (SEQ ID NO: 3) to which a linker is bound, an Fc(29)RE domain (SEQ ID NO: 99), a linker (SEQ ID NO: 5), and an IL-21 fragment (SEQ ID NO: 6), in this order, from the N-terminus. The polynucleotide was introduced into UCOE-GS vector (Merck KGaA).

The expression vector was introduced into CHO cells (EXPICHO-S^{™}, Thermo Fisher Scientific) to express the fusion protein. After the CHO cells were transfected with the expression vector and cultured for 7 days using the manufacturer's Max Protocol, the culture solution was collected and the fusion protein was purified therefrom. The purified fusion protein (SEQ ID NO: 101) was designated "hPD-1 D-Fc(29)RE-hII,-21", and as such, the fusion protein dimer comprising PD-1 and IL-21 was collectively designated "BNS001RE". The purification and collection of the fusion protein were carried out in the same manner as in Preparation Example 17 above.

### Preparation Example 19. Preparation of hPD-1v D-Fc(29)REA-hIL-21

In order to produce a fusion protein comprising a human PD-1 variant fragment, a persistent Fc(29)REA domain, and a human IL-21 fragment, a polynucleotide was synthesized through the Gene Cloning service of Cosmogenetech Co, Ltd. Specifically, the polynucleotide comprises a nucleotide sequence (SEQ ID NO: 107) which encodes a fusion protein (SEQ ID NO: 105) that comprises a signal peptide (SEQ ID NO: 94), PD-1v fragment (SEQ ID NO: 103), an Ig hinge (SEQ ID NO: 3) to which a linker is bound, an Fc(29)REA domain (SEQ ID NO: 104), a linker (SEQ ID NO: 5), and an IL-21 fragment (SEQ ID NO: 6), in this order, from the N-terminus. The polynucleotide was introduced into UCOE-GS vector (Merck KGaA).

The expression vector was introduced into CHO cells (EXPICHO-S^{™}, Thermo Fisher Scientific) to express the fusion protein. After the CHO cells were transfected with the expression vector and cultured for 7 days using the manufacturer's Max Protocol, the culture solution was collected and the fusion protein was purified therefrom. The purified fusion protein (SEQ ID NO: 106) was designated "hPD-1v D-Fc(29)REA-hIL-21", and as such, the fusion protein dimer comprising PD-1 and IL-21 was collectively designated "BNS001REA". The purification and collection of the fusion protein were carried out in the same manner as in Preparation Example 17 above.

### Preparation Example 20. Preparation of hPD-1 D-Fc(wt)R-hIL-21

In order to produce a fusion protein comprising a human PD-1 fragment, a persistent Fc(wt)R domain, and a human IL-21 fragment, a polynucleotide was synthesized through the Gene Cloning service of Cosmogenetech Co, Ltd. Specifically, the polynucleotide comprises a nucleotide sequence (SEQ ID NO: 111) which encodes a fusion protein (SEQ ID NO: 109) that comprises a signal peptide (SEQ ID NO: 94), a PD-1 fragment (SEQ ID NO: 2), an Ig hinge (SEQ ID NO: 3) to which a linker is bound, an Fc(wt)R domain (SEQ ID NO: 108), a linker (SEQ ID NO: 5), and an IL-21 fragment (SEQ ID NO: 6), in this order, from the N-terminus. The polynucleotide was introduced into UCOE-GS vector (Merck KGaA).

The expression vector was introduced into CHO cells (EXPICHO-S^{™}, Thermo Fisher Scientific) to express the fusion protein. After the CHO cells were transfected with the expression vector and cultured for 7 days using the manufacturer's Max Protocol, the culture solution was collected and the fusion protein was purified therefrom. The purified fusion protein (SEQ ID NO: 110) was designated "hPD-1 D-Fc(wt)R-hIL-21", and as such, the fusion protein dimer comprising PD-1 and IL-21 was collectively designated "BNS001wR". The purification and collection of the fusion protein were carried out in the same manner as in Preparation Example 17 above.

### Preparation Example 21. Preparation of hPD-1 D-Fc(wt)RE-hIL-21

In order to produce a fusion protein comprising a human PD-1 fragment, a persistent Fc(wt)RE domain, and a human IL-21 fragment, a polynucleotide was synthesized through the Gene Cloning service of Cosmogenetech Co, Ltd. Specifically, the polynucleotide comprises a nucleotide sequence (SEQ ID NO: 115) which encodes a fusion protein (SEQ ID NO: 113) that comprises a signal peptide (SEQ ID NO: 94), a PD-1 fragment (SEQ ID NO: 2), an Ig hinge (SEQ ID NO: 3) to which a linker is bound, an Fc(wt)RE domain (SEQ ID NO: 112), a linker (SEQ ID NO: 5), and an IL-21 fragment (SEQ ID NO: 6), in this order, from the N-terminus. The polynucleotide was introduced into UCOE-GS vector (Merck KGaA).

The expression vector was introduced into CHO cells (EXPICHO-S^{™}, Thermo Fisher Scientific) to express the fusion protein. After the CHO cells were transfected with the expression vector and cultured for 7 days using the manufacturer's Max Protocol, the culture solution was collected and the fusion protein was purified therefrom. The purified fusion protein (SEQ ID NO: 114) was designated "hPD-1 D-Fc(wt)RE-hII,-21", and as such, the fusion protein dimer comprising PD-1 and IL-21 was collectively designated "BNS001wRE". The purification and collection of the fusion protein were carried out in the same manner as in Preparation Example 17 above.

### Preparation Example 22. Preparation of hPD-1v D-Fc(wt)REA-hIL-21

In order to produce a fusion protein comprising a human PD-1 variant fragment, a persistent Fc(wt)REA domain, and a human IL-21 fragment, a polynucleotide was synthesized through the Gene Cloning service of Cosmogenetech Co, Ltd. Specifically, the polynucleotide comprises a nucleotide sequence (SEQ ID NO: 119) which encodes a fusion protein (SEQ ID NO: 117) that comprises a signal peptide (SEQ ID NO: 94), PD-1v fragment (SEQ ID NO: 103), an Ig hinge (SEQ ID NO: 3) to which a linker is bound, an Fc(wt)REA domain (SEQ ID NO: 116), a linker (SEQ ID NO: 5), and an IL-21 fragment (SEQ ID NO: 6), in this order, from the N-terminus. The polynucleotide was introduced into UCOE-GS vector (Merck KGaA).

The expression vector was introduced into CHO cells (EXPICHO-S^{™}, Thermo Fisher Scientific) to express the fusion protein. After the CHO cells were transfected with the expression vector and cultured for 7 days using the manufacturer's Max Protocol, the culture solution was collected and the fusion protein was purified therefrom. The purified fusion protein (SEQ ID NO: 118) was designated "hPD-1v D-Fc(wt)REA-hIL-21", and as such, the fusion protein dimer comprising PD-1 and IL-21 was collectively designated "BNS001wREA". The purification and collection of the fusion protein were carried out in the same manner as in Preparation Example 17 above.

### II. Purity analysis of BNS001 fusion protein

### Experimental Example 1. SEC-HPLC analysis

In order to identify the purity of the isolated and purified fusion protein, SEC-HPLC experiment was performed. Size exclusion HPLC (high performance liquid chromatography) was performed on a Waters Alliance HPLC system with UV detector. The column (TSK G3000 SW_{XL}, 5 µm SEC column) was purchased from Tosoh Bioscience. 100 mM NaPi, 150 mM NaCl (pH 7.0) were used as mobile phases. The purity of the fusion protein was analyzed by measuring the absorbance at a wavelength of 280 nm over time at a flow rate of 0.5 mL/min under 100% isocratic transfer conditions (Figure 2).

The isolated and purified BNS001 fusion protein was identified to have a purity of 98% or more (Figure 2).

### III. Concentration analysis of BNS001 fusion protein

### Experimental Example 2. Concentration analysis

The fusion protein was diluted to concentrations of 0.2, 0.4, 0.6, 0.8, and 1.0 mg/mL. UV absorbance was measured at a wavelength range of 230 nm to 400 nm using a UV spectrophotometer (Little lunatic, Unchained Labs). The fusion protein at each concentration was diluted to a concentration of 0.2 mg/mL, and then acid hydrolysis was performed. The hydrolyzed amino acids were derivatized, and UPLC (ultra performance liquid chromatography) was performed using a Waters ACQUITY UPLC system. The column used was an AccQ-Tag Ultra RP column (2.1 mm × 100 mm, 1.7 µm, 130 Å). Mobile phase A used was 5% AccQ-tag A solvent, and mobile phase B used was AccQ-tag B solvent, and a gradient system was performed using an A:B ratio starting at 99.9:0.1 and ending at 40.4:59.6 over 7.5 minutes. The sample injection volume was 1 µl, the flow rate was 0.7 mL/min, and the column temperature was 55°C. The data system used Enpower 3 Software, and the data were fit to the standard solution (I.S.) added in the sample pretreatment step.

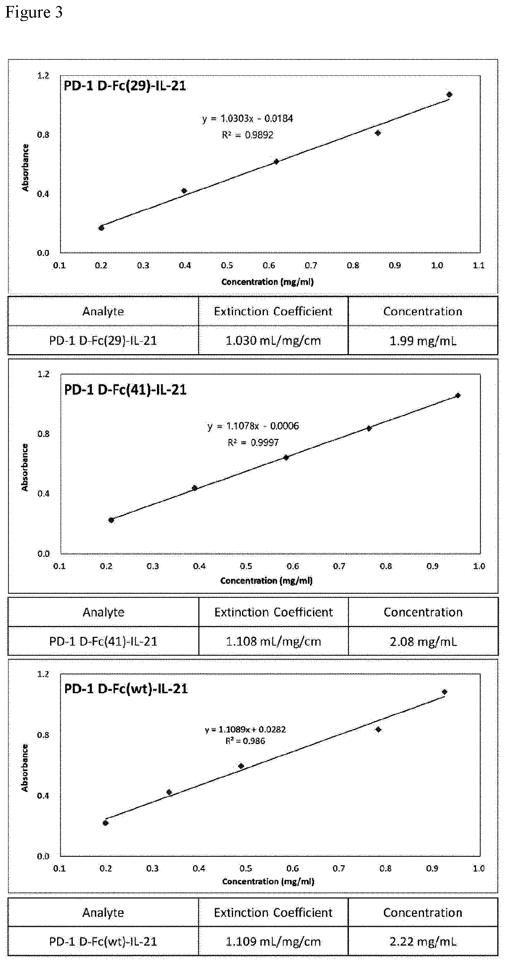

The extinction coefficient was determined by checking the content according to the absorbance of the isolated and purified BNS001 fusion protein. The concentration of the fusion protein was identified using the extinction coefficient, and it was identified that the fusion protein was contained at a concentration of 2.08 to 2.22 mg/mL (Figure 3).

### IV. Physical property analysis of BNS001 fusion protein

### Experimental Example 3. Thermodynamic stability analysis

In order to identify the thermodynamic stability of the isolated and purified fusion protein, Tₘ, T_{agg} & Tₒₙₛₑₜ analysis was performed using an UNCHAINED LABS UNcle spectrophotometer system with a fluorescence, static light scattering (SLS), and dynamic light scattering (DLS) detector. The buffer for the fusion protein was exchanged using ultrapure water, and the sample was prepared so that the protein concentration was 1.25 times the protein concentration required for analysis using an UNCHAINED LABS Little Lunatic equipment. The prepared sample was diluted 1.25× and the formulation buffer was diluted 5× using ultrapure water. The prepared sample was loaded into Uni (UNCHAINED LABS), and Tₘ, T_{agg} & Tₒₙₛₑₜ were measured with a fluorescence & SLS detector at a wavelength of 266 nm at a temperature from 15 to 95°C. The data were analyzed using an UNCHAINED LABS software Uncle Analysis V4.01.

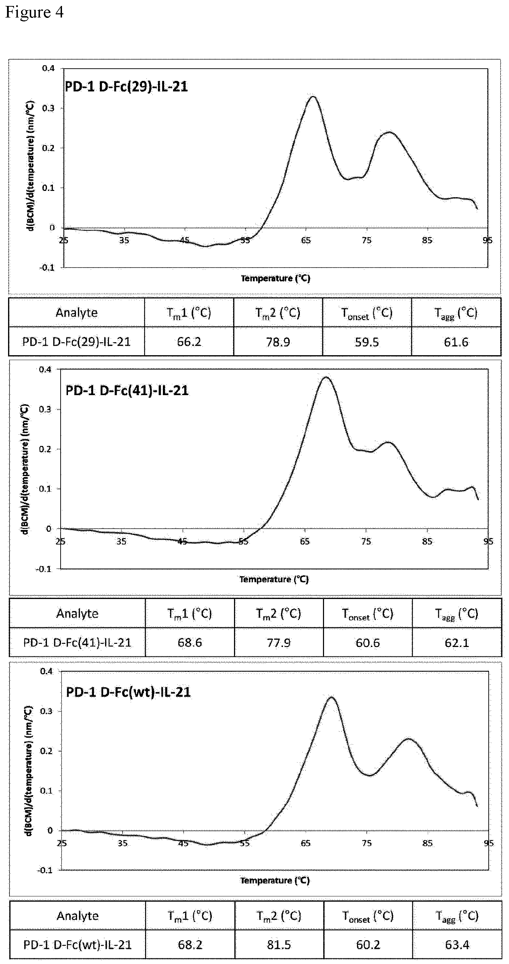

The thermodynamic stability of the isolated and purified BNS001 fusion protein was measured as shown in Figure 4.

### Experimental Example 4. Structural stability analysis

In order to identify the structural stability of the isolated and purified fusion protein, secondary structure analysis was performed using an FT-IR spectrophotometer (Thermo Nicolet IS50, Single-Bounce ATR Diamond Crystal attached). Samples were prepared so that the fusion protein concentration was 10 mg/mL. The analysis conditions of the FT-IR equipment were set to phase resolution of 4 cm⁻¹ and data spacing of 1 cm⁻¹, and measurements were repeated 128 times in the range of 500 to 4000 cm⁻¹. In order to analyze the secondary structure content of the fusion protein, zero order FT-IR spectrum data in the range of 1700 to 1600 cm⁻¹ was used. At this time, as analysis was performed using the ATR sampling accessory, ATR correction was performed on the initially acquired FT-IR spectrum data, and the water vapor band at 2300 cm⁻¹ was removed from the subtracted spectrum obtained after correction. In addition, the final zero order FT-IR spectrum was obtained by baseline correction and fitting with an 11-point Savitzky-Golay function. Among the zero order FT-IR spectra obtained through FT-IR analysis, data in the range of 1700 to 1600 cm⁻¹ were calculated using a linear-fit function to obtain a second derivative spectrum. The peaks were extracted from the second derivative spectrum, and the extracted peaks were fit with a Gaussian function. The size and position of the peak were adjusted with a tolerance error of 3% to obtain the final extracted spectrum. Secondary structures were assigned to peaks extracted from the second derivative spectrum according to wavenumber, and the peak area was calculated for the assigned peaks to calculate the secondary structure content in each sample.

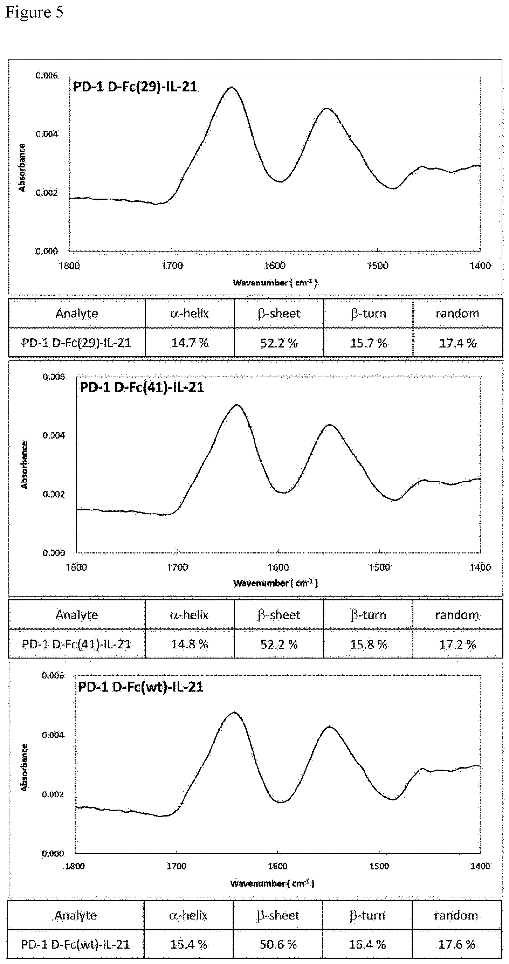

Secondary structure analysis of the isolated and purified BNS001 fusion protein was measured as shown in Figure 5.

### Experimental Example 5. Molecular weight analysis

The molecular weight of the fusion protein was identified through Matrix Assisted Laser Desorption/Ionization Time of Flight (MALDI-TOF) mass spectrometry. DPBS (pH 7.15) was used as a buffer, and the concentration of the fusion protein was diluted to 1 mg/mL and measured using the MALDI-TOF/TOF^{™}5800 system (AB SCIEX). The positive ion linear mode was used as a mode, and a mixture ofIgG 1 (charge +2, average 74,249) and IgG1 (charge +1, average 148,500) was used as the standard material. The matrix used was sinapinic acid (0.1% TFA/30% ACN) at a concentration of 10 mg/mL, and the fusion protein sample was mixed with the matrix in a 1:5 ratio after pretreatment with a zip-tip, and 6 spots were injected into a plate. The data were analyzed using a Data explorer version 4.11 (AB SCIEX), and the data were derived by accumulating six times in the program. The molecular weight range (m/z) was measured from 20 to 200 kDa.

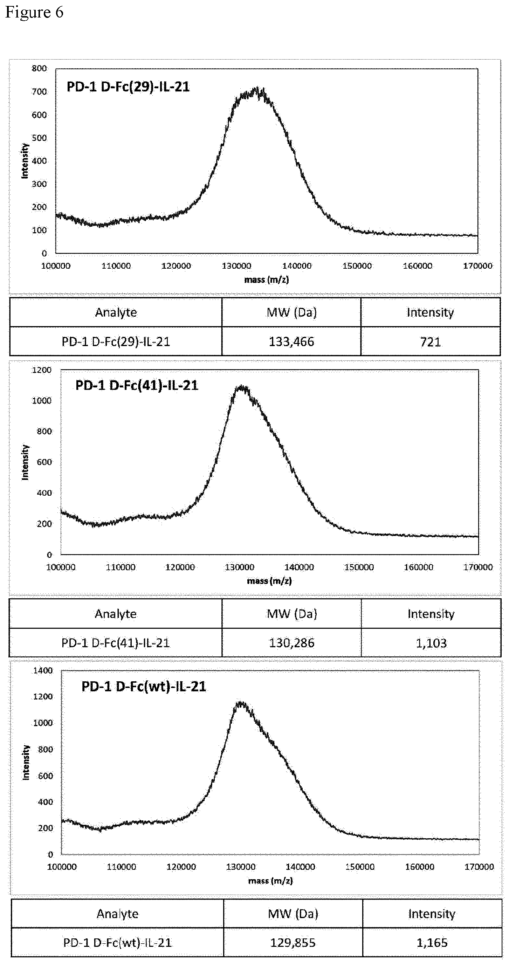

MALDI-TOF analysis of the isolated and purified BNS001 fusion protein was measured as shown in Figure 6.

### V. Identification of kinetic binding affinity between BNS001 fusion protein and ligand or receptor

In order to identify the kinetic binding affinity of the BNS001 fusion protein and the ligand, the binding affinity was measured by surface plasmon resonance (SPR) analysis using Biacore T200 (GE Healthcare).

### Experimental Example 6. Identification of binding affinity between hPD-L1 ligand and BNS001 fusion protein

PD-L1 ligand was diluted to 3 to 4 µg/mL in C₂H₃NaO₂ (pH 4.0, pH 5.5) and immobilized to about 1000 RU using amine coupling on a CM5 sensor chip (GE Healthcare) pre-activated with HBS-EP, pH 7.4. Sensorgrams were recorded by flowing diluted solutions of the BNS001 fusion protein prepared with HBS-EP, pH 7.4, in various concentration ranges from 0.78 nM to 200 nM. Binding between the BNS001 fusion protein and the PD-L1 ligand was measured at a flow rate of 30 µl/min with a 5-minute association period and a 10-minute dissociation period. The sensor chip surface was regenerated by injecting 10 mM NaOH between each run. Binding kinetics was analyzed using data analysis software (Ver3.2), and the data were fit using a 1:1 binding model.

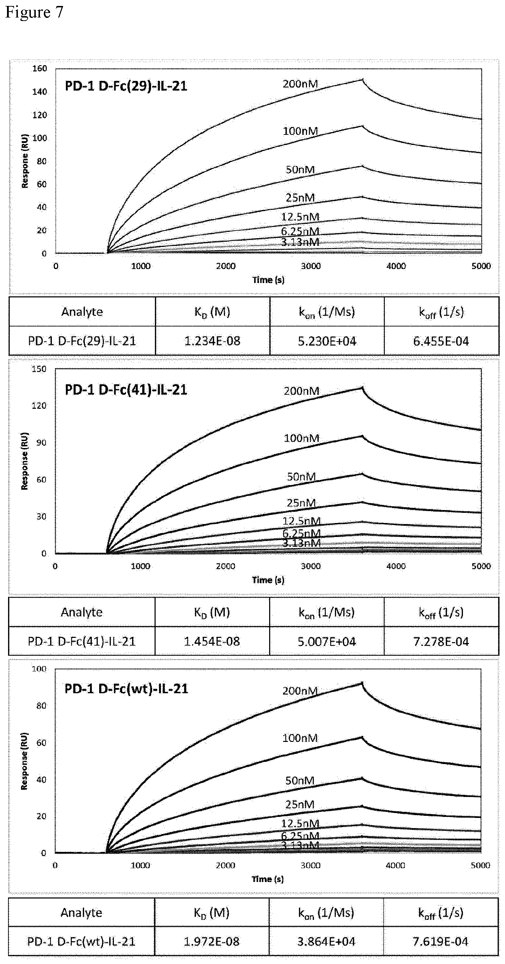

As a result, the binding affinity between the hPD-L1 ligand and the BNS001 fusion protein was measured as shown in Figure 7.

### Experimental Example 7. Identification of binding affinity between hPD-L1 ligand and hPD-1 protein

In order to identify the strength of the binding affinity between the BNS001 fusion protein and the hPD-L1 ligand, the binding affinity between the hPD-1 protein and the hPD-L1 ligand was compared. PD-L1 ligand was diluted to 3 to 4 µg/mL in C₂H₃NaO₂ (pH 4.0, pH 5.5) and immobilized to about 1000 RU using amine coupling on a CM5 sensor chip (GE Healthcare) pre-activated with HBS-EP, pH 7.4. Sensorgrams were recorded by flowing diluted solutions of the hPD-1 protein prepared with HBS-EP, pH 7.4, in various concentration ranges from 0.78 nM to 200 nM. Binding between the hPD-L1 ligand and the hPD-1 protein was measured at a flow rate of 30 µl/min with a 5-minute association period and a 10-minute dissociation period. The sensor chip surface was regenerated by injecting 10 mM NaOH between each run. Binding kinetics was analyzed using data analysis software (Ver3.2), and the data were fit using a 1:1 binding model.

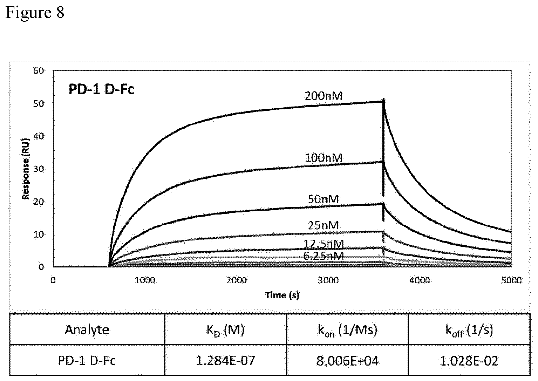

As a result, the binding affinity between the hPD-L1 ligand and the hPD-1 protein was measured as shown in Figure 8. The binding affinity between the BNS001 fusion protein and the hPD-L1 ligand was identified to be approximately 10 times higher than the binding affinity between the hPD-1 protein and the hPD-L1 ligand.

### Experimental Example 8. Identification of binding affinity between hPD-L2 ligand and BNS001 fusion protein

PD-L2 ligand was diluted to 3 to 4 µg/mL in C₂H₃NaO₂ (pH 4.0, pH 5.5) and immobilized to about 1000 RU using amine coupling on a CM5 sensor chip (GE Healthcare) pre-activated with HBS-EP, pH 7.4. Sensorgrams were recorded by flowing diluted solutions of the BNS001 fusion protein prepared with HBS-EP, pH 7.4, in various concentration ranges from 0.78 nM to 200 nM. Binding between the BNS001 fusion protein and the PD-L2 ligand was measured at a flow rate of 30 µl/min with a 5-minute association period and a 10-minute dissociation period. The sensor chip surface was regenerated by injecting 10 mM NaOH between each run. Binding kinetics was analyzed using data analysis software (Ver3.2), and the data were fit using a 1:1 binding model.

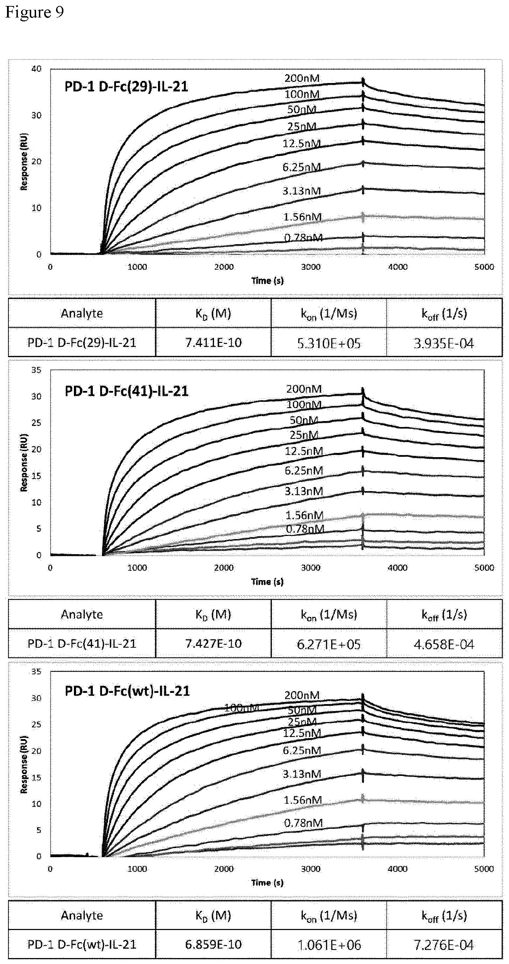

As a result, the binding affinity between the hPD-L2 ligand and the BNS001 fusion protein was measured as shown in Figure 9.

### Experimental Example 9. Identification of binding affinity between hPD-L2 ligand and hPD-1 protein

In order to identify the strength of the binding affinity between the BNS001 fusion protein and the hPD-L2 ligand, the binding affinity between the hPD-1 protein and the hPD-L2 ligand was compared. PD-L2 ligand was diluted to 3 to 4 µg/mL in C₂H₃NaO₂ (pH 4.0, pH 5.5) and immobilized to about 1000 RU using amine coupling on a CM5 sensor chip (GE Healthcare) pre-activated with HBS-EP, pH 7.4. Sensorgrams were recorded by flowing diluted solutions of the hPD-1 protein prepared with HBS-EP, pH 7.4, in various concentration ranges from 0.78 nM to 200 nM. Binding between the hPD-L2 ligand and the hPD-1 protein was measured at a flow rate of 30 µl/min with a 5-minute association period and a 10-minute dissociation period. The sensor chip surface was regenerated by injecting 10 mM NaOH between each run. Binding kinetics was analyzed using data analysis software (Ver3.2), and the data were fit using a 1:1 binding model.

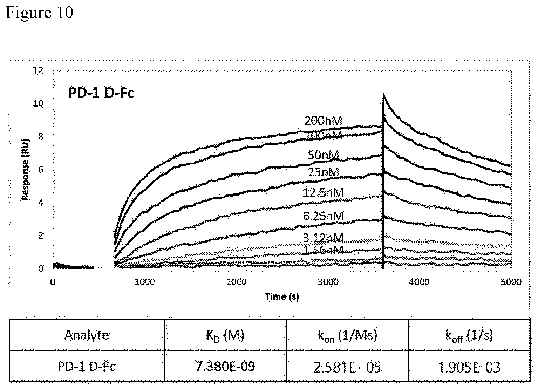

As a result, the binding affinity between the hPD-L2 ligand and the hPD-1 protein was measured as shown in Figure 10. The binding affinity between the BNS001 fusion protein and the hPD-L2 ligand was identified to be approximately 10 times higher than the binding affinity between the hPD-1 protein and the hPD-L2 ligand.

### Experimental Example 10. Identification of binding affinity between hIL-21 receptor and BNS001 fusion protein

hIL-21 receptor was diluted to 3 to 4 µg/mL in C₂H₃NaO₂ (pH 4.0, pH 5.5) and immobilized to about 1000 RU using amine coupling on a CM5 sensor chip (GE Healthcare) pre-activated with HBS-EP, pH 7.4. Sensorgrams were recorded by flowing diluted solutions of the BNS001 fusion protein prepared with HBS-EP, pH 7.4, in various concentration ranges from 0.78 nM to 200 nM. Binding between the BNS001 fusion protein and the hIL-21 receptor was measured at a flow rate of 30 µl/min with a 5-minute association period and a 10-minute dissociation period. The sensor chip surface was regenerated by injecting 10 mM NaOH between each run. Binding kinetics was analyzed using data analysis software (Ver3.2), and the data were fit using a 1:1 binding model.

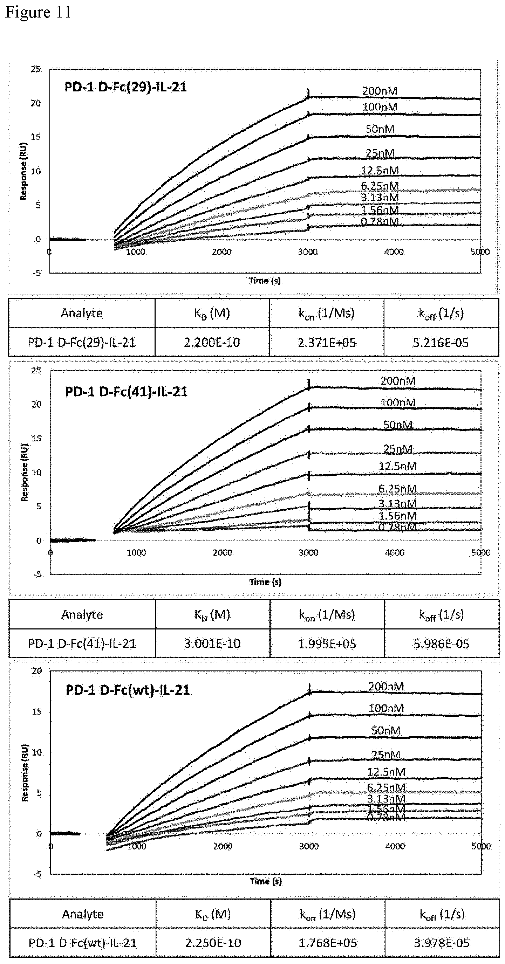

As a result, the binding affinity between the hIL-21 receptor and the BNS001 fusion protein was measured as shown in Figure 11.

### Experimental Example 11. Identification of binding affinity between hIL-21 receptor and hIL-21 protein

In order to identify the strength of the binding affinity between the BNS001 fusion protein and the hIL-21 receptor, the binding affinity between the hIL-21 protein and the hIL-21 receptor was compared. hIL-21 receptor was diluted to 3 to 4 µg/mL in C₂H₃NaO₂ (pH 4.0, pH 5.5) and immobilized to about 1000 RU using amine coupling on a CM5 sensor chip (GE Healthcare) pre-activated with HBS-EP, pH 7.4. Sensorgrams were recorded by flowing diluted solutions of the hIL-21 protein prepared with HBS-EP, pH 7.4, in various concentration ranges from 0.04 nM to 5 nM. Binding between the hIL-21 receptor and the hIL-21 protein was measured at a flow rate of 30 µl/min with a 5-minute association period and a 10-minute dissociation period. The sensor chip surface was regenerated by injecting 10 mM NaOH between each run. Binding kinetics was analyzed using data analysis software (Ver3.2), and the data were fit using a 1:1 binding model.

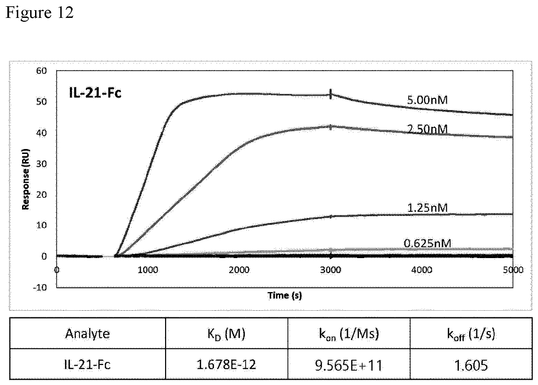

As a result, the binding affinity between the hIL-21 receptor and the hIL-21 protein was measured as shown in Figure 12. The binding affinity between the BNS001 fusion protein and the hIL-21 receptor was identified to be approximately 100 times lower than the binding affinity between the hIL-21 protein and the hIL-21 receptor.

### Experimental Example 12. Identification of binding affinity between hFcRn receptor and BNS001 fusion protein

hFcRn receptor was diluted to 5 µg/mL in C₂H₃NaO₂ (pH 5.0) and immobilized to about 1000 RU using amine coupling on a CM5 sensor chip (GE Healthcare) pre-activated with 20 mM phosphate, 150 mM NaCl, pH 6.0 and pH 7.4. Sensorgrams were recorded by flowing diluted solutions of the fusion protein prepared with 20 mM phosphate, 150 mM NaCl, 0.05% Tween20, pH 6.0, in various concentration ranges from 0.78 nM to 100 nM. Binding with the fusion protein was measured at a flow rate of 30 µl/min with a 4-minute association period and a 10-minute dissociation period. The sensor chip surface was regenerated by injecting 50 mM NaOH between each run. Binding kinetics was analyzed using data analysis software (Ver3.2), and the data were fit using a 1:1 binding model.

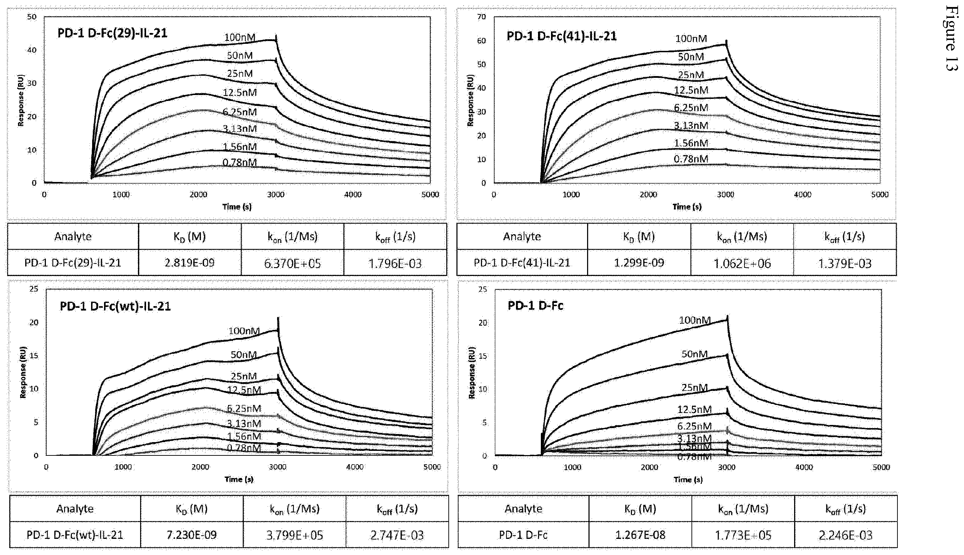

As a result, the binding affinity between the hFcRn receptor and the BNS001 fusion protein was measured as shown in Figure 13. Among the BNS001 fusion proteins, PD-1 D-Fc(29)-IL-21 and PD-1 D-Fc(41)-IL-21 proteins were identified to have a stronger binding affinity for the hFcRn receptor than that of PD-1 D-Fc(wt)-IL-21 and PD-1 D-Fc proteins.

### VI. Identification of immunological binding affinity between BNS001 fusion protein and ligand or receptor

In order to identify the binding affinity between the BNS001 fusion protein and the ligand, the binding affinity was measured by immunological analysis.

### Experimental Example 13. Identification of binding affinity between BNS001 fusion protein and PD-L1 ligand through ELISA analysis

0.1 mL of PD-L1-Fc ligand at a concentration of 100 ng/mL was immobilized on a 96 well plate (High binding plate, Corning, #CT9018) for 16 hours at 4°C, and then blocked with 250 µl of blocking buffer for 2 hours at room temperature. After blocking, the washing process was performed four times with 300 µl of washing buffer. Next, the serially diluted BNS001 fusion protein was divided into 50 µl aliquots, and then 50 µl of sample diluent was added. 50 µl/well of detection antibody (biotinylated goat anti-human IL-21 Ab) diluted to 25 ng/mL was added and reacted at room temperature for 2 hours. After the reaction was completed, the plate was washed four times with 400 µl of washing buffer, and the remaining solution was completely removed. 100 µl of streptavidin-HRP diluted at a ratio of 1:200 using 1× assay buffer was added to the well and reacted at room temperature for 1 hour. After the reaction was completed, the plate was washed four times with 400 µl of washing buffer, and the remaining solution was completely removed. After treating each well with 100 µl of TMB solution, the reaction was performed at room temperature for 30 minutes while blocking light. After reaction, 100 µl of stop solution was added to each well, and the absorbance was measured using a plate reader (wavelength: 450 nm). At this time, each experiment was conducted in duplicate.

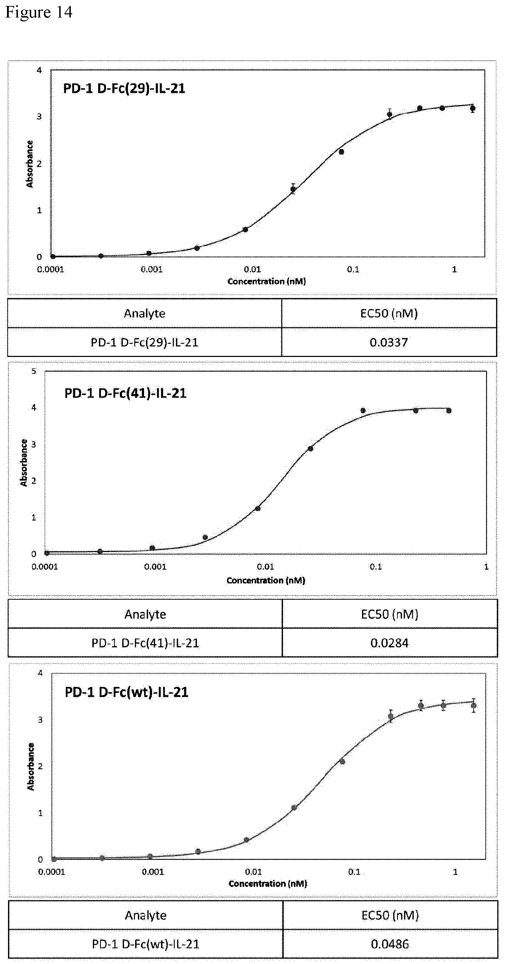

As a result of ELISA analysis, the binding affinity between the BNS001 fusion protein and the PD-L1 ligand was measured as shown in Figure 14.

### Experimental Example 14. Identification of binding affinity between BNS001 fusion protein and PD-L2 ligand through ELISA analysis

0.1 mL of PD-L2-Fc ligand at a concentration of 100 ng/mL was immobilized on a 96 well plate (High binding plate, Corning, #CT9018) for 16 hours at 4°C, and then blocked with 250 µl of blocking buffer for 2 hours at room temperature. After blocking, the washing process was performed four times with 300 µl of washing buffer. Next, the serially diluted BNS001 fusion protein was divided into 50 µl aliquots, and then 50 µl of sample diluent was added. 50 µl/well of detection antibody (biotinylated goat anti-human IL-21 Ab) diluted to 25 ng/mL was added and reacted at room temperature for 2 hours. After the reaction was completed, the plate was washed four times with 400 µl of washing buffer, and the remaining solution was completely removed. 100 µl of streptavidin-HRP diluted at a ratio of 1:200 using 1× assay buffer was added to the well and reacted at room temperature for 1 hour. After the reaction was completed, the plate was washed four times with 400 µl of washing buffer, and the remaining solution was completely removed. After treating each well with 100 µl of TMB solution, the reaction was performed at room temperature for 30 minutes while blocking light. After reaction, 100 µl of stop solution was added to each well, and the absorbance was measured using a plate reader (wavelength: 450 nm). At this time, each experiment was conducted in duplicate.

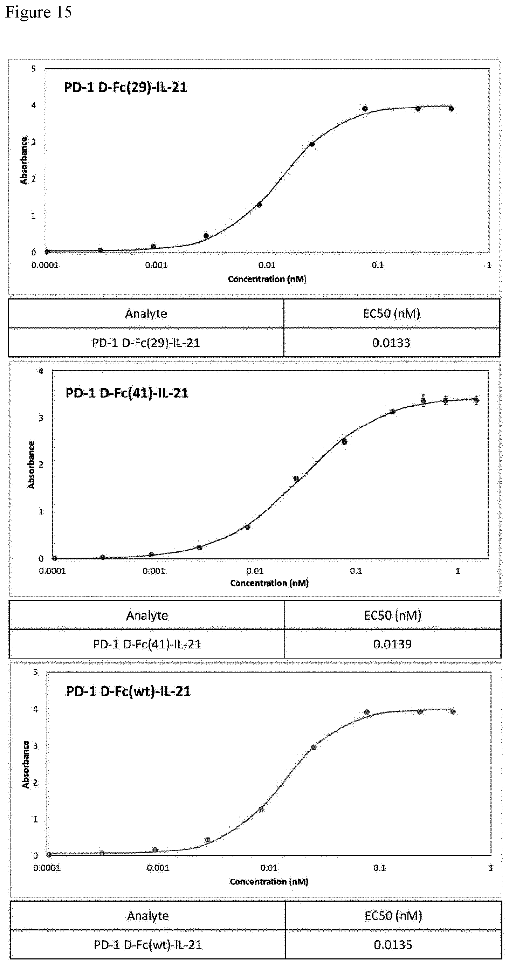

As a result of ELISA analysis, the binding affinity between the BNS001 fusion protein and the PD-L2 ligand was measured as shown in Figure 15.

### Experimental Example 15. Identification of binding affinity between BNS001 fusion protein and IL-21 receptor through ELISA analysis

0.1 mL of IL-21 receptor at a concentration of 100 ng/mL was immobilized on a 96 well plate (High binding plate, Corning, #CT9018) for 16 hours at 4°C, and then blocked with 250 µl of blocking buffer for 2 hours at room temperature. After blocking, the washing process was performed four times with 300 µl of washing buffer. Next, the serially diluted BNS001 fusion protein was divided into 50 µl aliquots, and then 50 µl of sample diluent was added. 50 µl/well of detection antibody (biotinylated goat anti-human PD-1 Ab) diluted to 25 ng/mL was added and reacted at room temperature for 2 hours. After the reaction was completed, the plate was washed four times with 400 µl of washing buffer, and the remaining solution was completely removed. 100 µl of streptavidin-HRP diluted at a ratio of 1:200 using 1× assay buffer was added to the well and reacted at room temperature for 1 hour. After the reaction was completed, the plate was washed four times with 400 µl of washing buffer, and the remaining solution was completely removed. After treating each well with 100 µl of TMB solution, the reaction was performed at room temperature for 30 minutes while blocking light. After reaction, 100 µl of stop solution was added to each well, and the absorbance was measured using a plate reader (wavelength: 450 nm). At this time, each experiment was conducted in duplicate.

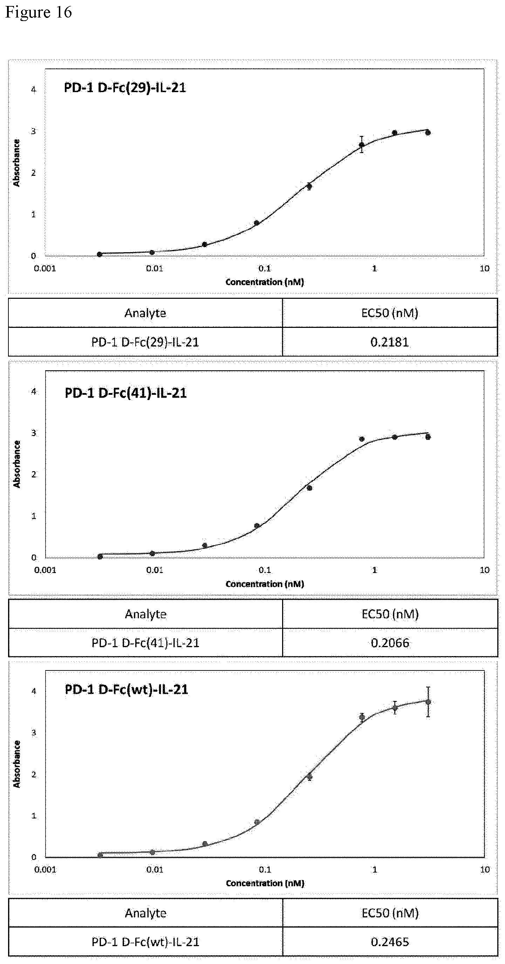

As a result of ELISA analysis, the binding affinity between the BNS001 fusion protein and the IL-21 receptor was measured as shown in Figure 16.

### Experimental Example 16. Identification of binding affinity between BNS001 fusion protein and FcRn receptor through FcRn binding immunoassay

In order to measure the binding force of the BNS001 fusion protein to FcRn (neonatal Fc receptor), which can exhibit the effector function on FcRn (neonatal Fc receptor), analysis was performed using the Promega's LUMIT^{™} FcRn binding immunoassay kit. Since FcRn is known to have pH-dependent binding force, the BNS001 fusion proteins were adjusted to pH 6.0 using a pH adjustment buffer and prepared by diluting with FcRn assay buffer at respective concentrations. 25 µl of the Tracer-LgBit solution in the kit was dispensed into each well, and 25 µl of the BNS001 fusion proteins diluted at respective concentrations were dispensed into each well. Thereafter, 50 µl of the hFcRn-SmBiT solution in the kit was dispensed into each well and reacted at room temperature for 1 hour, and 25 µl of the LUMIT^{™} FcRn Detection reagent was added to each well and reacted for 5 minutes. After reaction, it was analyzed using Synergy Neo2 (BioTek).

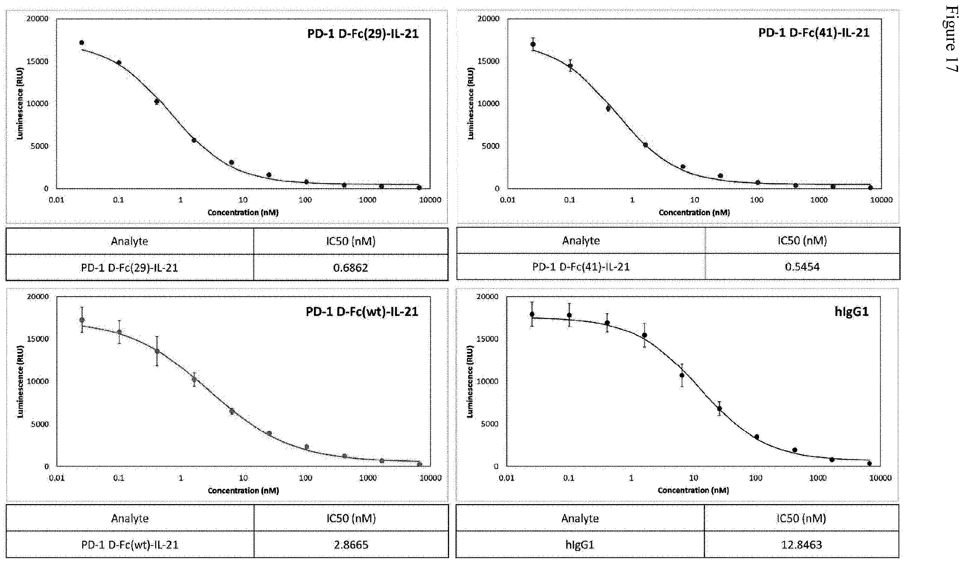

As a result of LUMIT^{™} FcRn binding immunoassay analysis, the binding affinity between the BNS001 fusion protein and the FcRn receptor was measured as shown in Figure 17.

### VII. Identification of binding affinity between BNS001 fusion protein and IL-21 receptor through cell-based assay

### Experimental Example 17. Identification of cell-based binding affinity between BNS001 fusion protein and IL-21 receptor

The binding affinity of the BNS001 fusion protein to the IL-21 receptor was measured using the PATHHUNTER^{®} eXpress Dimerization Assay Kit (Eurofins), and PATHHUNTER^{®} eXpress Dimerization Cells stored in liquid nitrogen were thawed using the warmed cell plating reagent. The thawed cells were dispensed at 100 µl each into a 96 well plate (SPL), treated with the BNS001 fusion protein at respective concentrations, and then each well was treated with 110 µl of the PATHHUNTER^{®} Flash Detection reagent. After treatment, the reaction was performed for 1 hour at room temperature and dark conditions. After reaction, chemiluminescence was measured using Synergy Neo2 (BioTek). Based on the measured values, the EC₅₀ (half maximal effective concentration; the concentration of the drug that show about half the maximum effect that the drug can have when administered) values for the fusion proteins were identified.

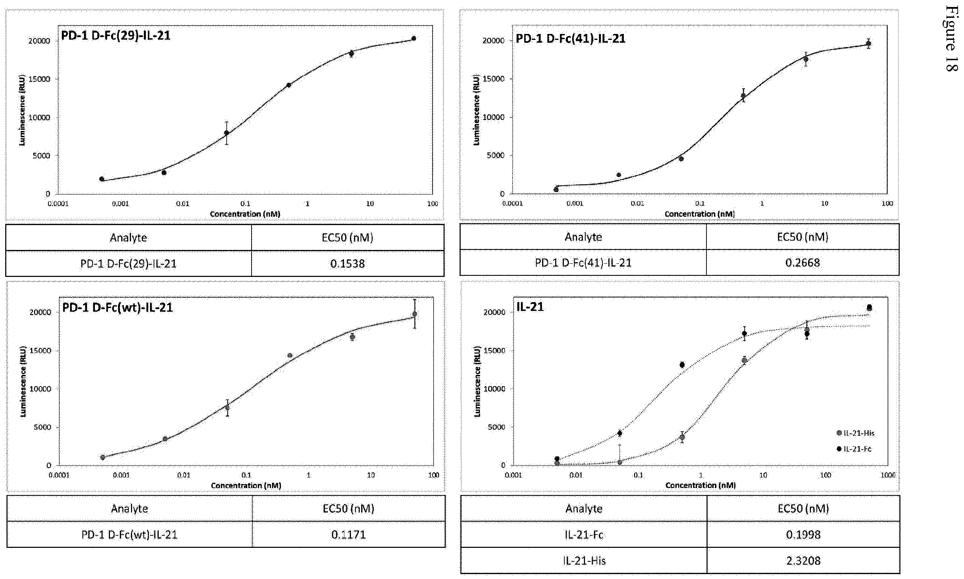

As a result of PATHHUNTER^{®} eXpress Dimerization Assay measurement, the binding affinity between the BNS001 fusion protein and the IL-21 receptor was measured as shown in Figure 18.

### VIII. Identification of immune activity of BNS001 fusion protein

### Experimental Example 18. Identification of IFN-γ production by BNS001 fusion protein

### Experimental Example 18.1. Culture of PBMCs

In order to actively culture peripheral blood mononuclear cells (PBMC) isolated from humans, the day before PBMC thawing, a T75 flask was treated with anti-CD3 antibody (OKT3, 1 µg/ml, Invitrogen) and anti-CD28 antibody (CD28.2, 1 µg/ml, Invitrogen) together in culture medium (RPMI1640 medium containing 10% FBS, 200 µl of penicillin/streptomycin) and coated overnight at 4°C. The next day, PBMCs were slowly thawed at 37°C, centrifuged at 1,500 rpm at 4°C for 5 minutes, and then resuspended in RPMI1640 culture medium (10% FBS, 200 µl of penicillin/streptomycin, anti-CD3/anti-CD28 antibodies (1 µg/ml each)), and cultured in an incubator at 37°C and 5% CO₂. At this time, under conditions of treatment with or without, anti-CD3/anti-CD28 antibodies (1 µg/ml each), the cells were treated with BNS001 fusion protein and IL-21 and cultured in an incubator for 3 days.

### Experimental Example 18.2. Identification of human IFN-γ secretion amount through ELISA analysis

The amount of human IFN-γ secreted in the culture supernatant of cells treated with each sample and cultured in Experimental Example 18.1. above was measured using a human IFN-γ ELISA kit (Biolegend, cat No.430103). The anti-human-IFN-γ antibody was added to the ELISA plate and coated by reacting overnight at 4°C. Thereafter, blocking was performed with a PBS solution containing 1% BSA at room temperature for 1 hour. After washing with a washing buffer (0.05% Tween-20 in PBS), the standard solution and each sample were diluted two-fold and added, and then reacted at room temperature for 2 hours. After the reaction was completed, the plate was washed, and a secondary detection antibody was added and reacted at room temperature for 1 hour. After washing with a washing buffer, an avidin-HRP solution was added and reacted at room temperature for 30 minutes, and a substrate solution was added to induce a color reaction for 15 minutes at room temperature and dark conditions. Finally, H₂SO₄ was added to stop the color reaction, and the absorbance was measured at 450 nm using Synergy Neo2 (BioTek) to calculate the concentration.

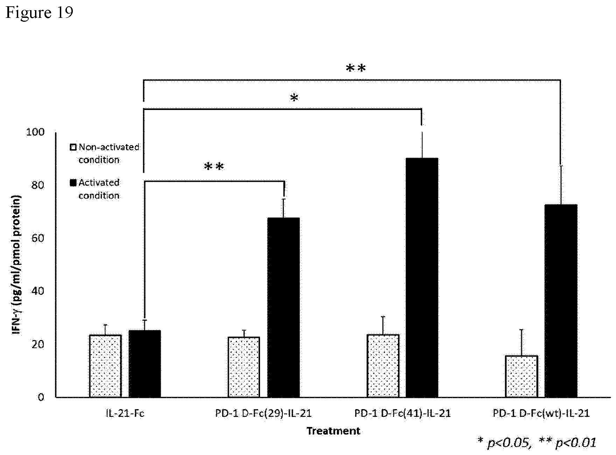

As a result, it was identified that the amount of IFN-γ secretion was statistically significantly increased in the cells treated with the BNS001 fusion protein compared to the cells treated with the IL-21 protein (Figure 19).

### Experimental Example 19. Identification of effect of BNS001 fusion protein on proliferation of CD8+ T cells

PBMCs isolated from humans were thawed at 37°C and centrifuged at 1,500 rpm at 4°C for 5 minutes. After centrifugation, the suspension was removed, resuspended in RPMI1640 culture medium (10% FBS, 200 µl of penicillin/streptomycin, BNS001 fusion protein, IL-21 protein (10 µg/ml each)), and cultured in an incubator at 37°C and 5% CO₂. After culturing, the PBMCs were washed twice with cold PBS (Sigma, pH 7.4), centrifuged, and then resuspended in a 1.5 ml microtube with PBS (containing 1% FBS) to reach 10⁶ cells per 100 µl (10⁶ cells/100 µl). Thereafter, the cells were stained with anti-CD8-PE-Texas Red (1:100, Invitrogen, cat No. MHCD0817) for 30 minutes at room temperature. After dispensing 30 µl per well into a V-bottom a 96 well plate, the level of proliferation of these cells was identified by measuring the percentage of unlabeled cells among CD8+ T cells using flow cytometry.

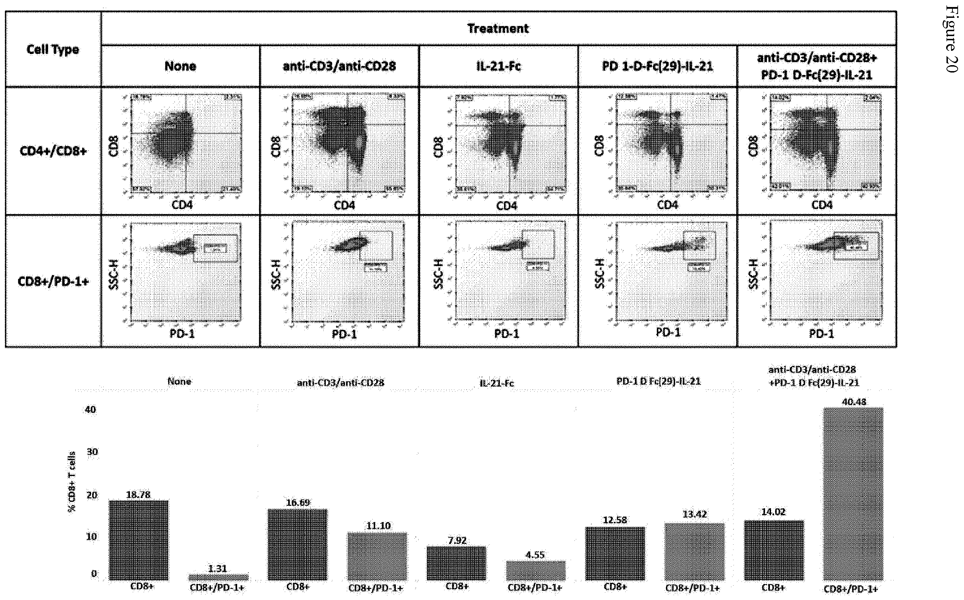

As a result, it was identified that the BNS001 fusion protein activated the proliferation of CD8+ T cells to a similar extent as the IL-21 protein (Figure 20).

### Experimental Example 20. Identification of effect of BNS001 fusion protein on proliferation of CD4+ T cells

PBMCs isolated from humans were thawed at 37°C and centrifuged at 1,500 rpm at 4°C for 5 minutes. After centrifugation, the suspension was removed, resuspended in RPMI1640 culture medium (10% FBS, 200 µl of penicillin/streptomycin, BNS001 fusion protein, IL-21 protein (10 µg/ml each)), and cultured in an incubator at 37°C and 5% CO₂. After culturing, the PBMCs were washed twice with cold PBS (Sigma, pH 7.4), centrifuged, and then resuspended in a 1.5 ml microtube with PBS (containing 1% FBS) to reach 10⁶ cells per 100 µl (10⁶ cells/100 µl). Thereafter, the cells were stained with anti-CD4-PE-Pacific Blue (2 µg/10⁶ cells, BioLegend, cat No. 344620) for 30 minutes at room temperature. After dispensing 30 µl per well into a V-bottom a 96 well plate, the level of proliferation of these cells was identified by measuring the percentage of unlabeled cells among CD4+ T cells using flow cytometry.

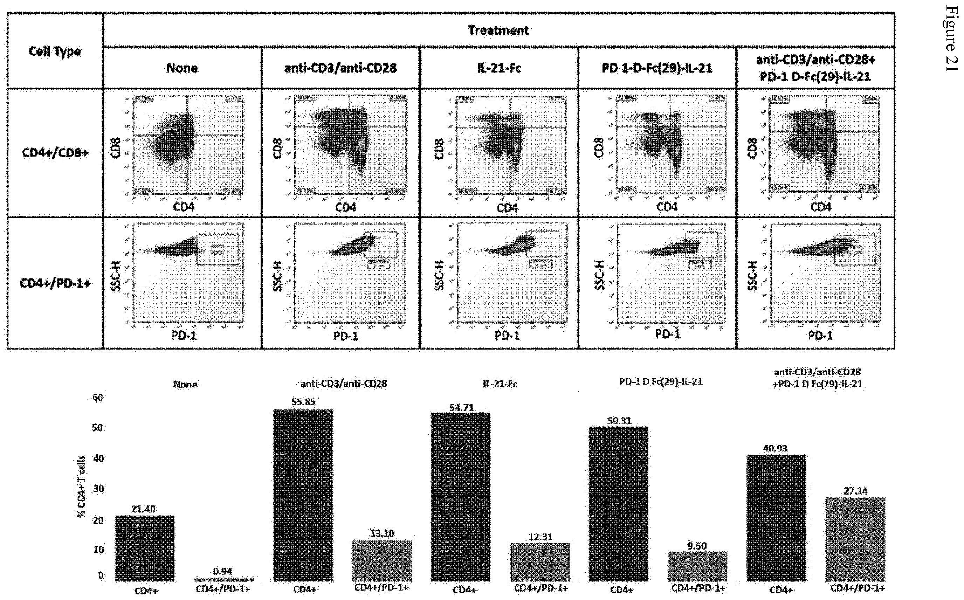

As a result, it was identified that the BNS001 fusion protein activated the proliferation of CD4+ T cells to a similar extent as the IL-21 protein, and did not increase the proliferation of CD4+/FoxP3+ Treg cells (Figure 21).

### Experimental Example 21. Identification of effect of BNS001 fusion protein on proliferation of NK cells

PBMCs isolated from humans were slowly thawed at 37°C and centrifuged at 1,500 rpm at 4°C for 5 minutes. After centrifugation, the suspension was removed, resuspended in RPMI1640 culture medium (10% FBS, 200 µl of penicillin/streptomycin, BNS001 fusion protein, IL-21 protein (10 µg/ml each)), and cultured in an incubator at 37°C and 5% CO₂. After culturing, the PBMCs were washed twice with cold PBS (Sigma, pH 7.4), centrifuged, and then resuspended in a 1.5 ml microtube with PBS (containing 1% FBS) to reach 10⁶ cells per 100 µl (10⁶ cells/100 µl). Thereafter, the cells were stained with anti-CD56-PE (0.4 µg/10⁶ cells, BioLegend, cat No. 362508) and anti-CD16-APC (5 µl/10⁶ cells, BioLegend, cat No. 302012) for 30 minutes at room temperature. After dispensing 30 µl per well into a V-bottom a 96 well plate, the level of proliferation of these cells was identified by measuring the percentage of unlabeled cells among CD56+/CD16+ NK cells using flow cytometry.

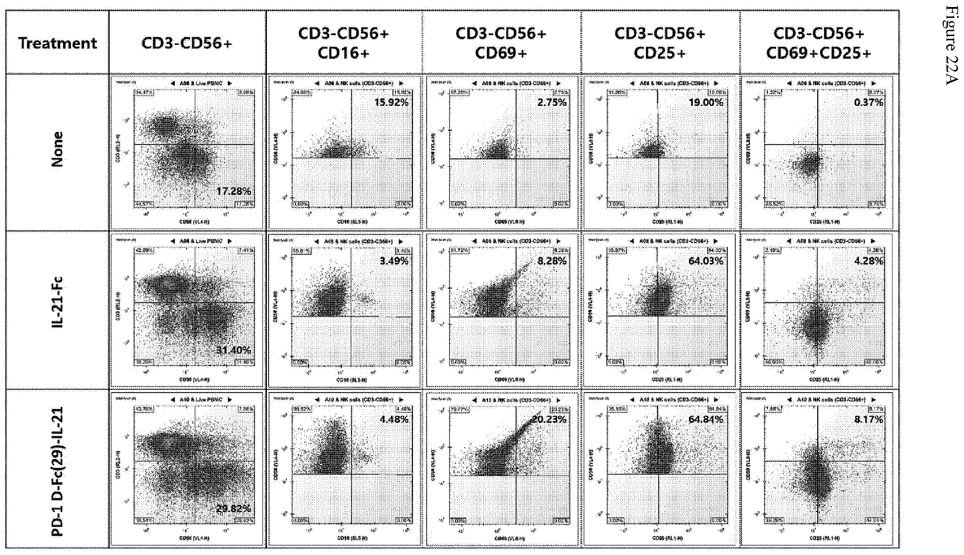

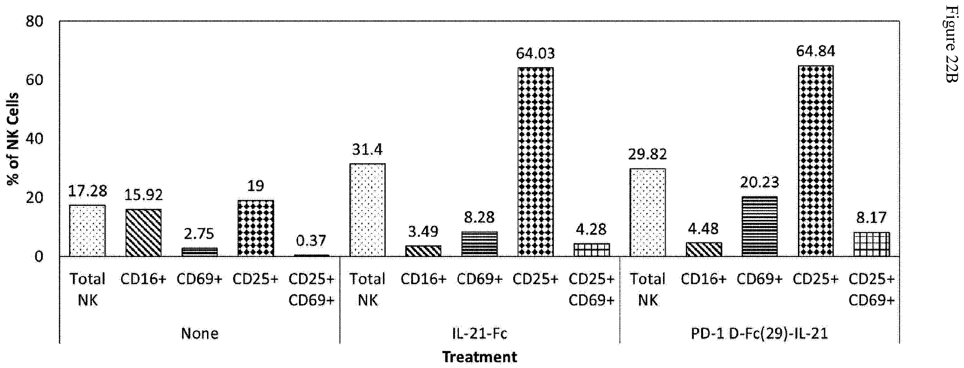

As a result, it was identified that the BNS001 fusion protein activated the proliferation of NK cells to a similar extent as the IL-21 protein (Figures 22a and 22b).

### Experimental Example 22. Identification of effect of BNS001 fusion protein on T cell function

### Experimental Example 22.1. PD-L1/PD-1 blockade assay

The experiment was conducted using the PD-1/PD-L1 blockade bioassay kit (Promega cat No. J1252).

PD-L1 aAPC/CHO-K1 cells (target cells) stored in liquid nitrogen were prepared by thawing them in a water bath at 37°C for 1 minute and suspending them in pre-warmed culture medium (Ham's F12: 10% FBS). 100 µl per well was dispensed into a 96 well white plate (SPL, cat No. 30196) and cultured in an incubator at 37°C and 5% CO₂. The next day, the plate was taken out, 95 µl of culture medium was removed, 40 µl of the BNS001 fusion protein, atezolizumab, and PD-1-Fc serially diluted 2-fold were added to each well, and during the preparation time for Jurkat NFAT-Luc/PD-1 cells (effector cells), the plate was covered with a cover and stored at room temperature. The effector cells stored in liquid nitrogen were prepared by thawing them in a water bath at 37°C for 1 minute and suspending them in pre-warmed culture medium (Ham's F12: 10% FBS), and then 40 µl was dispensed into each well of the plate containing target cells and samples and cultured in an incubator at 37°C and 5% CO₂ for 6 hours.

After the reaction was completed, the plate was taken out, and Bio-Glo reagent was added while being careful to avoid creating bubbles. The Bio-Glo reagent was also added to the three edge wells and used as a blank to correct the background signal. After reacting at room temperature for 30 minutes, luminescence was measured using Synergy Neo2 (BioTek).

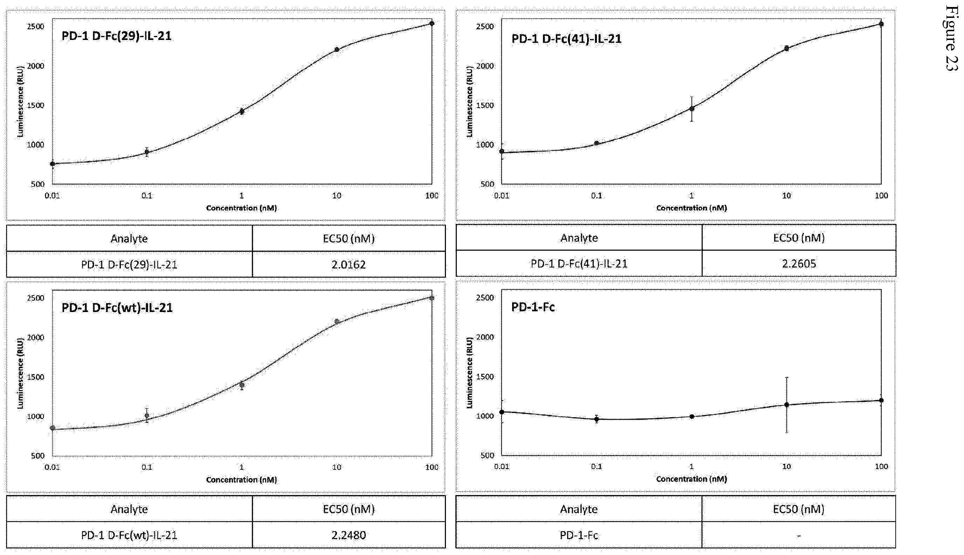

As a result, it was identified that the BNS001 fusion protein binds to PD-1 expressed on effector T cells and does not inhibit the function of T cells, but rather activates them (Figure 23).

### Experimental Example 22.2. PD-L2/PD-1 blockade assay

The experiment was conducted using the PD-1/PD-L2 blockade bioassay kit (Promega cat No. CS187131-1).

PD-L2 aAPC/CHO-K1 cells (target cells) stored in liquid nitrogen were prepared by thawing them in a water bath at 37°C for 1 minute and suspending them in pre-warmed culture medium (Ham's F12: 10% FBS). 100 µl per well was dispensed into a 96 well white plate (SPL, cat No. 30196) and cultured in an incubator at 37°C and 5% CO₂. The next day, the plate was taken out, 95 µl of culture medium was removed, 40 µl of the BNS001 fusion protein, atezolizumab, and PD-L2-Fc serially diluted 2-fold were added to each well, and during the preparation time for Jurkat NFAT-Luc/PD-1 cells (effector cells), the plate was covered with a cover and stored at room temperature. The effector cells stored in liquid nitrogen were prepared by thawing them in a water bath at 37°C for 1 minute and suspending them in pre-warmed culture medium (Ham's F12: 10% FBS), and then 40 µl was dispensed into each well of the plate containing target cells and samples and cultured in an incubator at 37°C and 5% CO₂ for 6 hours.

After the reaction was completed, the plate was taken out, and Bio-Glo reagent was added while being careful to avoid creating bubbles. The Bio-Glo reagent was also added to the three edge wells and used as a blank to correct the background signal. After reacting at room temperature for 30 minutes, luminescence was measured using Synergy Neo2 (BioTek).

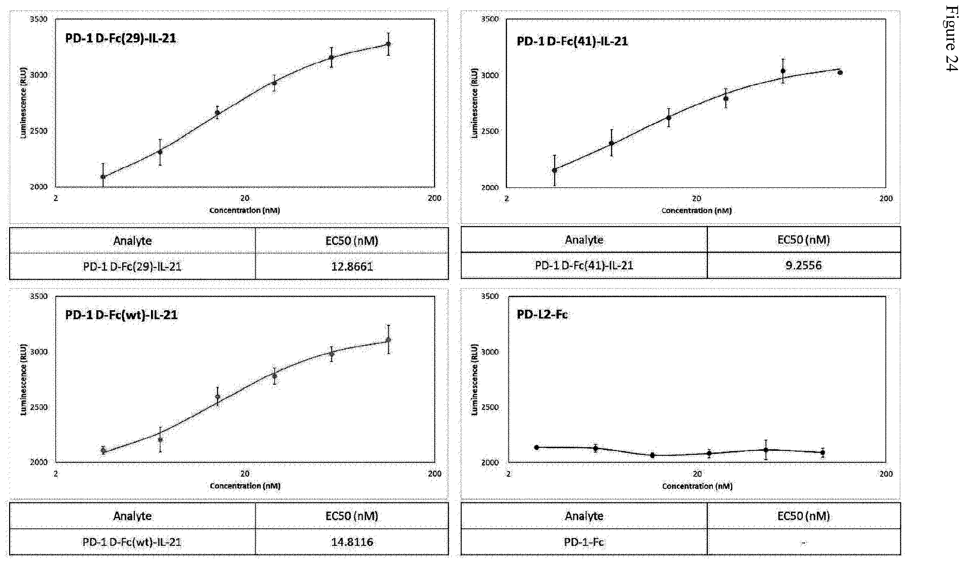

As a result, it was identified that the BNS001 fusion protein binds to PD-1 expressed on effector T cells and does not inhibit the function of T cells, but rather activates them (Figure 24).

### IX. Identification of anti-cancer effect of BNS001 fusion protein

### Experimental Example 23. Establishment of cancer cell line for identifying anti-cancer effect of BNS001 fusion protein

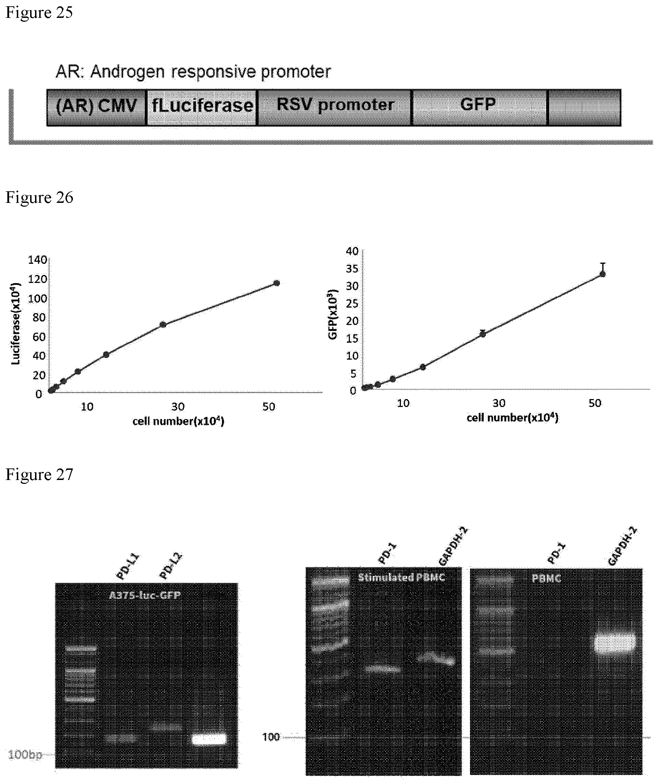

1×10⁵ cells of the human melanoma cell line A375 (ATCC, CRL-1619) were dispensed into a 6 well plate and cultured in an incubator at 37°C and 5% CO₂. The next day, after removing the culture medium, 25 MOI (Multiplicity of infection, 25 µl) of lentivirus (Gentarget, cat No. LVP914-G)(Figure 25) transfected with firefly luciferase (fLuc) and GFP gene was added and cultured for 4 hours. After culturing for 4 hours, the cells were collected with 0.05% Trypsin-EDTA (Gibco) solution and then transferred to a 150 mm culture dish (TPP) and cultured. During culture, only GFP-expressing cells were selected and cultured while observing with a fluorescence microscope, and the A375-Luc-GFP cell line, a human melanoma cell line expressing firefly luciferase, was established.

The A375-Luc-GFP cell line was dispensed into 96-well white or black plates at 5×10⁵ cells (n = 3) with 100 µl of cell culture solution, and then serially diluted in half with 100 µl of cell culture solution (8 points). After adding 100 µl of the Bio-Glo solution per well, luminescence (luciferase) or fluorescence (GFP) images were acquired for 1 second using the Neo2 equipment. As the number of cells in the A375-Luc-GFP cell line was increased, the intensity of the luminescence or fluorescence signal was also increased (Figure 26).

### Experimental Example 24. Identification of effect of BNS001 fusion protein on cancer cells expressing PD-L1 and PD-L2

### Experimental Example 24.1 Co-culture of cancer cells and PBMCs

PBMCs isolated from humans were stained by reacting with a 1.25 µM concentration of membrane-dye (Red) (Sigma, cat No. PKH26) dye at room temperature for 1 minute, and then the staining reaction was stopped by adding the same volume of FBS. The dye that did not bind to the cells was centrifuged at 400×g for 10 minutes to remove and wash the suspension, and then resuspended in RPMI1640 culture medium (10% FBS, 200 µl of penicillin/streptomycin). At this time, the washing process was performed a total of three times. After resuspending in RPMI1640 culture medium (10% FBS, 200 µl of penicillin/streptomycin), fluorescence was observed. For co-culture of the fluorescently stained PBMCs and the established cancer cell line (A375-fLuc-GFP), A375-fLuc-GFP cells (1×10⁵) were dispensed and the fluorescently stained PBMCs (1×10⁶) were dispensed into a 6 well plate, and co-culture was performed. At this time, under conditions of treatment with or without anti-CD3 antibody (OKT3, 1 µg/ml, Invitrogen) and anti-CD28 antibody (CD28.2, 1 µg/ml, Invitrogen) together, the cells were treated with the BNS001 fusion protein (10 µg/ml) and cultured in an incubator at 37°C and 5% CO₂ for 3 days.

### Experimental Example 24.2. Identification of PD-L1, PD-L2, and PD-1 gene expression in co-cultured cancer cells and PBMCs

In order to identify the transcription and translation of PD-L1, PD-L2, and PD1 genes in malignant melanoma cells A375 (ATCC, # CRL-1619) and PBMCs (ATCC, # PCS-800-011), reverse transcription PCR was performed. PBMCs were co-stimulated with anti-CD3 antibody (1 µg/ml, Invitrogen) and anti-CD28 antibody (1 µg/ml, Invitrogen) and used for active culture. Total RNA was extracted from each cell line, cDNA was synthesized (TAKARA, RR036A), and PCR was performed using a primer set (Table 1) to identify PD-L1/L2 and PD-1 specific replicate products (120 bp, 173 bp, 289 bp) in A375 and PBMC.

**[Table 1]**

| **Primer name** | **Sequence** | **SEQ ID NO** |
|---|---|---|
| PD-L1 | F: 5'-TGGCATTTGCTGAACGCATTT-3' | 120 |
| PD-L1 | R: 5'-TGCAGCCAGGTCTAATTGTTTT-3' | 121 |
| PD-L2 | F: 5'-CAGCAATGTGACCCTGGAAT-3' | 122 |
| PD-L2 | R: 5'-GGACTTGAGGTATGTGGAACG-3' | 123 |
| PD-1 | F: 5'-TGCAGCTTCTCCAACACATC-3' | 124 |
| PD-1 | R: 5'-CTGCCCTTCTCTCTGTCACC-3' | 125 |
| GAPDH-2 | F: 5'-AGCCGCATCTTCTTTTGCGT-3' | 126 |
| GAPDH-2 | R: 5'-TGACGAACATGGGGGCATCA-3' | 127 |

As a result, the replicate products (120 bp, 173 bp, and 289 bp), specific for PD-L1 and PD-L2 genes in malignant melanoma cells (A375) and specific for PD-1 in PBMCs where immune cells were present, were identified (Figure 27).

### Experimental Example 24.3. Identification of human IFN-γ secretion amount through ELISA analysis

The amount of human IFN-γ secreted in the culture supernatant of cells treated with each sample and cultured in Experimental Example 24.1. above was measured using a human IFN-γ ELISA kit (Biolegend, cat No.430103). The anti-human-IFN-γ antibody was added to the ELISA plate and coated by reacting overnight at 4°C. Thereafter, blocking was performed with a PBS solution containing 1% BSA at room temperature for 1 hour. After washing with a washing buffer (0.05% Tween-20 in PBS), the standard solution and each sample were diluted at concentrations of 0.4 nM, 1.5 nM, and 7.5 nM and added, and then reacted at room temperature for 2 hours. After the reaction was completed, the plate was washed, and a secondary detection antibody was added and reacted at room temperature for 1 hour. After washing with a washing buffer, an avidin-HRP solution was added and reacted at room temperature for 30 minutes. A substrate solution was added to induce a color reaction for 20 minutes at room temperature and dark conditions. Finally, H₂SO₄ was added to stop the color reaction, and the absorbance was measured at 450 nm using Synergy Neo2 (BioTek) to calculate the concentration.

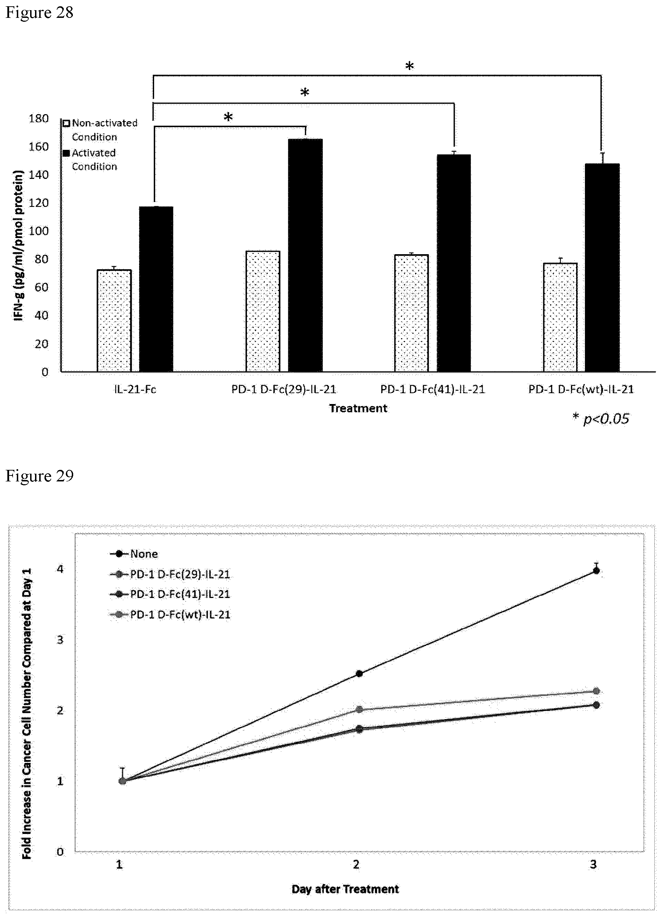

As a result, it was identified that the amount of IFN-γ secretion was statistically significantly increased in the cells treated with the BNS001 fusion protein compared to the cells treated with the IL-21 protein (Figure 28).

### Experimental Example 24.4. Identification of cancer cell-killing effect

In order to identify the immune cell activity and tumor cell killing effect, under conditions of treatment with or without 5 µg/mL of anti-CD3 antibody (OKT3, 1 µg/ml, Invitrogen) and anti-CD28 antibody (CD28.2, 1 µg/ml, Invitrogen), the co-cultured cells were treated with the BNS001 fusion protein at a concentration of 10 µg/ml and cultured in an incubator at 37°C and 5% CO₂ for 72 hours. In order to quantify the degree of cancer cell killing, after co-culturing for 24, 48, and 72 hours, the wells were washed twice with PBS, and the Bio-Glo reagent was added and reacted at room temperature for 10 minutes. After reaction, luciferase activity was measured using Synergy Neo2 (BioTek).

The degree of cancer cell killing measured on day 1 (24 hours), day 2 (48 hours), and day 3 (72 hours) after treatment with the BNS fusion protein was measured as shown in Figure 29.

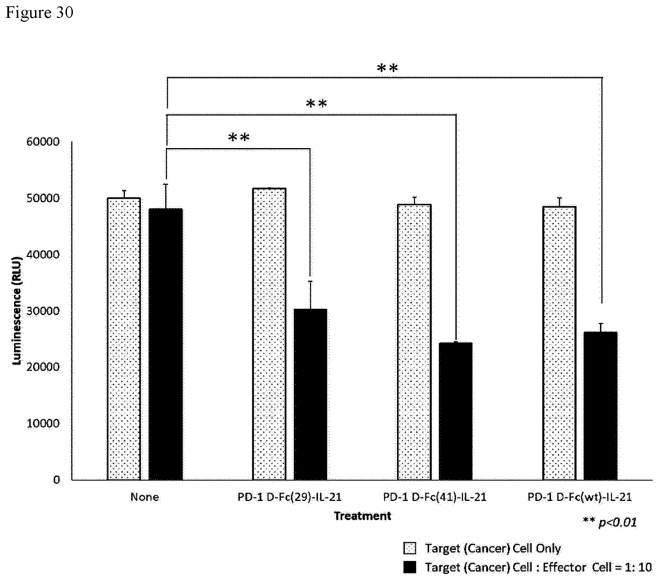

In addition, for statistical analysis of the cancer cell-killing effect, the degree of cancer cell killing measured day 2 after treatment with the BNS fusion protein was compared. As a result, it was identified that the cancer cell-killing effect was statistically significantly increased in the cells treated with the BNS001 fusion protein compared to the untreated cells (Figure 30).

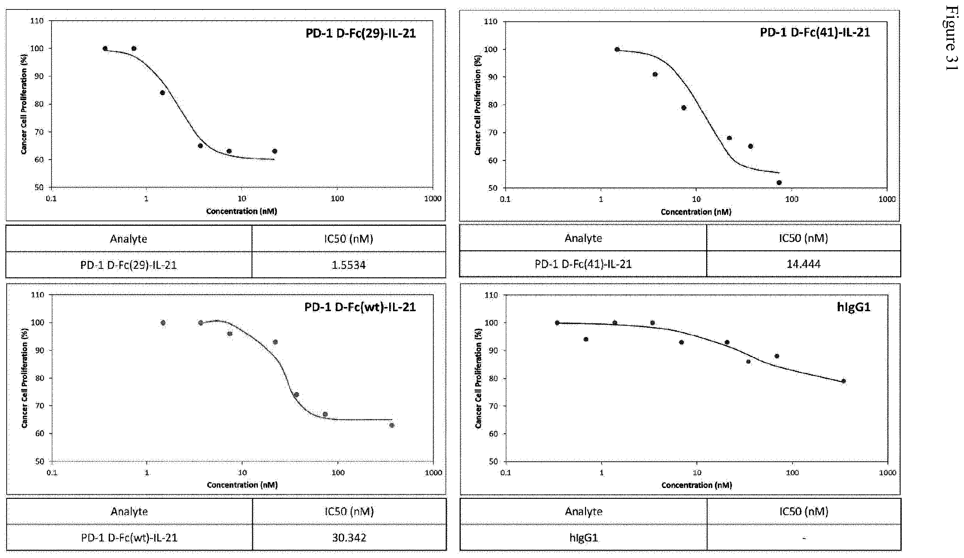

In addition, for EC₅₀ analysis of the cancer cell-killing effect, the degree of cancer cell killing measured day 2 after treatment with the BNS fusion protein at various concentrations was compared. As a result, it was identified that the cancer cell-killing effect was increased in the cells treated with the BNS001 fusion protein compared to the untreated cells (Figure 31).

### Experimental Example 25. Identification of cancer cell line-specific antibody dependent cellular cytotoxicity (ADCC) activity of BNS001 fusion protein

The A375 cell line was suspended in culture medium (RPMI1640: 10% FBS, Antibiotic-Antimycotic 1%), dispensed into a 96 well white plate at a concentration of 1×10⁴ cells/100 µl, and then cultured in an incubator overnight. The next day, after removing 95 µl of culture medium from the plate where A375 cells were dispensed, 25 µl each of ADCC assay buffer (RPMI1640 culture medium, low IgG serum 0.25%) prepared in advance was added, and the effector cells (NFAT-luc/FcγRIIIa) was cultured at room temperature during preparation. 630 µl of ADCC bioassay effector cells (Jurkat/NFAT-luc/FcγRIIIa) were suspended in 3.6 mL of pre-warmed ADCC assay buffer, and then 25 µl was dispensed into each well. Each well was treated with 25 µl of BNS001 fusion protein sample serially diluted 2.5 times. The well plate lid was closed and cultured in an incubator at 37°C and 5% CO₂ for 6 hours. After incubation, it was stored at room temperature for about 15 minutes, and then each well was treated with 75 µl of BIO-GLO^{™} Luciferase Assay Reagent. After 30 minutes, luciferase activity was measured using Synergy Neo2 (BioTek).

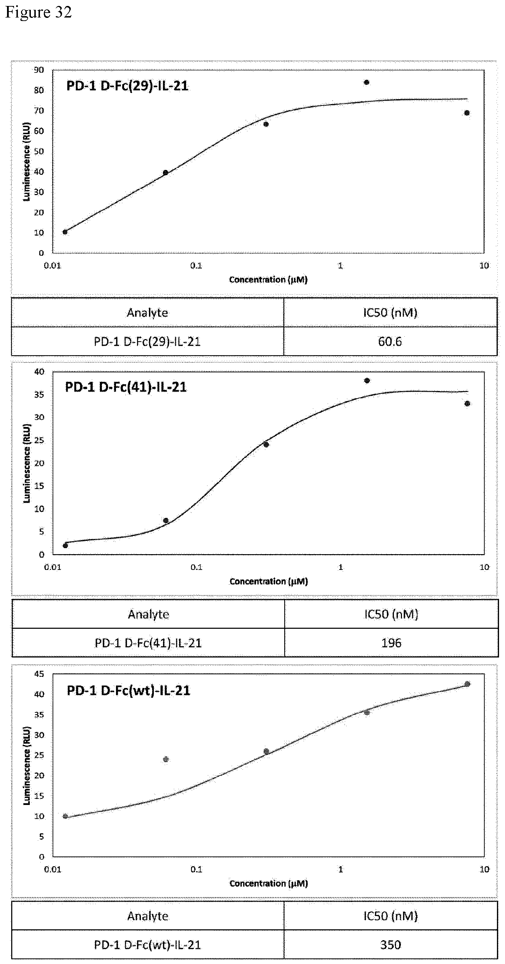

The luciferase activity of ADCC assay is a test method in which results can be obtained only when target cells (A375) with surface antigens (PD-L1/L2), specific antibodies (BNS001 fusion protein), and effector cells expressing FcyRIIIa are present, and the ADCC activity for the BNS001 fusion protein was measured as shown in Figure 32.

### Experimental Example 26. Identification of blood half-life of BNS001 fusion protein in mouse model

### Experimental Example 26.1. Quantitative measurement of BNS001 fusion protein concentration using ELISA analysis

In order to quantitatively analyze the concentration of the BNS001 fusion protein in the blood, ELISA analysis was performed using the human PD-1 ELISA kit (Invitrogen, Cat. No., BMS2214). The BNS001 fusion protein was diluted to concentrations of 0.0625, 0.125, 0.25, 0.5, 1, 2, and 4 ng/mL, respectively, and 50 µl was dispensed into each well of a plate (Invitrogen) immobilized with anti-PD-1 antibody. 50 µl of each of the sample diluent included in the kit and the biotinylated anti-IL-21 detection antibody (R&D) was added to each well and then reacted at room temperature for 2 hours. After the reaction was completed, the plate was washed four times with 400 µl of washing buffer (Invitrogen), and then 100 µl of streptavidin-HRP (Invitrogen) was dispensed into each well. Thereafter, the reaction was performed at room temperature for 1 hour, and the washing process was performed four times with 400 µl of washing buffer (Invitrogen). 100 µl of TMB substrate solution (Invitrogen) was added to each well to develop color. Thereafter, the reaction was terminated by adding 100 µl of stop solution (Invitrogen) and analyzed using Synergy Neo2 (BioTek).

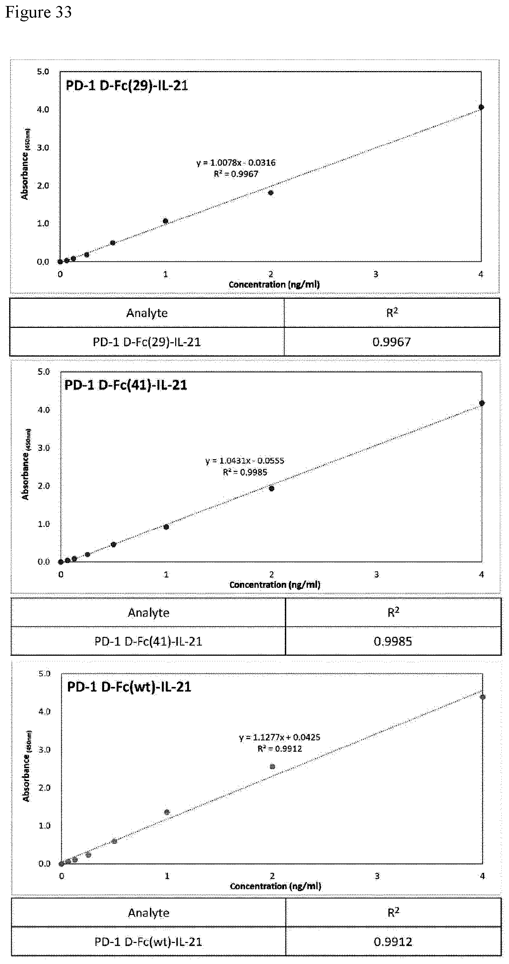

As a result of the ELISA measurement, the linearity and correlation coefficient for each concentration for the BNS001 fusion protein were measured as shown in Figure 33.

### Experimental Example 26.2. Identification of blood half-life of BNS001 fusion protein in normal BALB/c mice

The BNS001 fusion protein prepared in the normal BALB/c mouse animal model was administered to a total of 3 mice at 5 mg/kg per mouse through the caudal vein. For mice injected through the caudal vein, blood was collected over time at 0 minutes, 30 minutes, 1 hour, 6 hours, 24 hours, 3 days, 7 days, 14 days, 21 days, 28 days, 35 days, 42 days, and 50 days. The concentration analysis of the BNS001 fusion protein was performed in plasma using the ELISA analysis described in Experimental Example 26.1. The blood half-life of the BNS001 fusion protein was identified by performing non-compartmental analysis (NCA) with PKSolver.

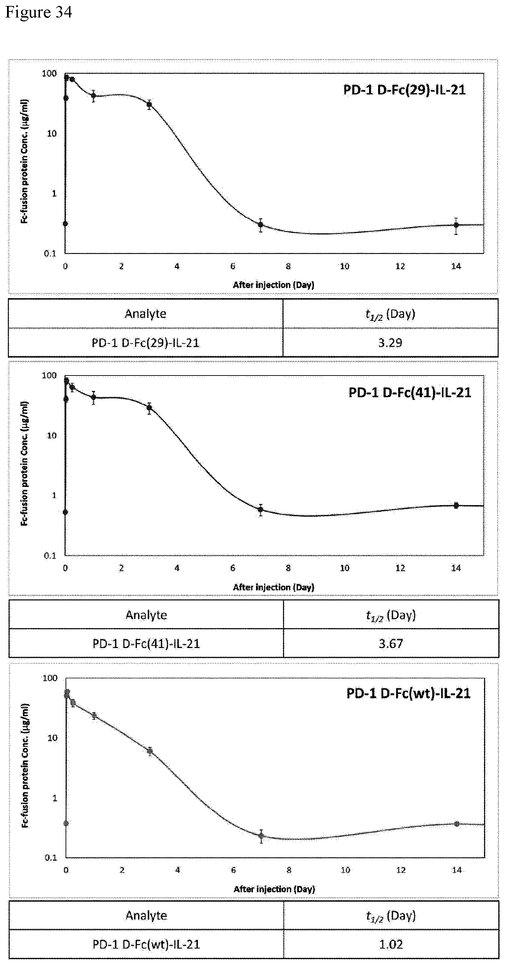

The blood half-life analysis results for the BNS001 fusion protein in BALB/c mice were measured as shown in Figure 34. The blood half-life of the PD-1 D-Fc(29)-IL-21 and PD-1 D-Fc(41)-IL-21 fusion proteins, which are persistent Fc variants, was found to be increased compared to the PD-1 D-Fc(wt)-IL-21 fusion protein having the wild type Fc.

### Experimental Example 26.3. Identification of blood half-life of BNS001 fusion protein in hFcRn TG mice

The BNS001 fusion protein prepared in the normal hFcRn TG mouse animal model was administered to a total of 3 mice at 5 mg/kg per mouse through the caudal vein. For mice injected through the caudal vein, blood was collected over time at 0 minutes, 30 minutes, 1 hour, 6 hours, 24 hours, 3 days, 7 days, 14 days, 21 days, 28 days, 35 days, 42 days, and 50 days. The concentration analysis of the BNS001 fusion protein was performed in plasma using the ELISA analysis described in Experimental Example 26.1. The blood half-life of the BNS001 fusion protein was identified by performing non-compartmental analysis (NCA) with PKSolver.

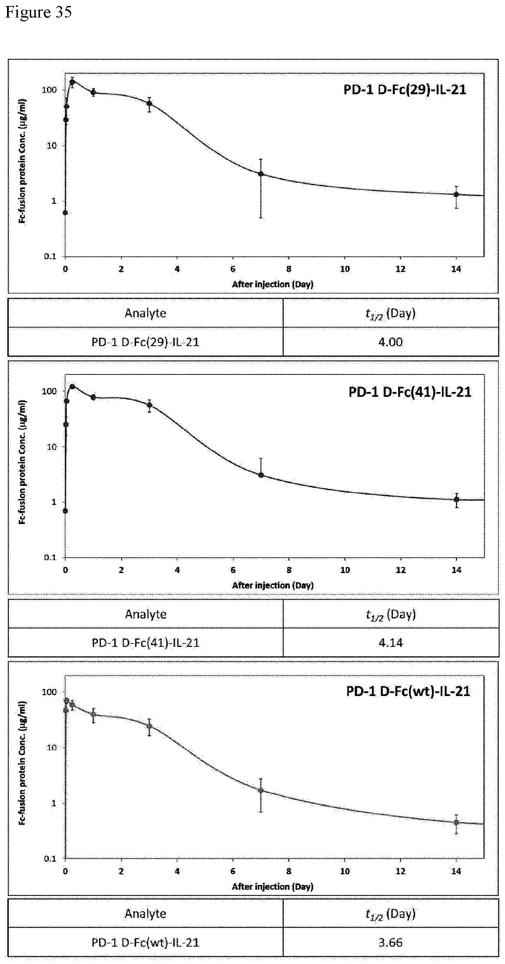

The blood half-life analysis results for the BNS001 fusion protein in hFcRn TG mice were measured as shown in Figure 35. The blood half-life of the PD-1 D-Fc(29)-IL-21 and PD-1 D-Fc(41)-IL-21 fusion proteins, which are persistent Fc variants, was found to be increased compared to the PD-1 D-Fc(wt)-IL-21 fusion protein having the wild type Fc.

### Experimental Example 27. Identification of anti-cancer effect of BNS001 fusion protein in mice allografted with mouse-derived colorectal cancer cells

### Experimental Example 27.1. Identification of tumor inhibitory effect

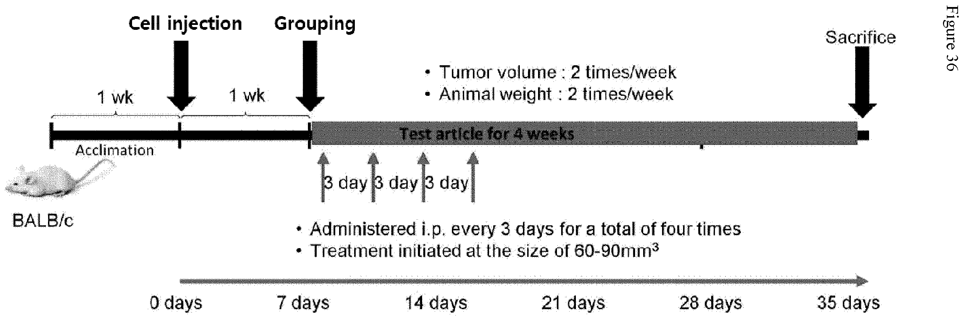

One vial of a frozen mouse tumor cell line, specifically the colorectal carcinoma cell line CT-26 cell line, was thawed using RPMI1640 medium containing heat inactivated 10% FBS (Gibco, 10082-147), placed in a cell culture flask, and cultured in an incubator at 37°C and 5% CO₂. After incubation, the cells were washed with PBS, diluted 10 times with 2.5% Trypsin-EDTA (Gibco, 15090), and then added to isolate the cells. After cell isolation, the supernatant was removed by centrifugation (1,000 rpm, 5 minutes), and then a cell suspension was obtained with a new medium. After checking the viability using a microscope, the cell lines were prepared by diluting them in medium to a concentration of 5.0×10⁶ cells/mL. The used mouse was a 5-week-old male BALB/c mouse (KOATECH). When transplanting cell lines, they were administered subcutaneously at a dosage volume of 5.0×10⁵ cells/0.1 mL/head. The tumor volume was measured after a certain period of time after transplantation with cancer cells. After the subjects whose tumor volume reached approximately 60-90 mm³ were separated, the BNS001 fusion protein was administered intravenously at a dose of 5 mg/kg. After the first administration, a total of 4 administrations were performed once every 3 days. At this time, PBS was administered as a negative control, and 5 mg/kg of atezolizumab, an anti-PD-L1 antibody, was administered as a positive control. In order to identify the anti-cancer effect, the tumor size was measured twice a week (Figure 36).

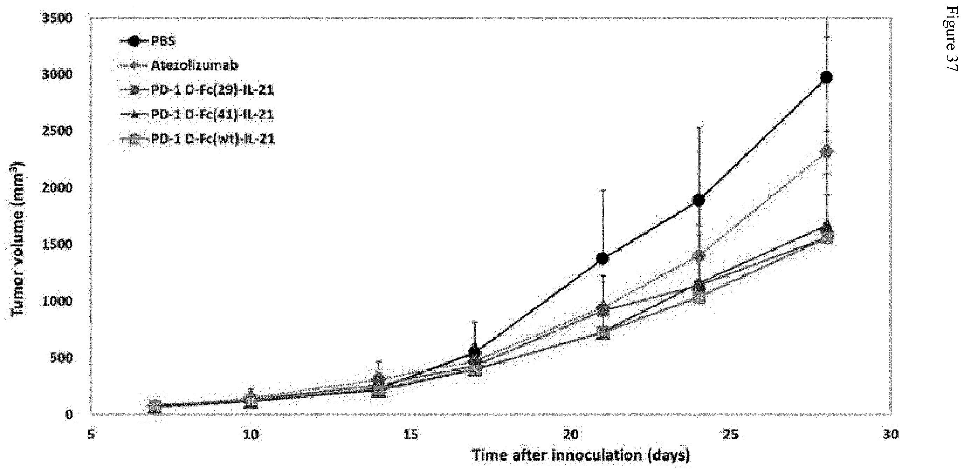

As a result, it was identified that the tumor size of mice transplanted with the CT-26 cancer cell line treated with the BNS001 fusion protein was significantly reduced compared to the negative control and positive control (Figure 37).

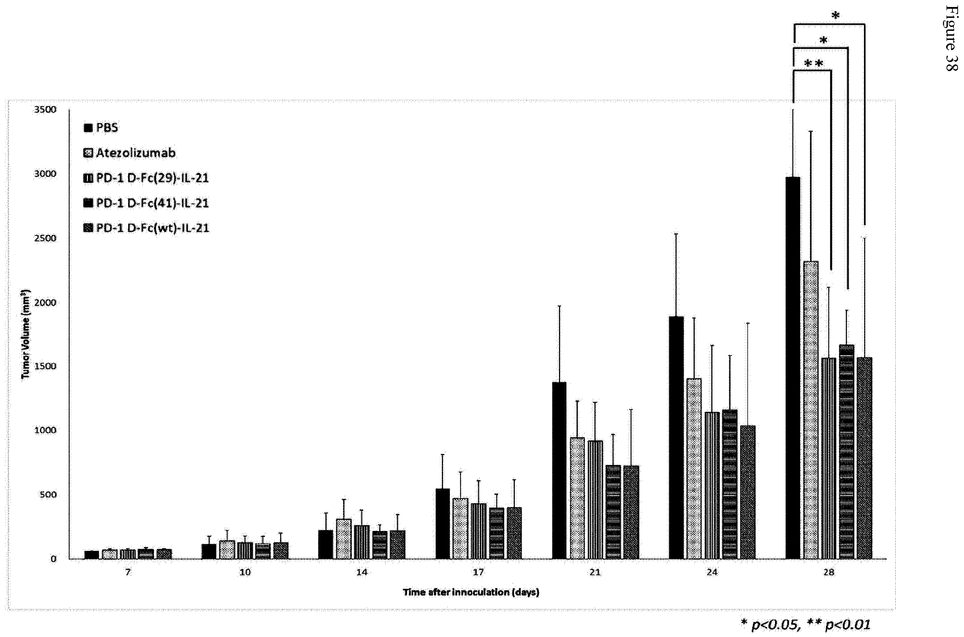

As a result of identifying the statistical significance of tumor inhibition in the group administered with the BNS001 fusion protein and the negative control, statistically significant inhibition was observed at some measurement time points (Figure 38).

### Experimental Example 27.2. Analysis of immune cells in cancer tissue

Among the mice in Experimental Example 27.1. above, tumors extracted from only 3 mice per group were placed in RPMI medium containing 10% FBS and used for FACS analysis. In order to analyze immune cells in cancer tissue, cancer tissue was isolated at the single cell level, and then analysis was carried out for T cells, NK cells, dendritic cells (DCs), and macrophages using the antibodies listed in Table 2 below.

**[Table 2]**

| Effector T cells | NK cells, DCs, Macrophages |
|---|---|
| CD3-EFLUOR^{®} 450 (Thermo, 48-0031-82) | CD3-EFLUOR^{®} 450 (Thermo, 48-0031-82) |
| | CD45-Super bright 600 (Thermo, 63-0451-82) |
| CD45-PerCP-cyanine 5.5 (Thermo, 45-0451-82) | CD335-PE (Thermo, 12-3351-82) |
| | CD11c-Percp-cy5 (Thermo, 45-0114-82) |
| CD4-PE-cy7 (Thermo, 25-0041-82) | F4/80-PE-cy7 (Thermo, 25-4801-82) |
| | CD49b-APC (Thermo, 17-5971-82) |
| CD8-APC (Thermo, 17-0081-82) | CD11b-APC-eFluor 770 (Thermo, 47-0112-82) |

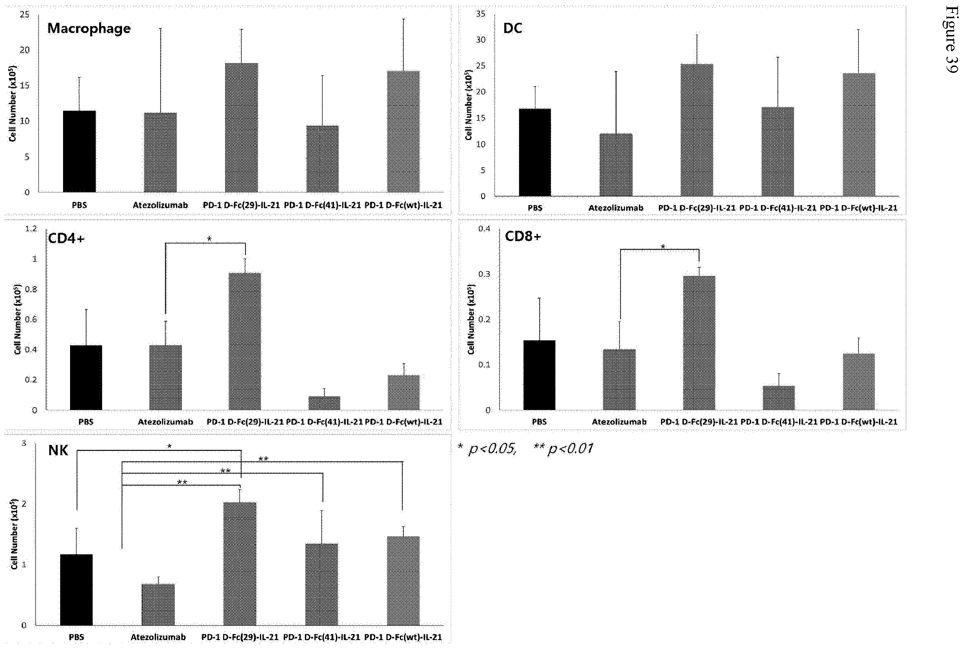

As a result, in the experimental group administered with the BNS001 fusion protein, macrophages, dendritic cells, CD4+ T cells, and CD8+ T cells were increased compared to the positive control and negative control (Figure 39). In addition, in the experimental group administered with the BNS001 fusion protein, NK cells were significantly increased compared to the positive control and negative control (Figure 39).

### Experimental Example 28. Identification of anti-cancer effect of BNS001 fusion protein in humanized mice transplanted with human-derived lung cancer cells (H460) and human peripheral blood mononuclear cells

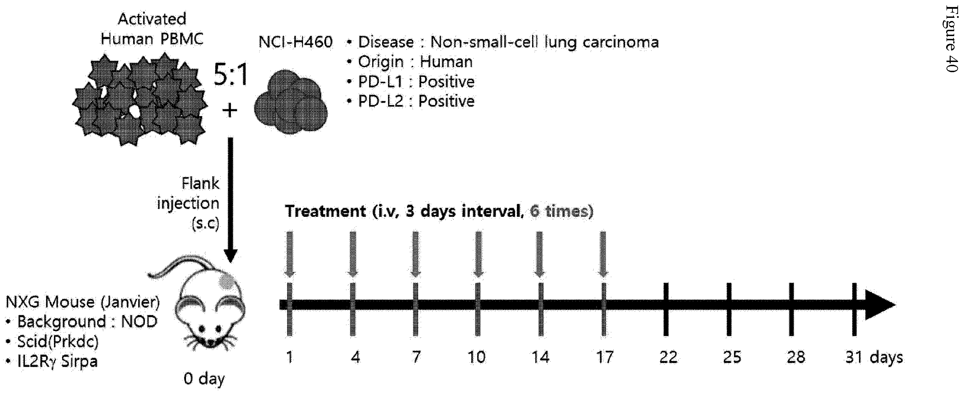

One vial of a frozen human tumor cell line, specifically the lung carcinoma cell line H460 cell line, was thawed using RPMI1640 medium containing heat inactivated 10% FBS (Gibco, 10082-147), placed in a cell culture flask, and cultured in an incubator at 37°C and 5% CO₂. After incubation, the cells were washed with PBS, diluted 10 times with 2.5% Trypsin-EDTA (Gibco, 15090), and then added to isolate the cells. After cell isolation, the supernatant was removed by centrifugation at 1,000 rpm for 5 minutes, and then a cell suspension was obtained with a new medium. After checking the viability using a microscope, the cell lines were prepared by diluting them in medium to a concentration of 5.0×10⁷ cells/mL. In addition, peripheral blood mononuclear cells (PBMC) isolated from humans were actively cultured for one week as in Experimental Example 18.1. After checking the viability using a microscope, the cell lines were prepared by diluting them in medium to a concentration of 2.5×10⁸ cells/mL. The used mouse was a 6-week-old male NXG mouse (Janvier-labs). After an acclimatization process for 1 week, body weight was measured and regrouping was performed to match the average weight of each group. When transplanting cell lines, they were administered subcutaneously at a dosage volume of 5.0×10⁶ cells (H460) and 2.5×10⁷ cells (hPBMC)/0.1 mL/head. The next day after transplanting the cancer cells, the mice were anesthetized with an inhalation anesthetic (2% isoflurane, Hana Pharm Co., Ltd.), and then the BNS001 fusion protein was administered into the tail vein at a dose of 10 mg/kg. After the first administration, a total of 6 administrations were performed once every 3 to 4 days. At this time, saline (JW Pharmaceutical) was administered as a negative control, and 10 mg/kg of atezolizumab (Evidentic), an anti-PD-L1 antibody, or PD-1 D-Fc was administered as a positive control. In order to identify the anti-cancer effect, the tumor size was measured twice a week (Figure 40).

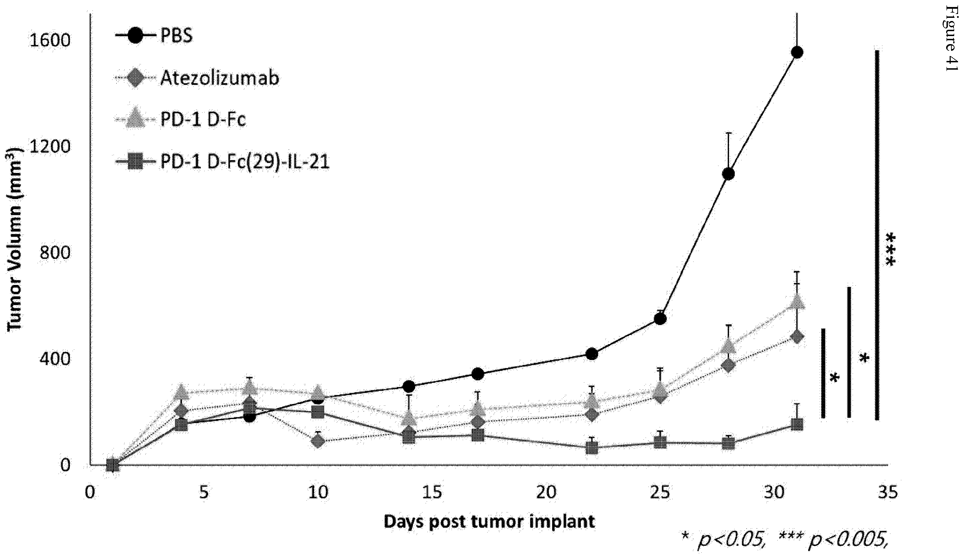

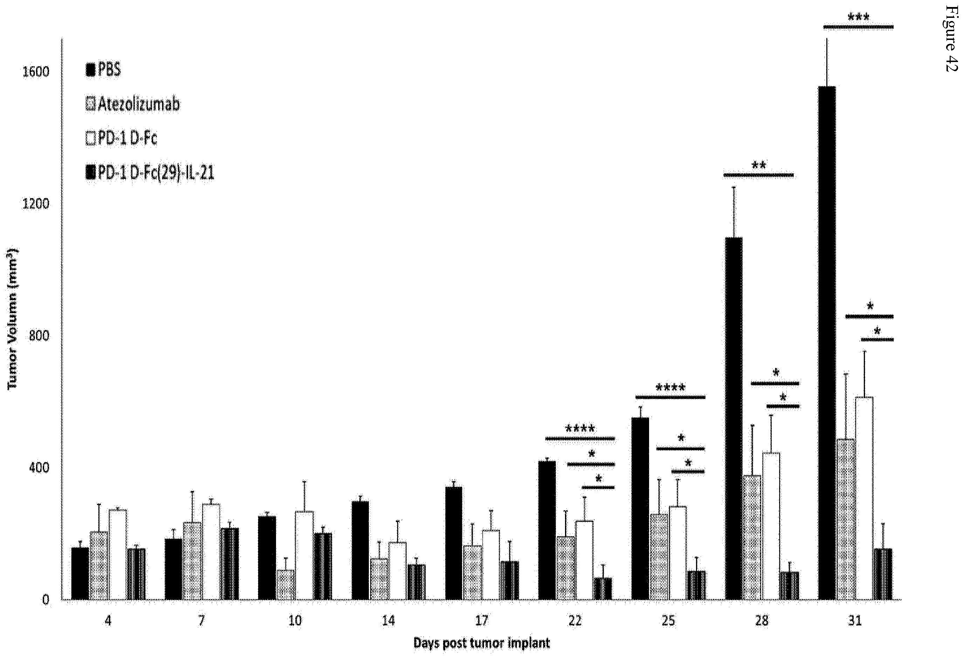

As a result, it was identified that the tumor size of mice transplanted with the H460 cancer cell line treated with the BNS001 fusion protein was reduced compared to the negative control and positive control (Figure 41), and this reduction was statistically significant (Figure 42).

### X. Evaluation of anti-cancer efficacy of mBNS001 fusion protein on allografted mouse tumor model

### Experimental Example 29. Identification of anti-cancer effect of mBNS001 fusion protein in mice allografted with mouse-derived colorectal cancer cells (MC38)

### Experimental Example 29.1. Establishment of test schedule for setting dose of mBNS001 fusion protein

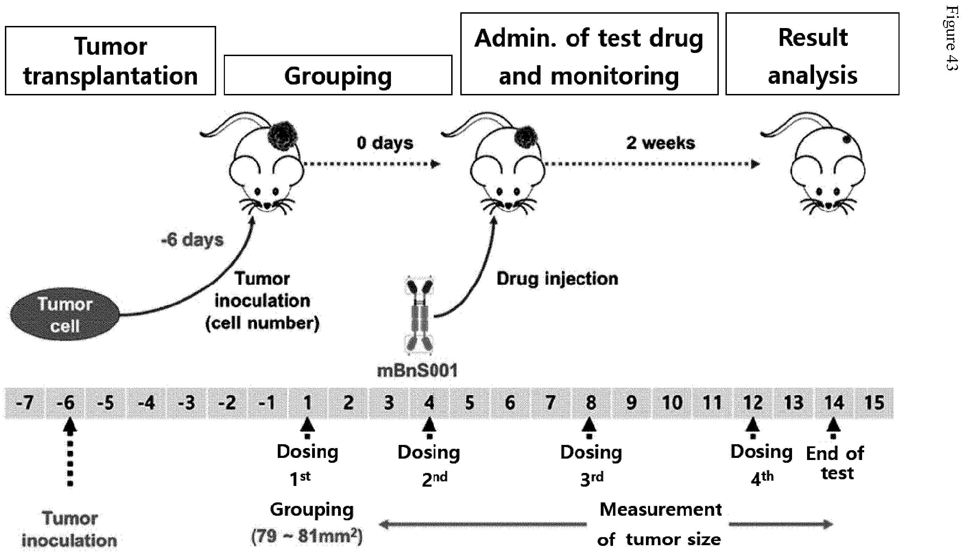

The test substance, mBNS001, was administered alone at different doses to female C57BL/6 mice transplanted with MC38 cells, a C57BL/6 mouse-derived colorectal cancer cell line, a type of mouse (*Mus musculus*) colon carcinoma. Thereafter, an experiment was conducted to set the dose at which an effective effect is achieved (Figure 43).

### Experimental Example 29.2. Preparation of cell line

As cells to be used in the test, MC38 cells were thawed, placed in a cell culture flask, and cultured in an incubator (MCO-170M, Panasonic, Japan) at 37°C and 5% CO₂. The cells were suspended using Trypsin-EDTA (Cat. 25200-072, Thermofisher scientific, U.S.A.). The suspended cells were collected by centrifugation (125×g, 5 minutes) using a centrifuge, transferred to a new medium and a new flask, and subcultured. On the day of cell line transplantation, the cultured cells were placed in a centrifuge tube and then collected. Thereafter, centrifugation (125 × g, 5 minutes) was performed, the supernatant was discarded, and cell suspension (5×10⁵ cells/0.05 mL) was made with PBS (Cat. ML 008-01, Welgene, KOREA) and stored on ice until inoculation.

### Experimental Example 29.3. Transplantation of cell line

The next day after the end of the quarantine and acclimatization period, body weight was measured, and then the prepared MC38 cell suspension (5×10⁵ cells/0.025 mL) was filled into a disposable syringe (31G, Cat. 328820, BD, U.S.A.) and administered subcutaneously to the right back of the animal at a dose of 0.05 mL/head and transplanted. After transplantation of the cell line, general symptoms were observed once a day during the engraftment and growth period.

### Experimental Example 29.4. Administration method and frequency of administration

For intravenous administration, administration was performed a total of 4 times according to the administration day using a disposable syringe (31G, 328820, BD, U.S.A.) (Table 3).

**[Table 3]**

| Group | | Dosage (mg/kg) | Dosage volume (mL/kg) | Number of animals (subject number) |
|---|---|---|---|---|
| G1 | Vehicle | 0 | 5 | 8(2101-2108) |
| G2 | mBNS001 | 5 | 5 | 8(2201-2208) |
| G3 | mBNS001 | 10 | 5 | 8(2301-2308) |
| G4 | mBNS001 | 20 | 5 | 8(2401-2408) |
| G5 | Normal | 0 | 0 | 6(2501-2506) |

### Experimental Example 29.5. Determination of dose of mBNS001 fusion protein for evaluation of anti-cancer efficacy

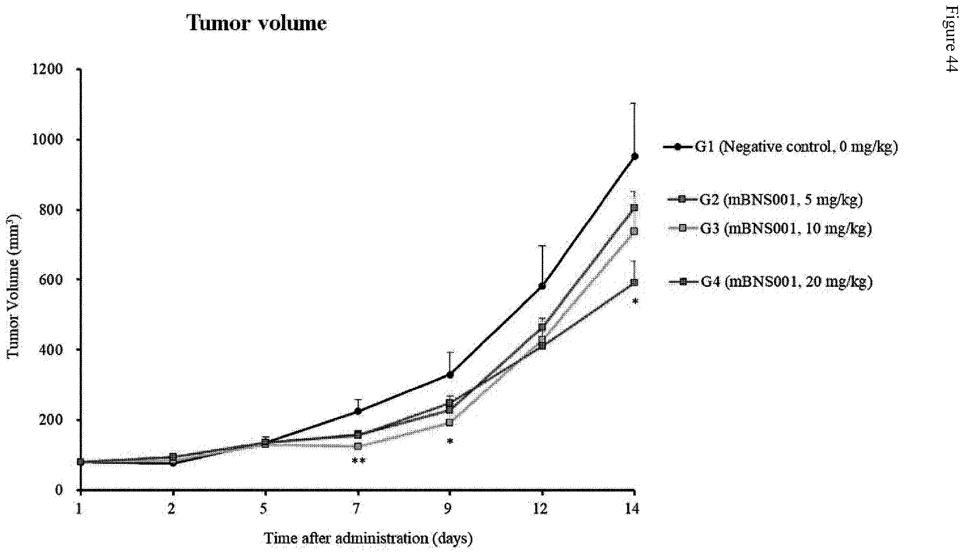

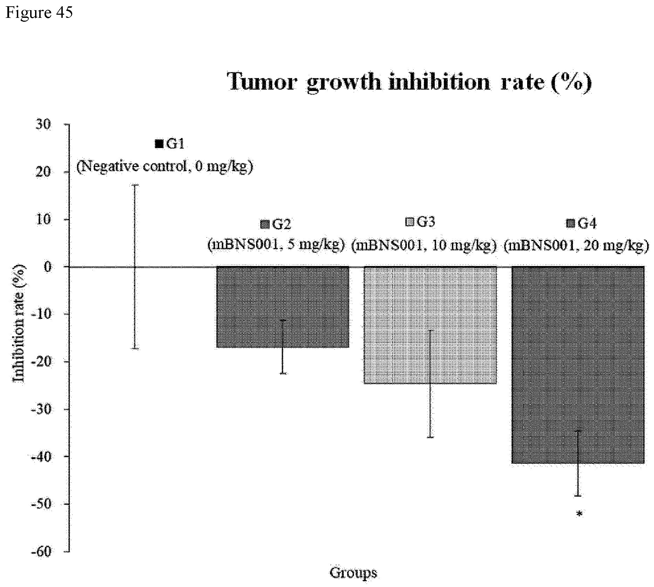

As a result of administering mBNS001 according to the test schedule and administration method of Experimental Example 29.1. and Experimental Example 29.4. above, a tendency to inhibit tumor growth in a dose-dependent manner due to administration of the test substance was observed. It was identified that the tumor growth was effectively inhibited by test substance administration in the group administered with mBNS001 at a dose of 20 mg/kg (Figures 44 and 45).

As a result, it was inferred that the BNS001 fusion protein would be effective in treating cancer at a dose of 20 mg/kg or higher when administered alone, and it was identified to have a dose-dependent effect even at doses lower than that. Accordingly, when developing the BNS001 fusion protein as a combination treatment, it was found that when the combination treatment and the BNS001 fusion protein at a dose of 10 mg/kg were used together, it was an appropriate dose to analyze the effect of administering each test substance.

### Experimental Example 30. Identification of anti-cancer effect of mBNS001 fusion protein in mice allografted with mouse-derived colorectal cancer cells (CT-26)

### Experimental Example 30.1. Establishment of test schedule for evaluating effectiveness of mBNS001 fusion protein

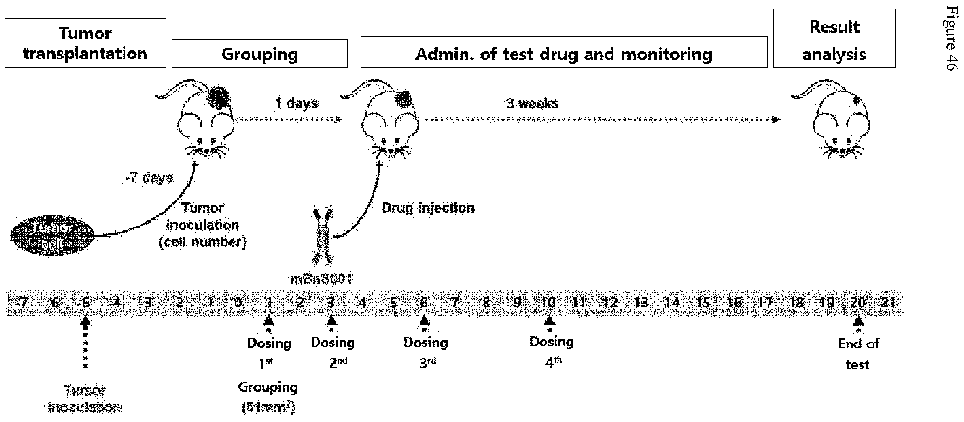

The test substances anti-PD1 Ab, anti-PD-L1 Ab, mPD1-Fc-mIL-21, mPD1-Fc, and Fc-mIL-21 were administered alone or in combination to female BALB/c mice allografted with CT-26 cells, a BALB/c mouse-derived colorectal cancer cell line, a type of mouse (*Mus musculus*) colon carcinoma. Thereafter, an experiment was conducted to evaluate the tumor growth inhibitory effect according to the combination treatment of the test substances (Figure 46).

### Experimental Example 30.2. Preparation of cell line

The cell line was prepared in the same manner as in Experimental Example 29.2., except that CT-26 cells were used as cells to be used in the test.

### Experimental Example 30.3. Transplantation of cell line

Transplantation of the cell line was performed in the same manner as in Experimental Example 29.3., except that CT-26 cell suspension was used as a cell suspension. After transplantation, general symptoms were observed once a day during the engraftment and growth period.

### Experimental Example 30.4. Administration method and frequency of administration

For intravenous administration, administration was performed a total of 4 times according to the administration day using a disposable syringe (31G, 328820, BD, U.S.A.) (Table 4).

**[Table 4]**

| Group | | Dosage (mg/kg) | Dosage volume (mL/kg) | Number of animals (subject number) |
|---|---|---|---|---|
| G1 | Vehicle | 0 | 10 | 8(2101-2108) |
| G2 | anti-PD1 Ab | 5 | 10 | 8(2201-2208) |
| G3 | anti-PD-L1 Ab | 10 | 10 | 8(2301-2308) |
| G4 | mPD1-Fc-mIL-21(mBNS001) | 10 | 10 | 8(2401-2408) |
| G5 | mPD1-Fc-mII,-21(mBNS001) | 20 | 10 | 8(2501-2508) |
| G6 | mPD1-Fc | 10 | 10 | 8(2601-2608) |
| G7 | Fc-mIL-21 | 10 | 10 | 8(2701-2708) |
| G8 | mPD1-Fc + Fc-mIL-21 | 5 + 5 | 10 | 8(2801-2808) |

### Experimental Example 30.5. Evaluation of tumor growth inhibition effectiveness of mBNS001 fusion protein

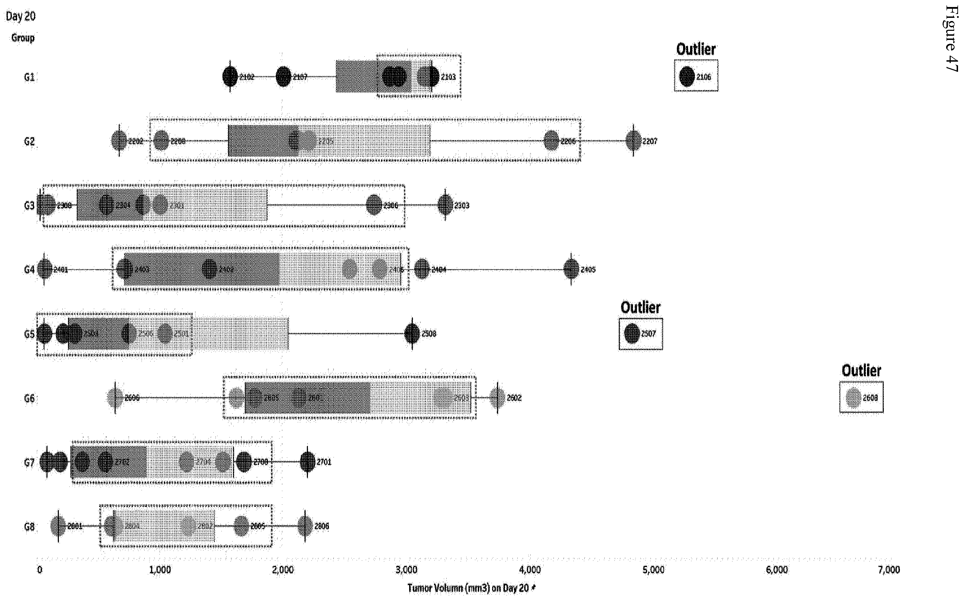

mBNS001 (mPD1-Fc-mIL-21) was administered according to the test schedule and administration method of Experimental Example 30.1. and Experimental Example 30.4. above, and the tumor growth inhibition effectiveness was evaluated. When identifying the effectiveness, in data selection, outliers on the box plot were excluded as shown in Figure 47, and data located between Q1 and Q3 were selected, or data located close to Q1 or Q3 value were selected, or data located between Min and Max were selected so that N=5 (number of subj ects).

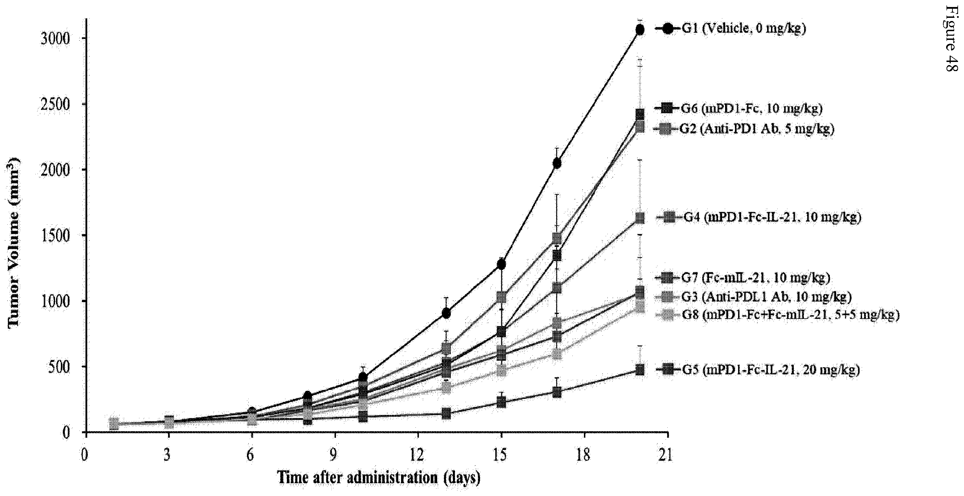

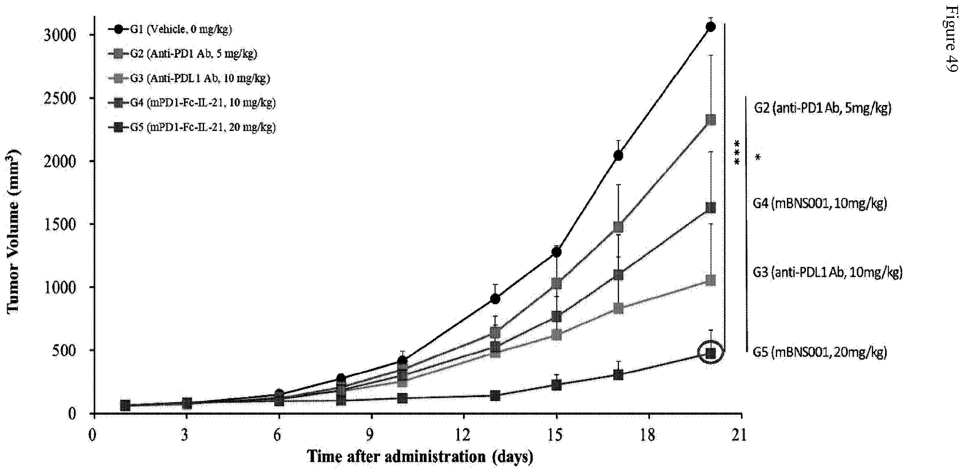

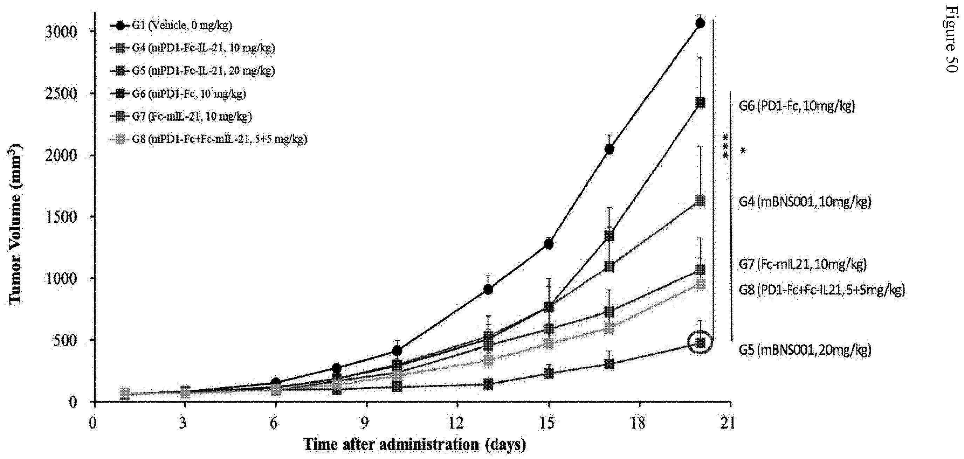

Specifically, the anti-cancer effectiveness evaluation results for colorectal cancer for N = 5 selected for each group (Table 4) are summarized in Figures 48 to 50.

### Experimental Example 31. Identification of anti-cancer effect of mBNS001 fusion protein in mice allografted with mouse-derived breast cancer cells (EMT-6)

### Experimental Example 31.1. Establishment of test schedule for evaluating effectiveness of mBNS001 fusion protein

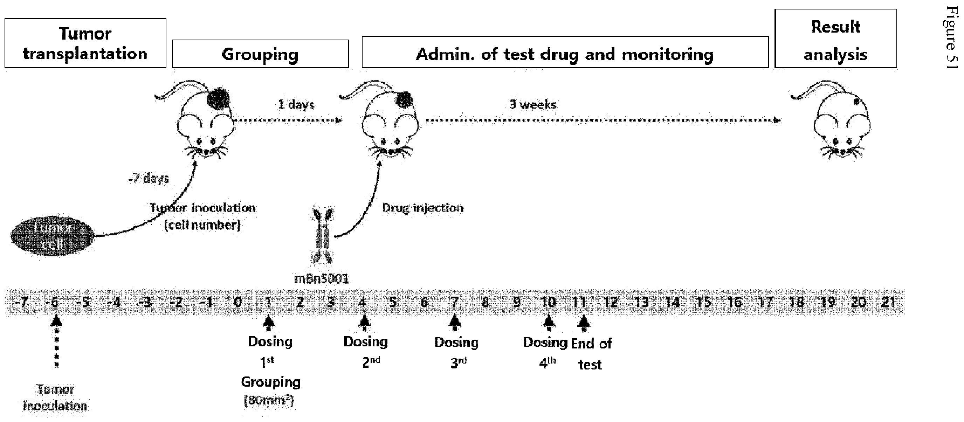

The test substances anti-PD1 Ab, anti-PD-L1 Ab, mPD1-Fc-mIL-21, mPD1-Fc, and Fc-mIL-21 were administered alone or in combination to female BALB/c mice allografted with EMT-6 cells, a BALB/c mouse-derived breast cancer cell line, a type of malignant neoplasm of mouse (*Mus musculus*) mammary gland. Thereafter, an experiment was conducted to evaluate the tumor growth inhibitory effect according to the combination treatment of the test substances (Figure 51).

### Experimental Example 31.2. Preparation of cell line

The cell line was prepared in the same manner as in Experimental Example 29.2., except that EMT-6 cells were used as cells to be used in the test.

### Experimental Example 31.3. Transplantation of cell line

Transplantation of the cell line was performed in the same manner as in Experimental Example 29.3., except that EMT-6 cell suspension was used as a cell suspension. After transplantation, general symptoms were observed once a day during the engraftment and growth period.

### Experimental Example 31.4. Administration method and frequency of administration

For intravenous administration, administration was performed a total of 4 times according to the administration day using a disposable syringe (31G, 328820, BD, U.S.A.) (Table 5).

**[Table 5]**

| Group | | Dosage (mg/kg) | Dosage volume (mL/kg) | Number of animals (subject number) |
|---|---|---|---|---|
| G1 | Vehicle | 0 | 10 | 8(2101-2108) |
| G2 | anti-PD1 Ab | 5 | 10 | 8(2201-2208) |
| G3 | anti-PD-L1 Ab | 10 | 10 | 8(2301-2308) |
| G4 | mPD1-Fc-mIL-21(mBNS001) | 10 | 10 | 8(2401-2408) |
| G5 | mPD1-Fc-mIL-21(mBNS001) | 20 | 10 | 8(2501-2508) |
| G6 | mPD1-Fc | 10 | 10 | 8(2601-2608) |
| G7 | Fc-mIL-21 | 10 | 10 | 8(2701-2708) |
| G8 | mPD1-Fc + Fc-mIL-21 | 5 + 5 | 10 | 8(2801-2808) |
| G9 | mPD1-Fc + Fc-mIL-21 | 10 + 10 | 10 | 8(2901-2908) |

### Experimental Example 31.5. Evaluation of tumor growth inhibition effectiveness of mBNS001 fusion protein

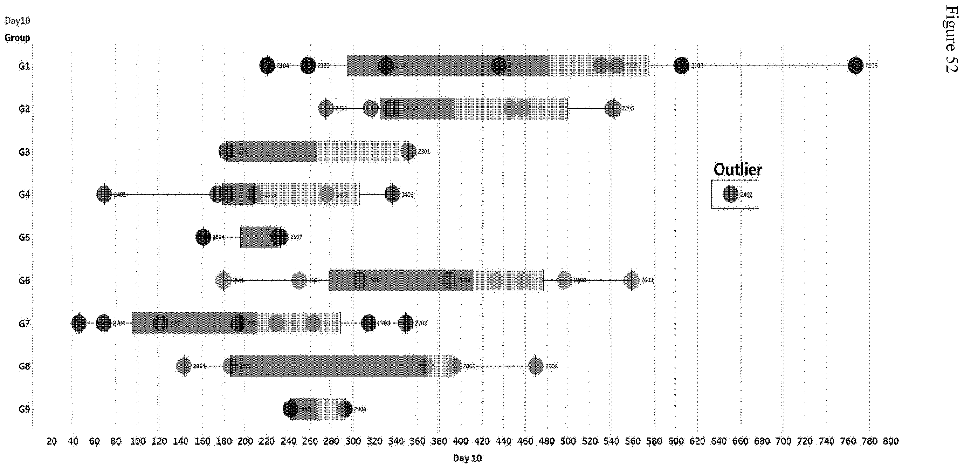

mBNS001 (mPD1-Fc-mIL-21) was administered according to the test schedule and administration method of Experimental Example 31.1. and Experimental Example 31.4. above, and the tumor growth inhibition effectiveness was evaluated. When identifying the effectiveness, in data selection, outliers on the box plot were excluded as shown in Figure 52, and data were selected and evaluated.

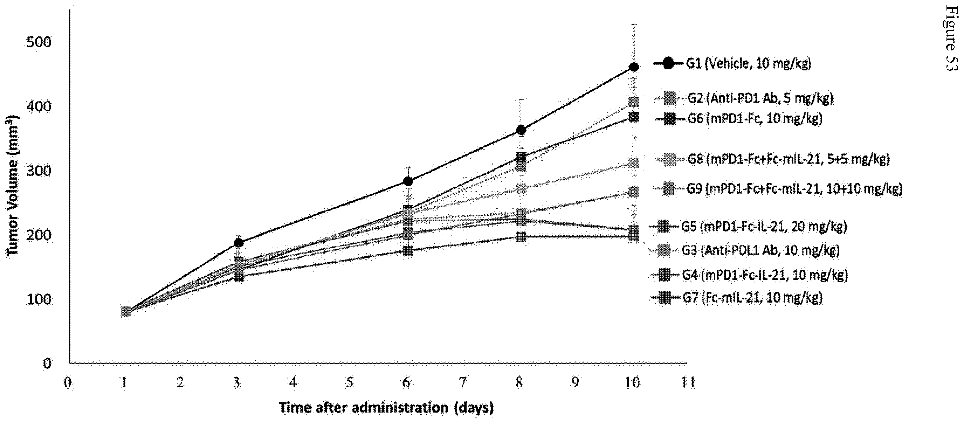

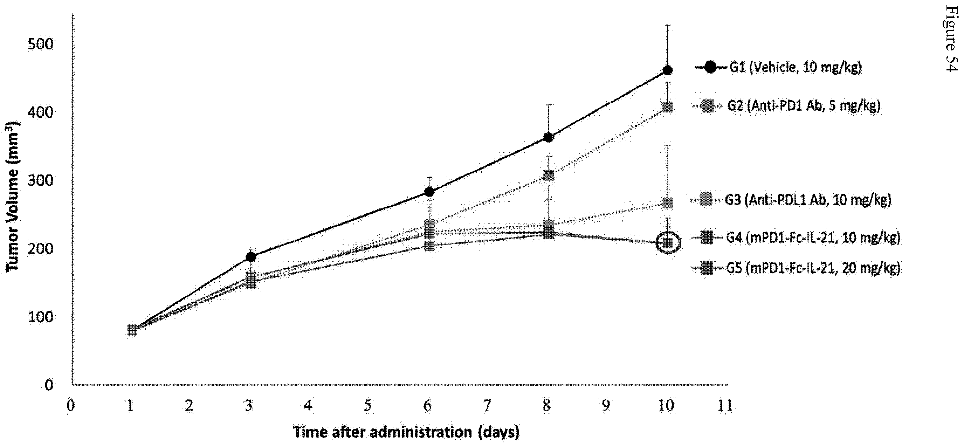

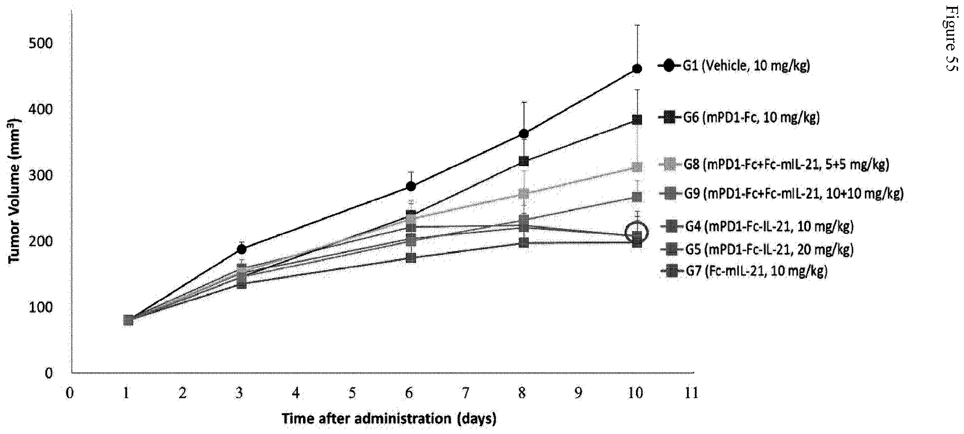

Specifically, the anti-cancer effectiveness evaluation results for breast cancer for each group (Table 5) are summarized in Figures 53 to 55.

### XI. Analysis of mechanism of action of BNS001 fusion protein

### Experimental Example 32. Identification of IFN-γ production by BNS001 fusion protein

Human PBMCs obtained from three healthy donors (donor 1, donor 2, and donor 3) were stimulated by treatment with PHA (Phytohaemagglutinin), and then the level of cytokine IFN-γ secretion induced by BNS001 treatment was identified through ELISA analysis. At this time, hIgG, atezolizumab (anti-PD-L1 (Tecentriq, Roche)), IL-21, and atezolizumab + IL-21 were used as comparative controls.

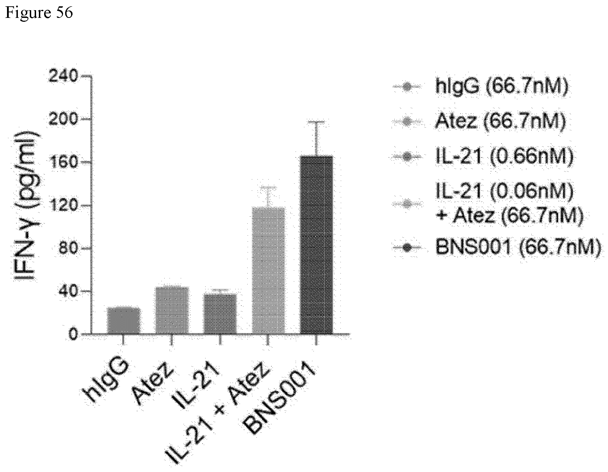

As a result, it was identified that the amount of IFN-γ secretion was statistically significantly increased in PBMCs obtained from donors treated with the BNS001 fusion protein compared to the cells treated with atezolizumab and IL-21 alone or in combination (Figure 56).

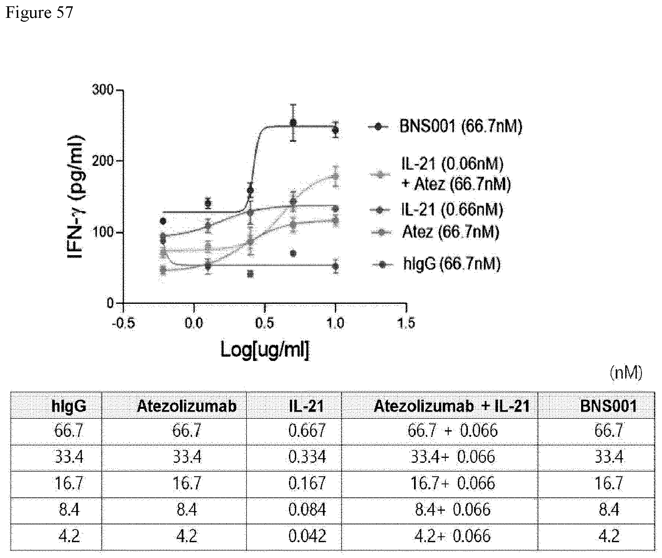

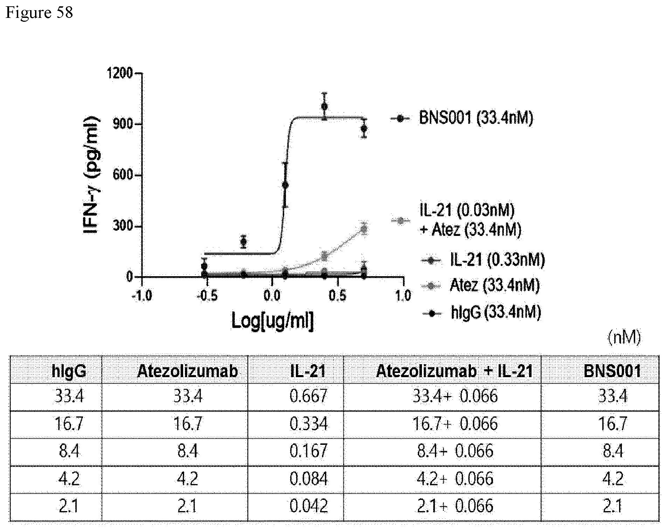

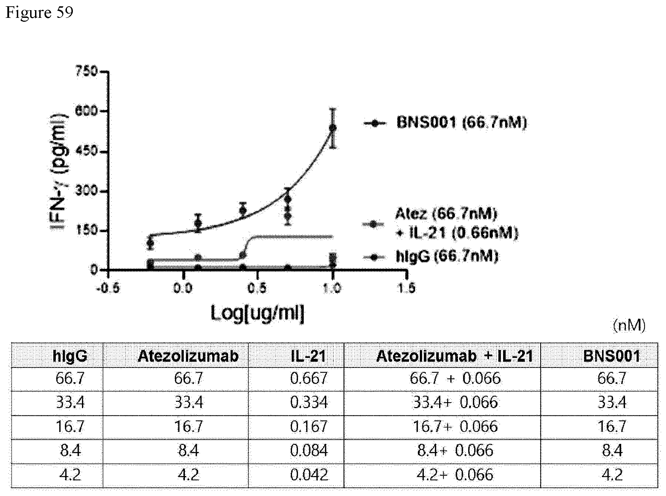

In addition, human PBMCs obtained from donor 1, donor 2, and donor 3 and stimulated with PHA were treated with BNS001, hIgG, atezolizumab, and IL-21 alone or in combination at various concentrations, and the level of IFN-γ secretion was identified. The analysis results are shown in Figures 57 to 59.

Specifically, it was identified that in PBMCs obtained from three donors, the amount of IFN-γ secretion was statistically significantly increased when treated with BNS001 compared to when treated with atezolizumab (anti-PD-L1) and IL-21 alone or in combination at various concentrations.

### Experimental Example 33. Measurement of changes in activity of immune cell populations in PBMC by BNS001 fusion protein

Human PBMCs obtained from three healthy donors were stimulated by treatment with PHA, and then changes in the activity of CD4+ T cell, CD8+ T cell, NK cell, and B cell populations induced by BNS001 treatment were identified. For changes in the activity of immune cell populations, the level of expression of immune activation markers such as CD69, CD38, 4-1BB, 2B4, and OX40 was identified through multi-color flow cytometry. At this time, hIgG, atezolizumab (anti-PD-L1 (Tecentriq, Roche)), IL-21, and atezolizumab + IL-21 were used as comparative controls.

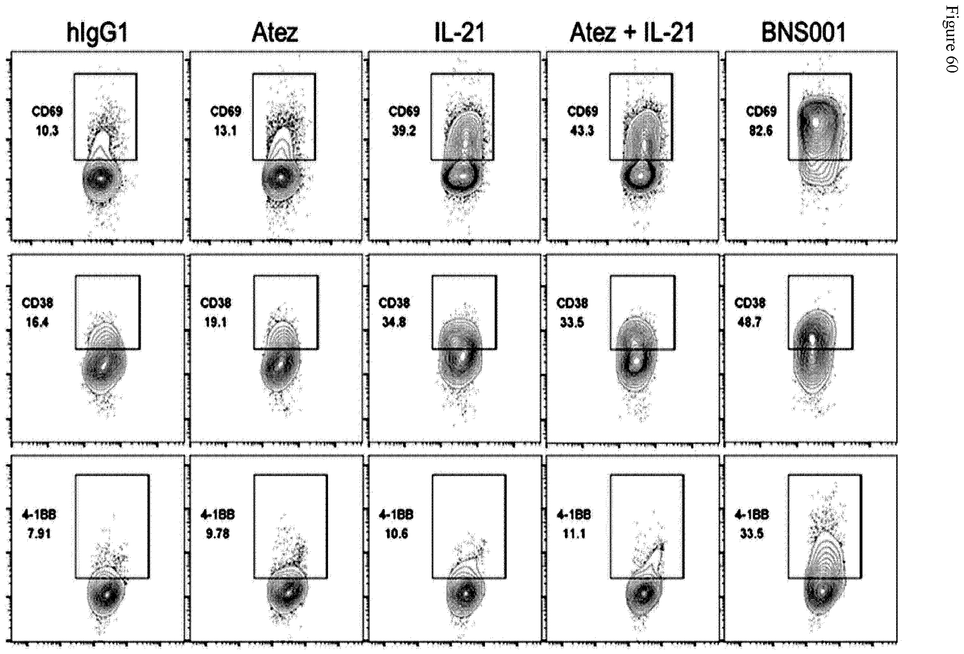

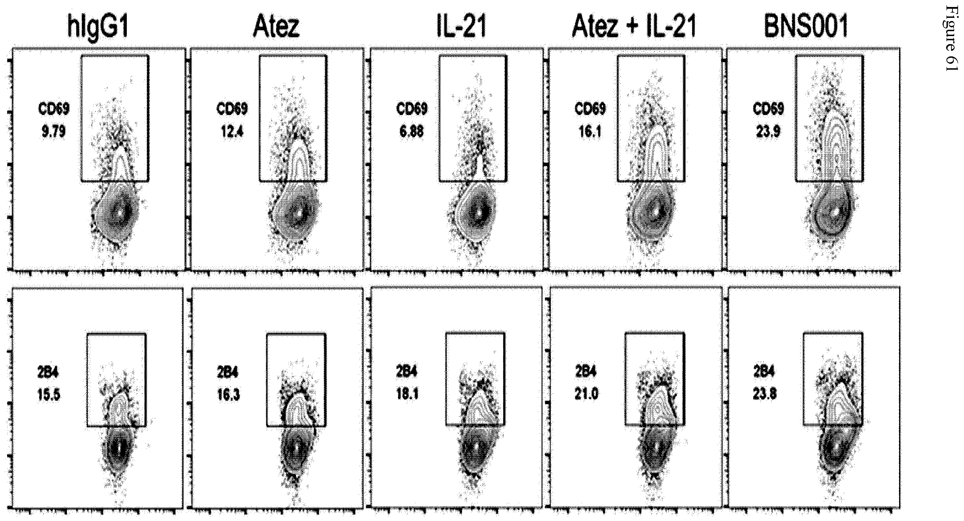

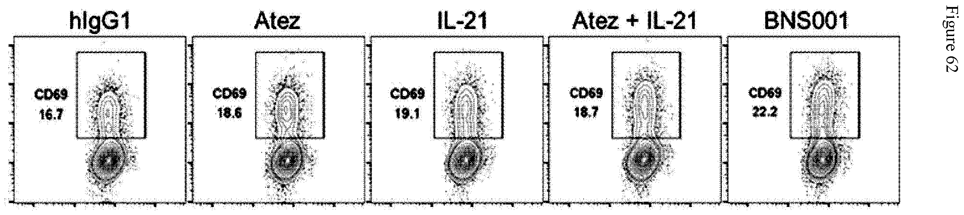

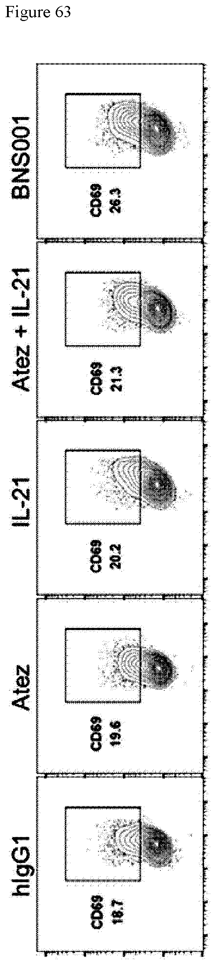

As a result, it was identified that activation of NK cells, CD8+ T cells, CD4+ T cells, and B cells in PBMCs obtained from donors treated with the BNS001 fusion protein was induced. In particular, it was identified that activation of NK cells was strongly induced (Figures 60 to 63).

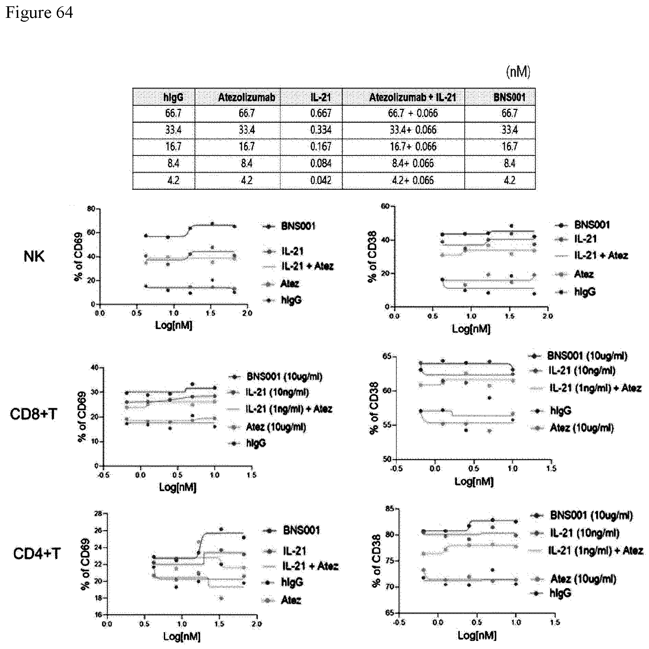

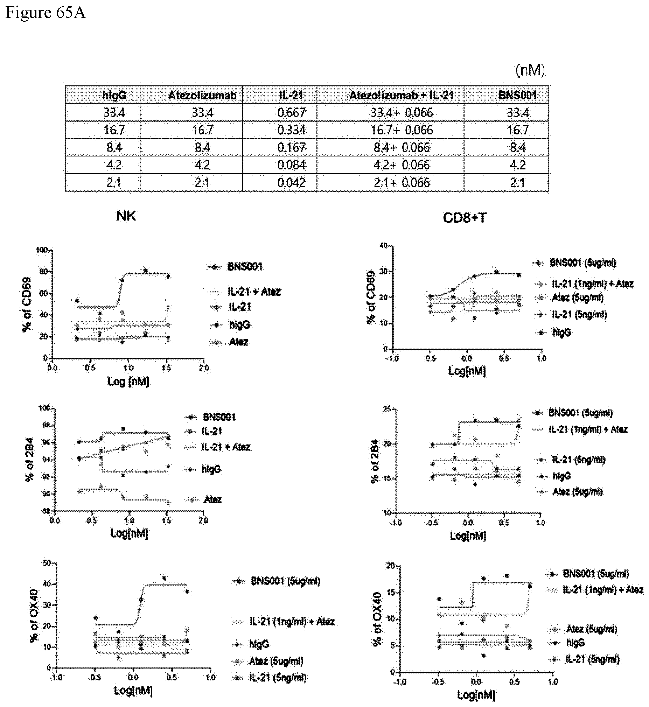

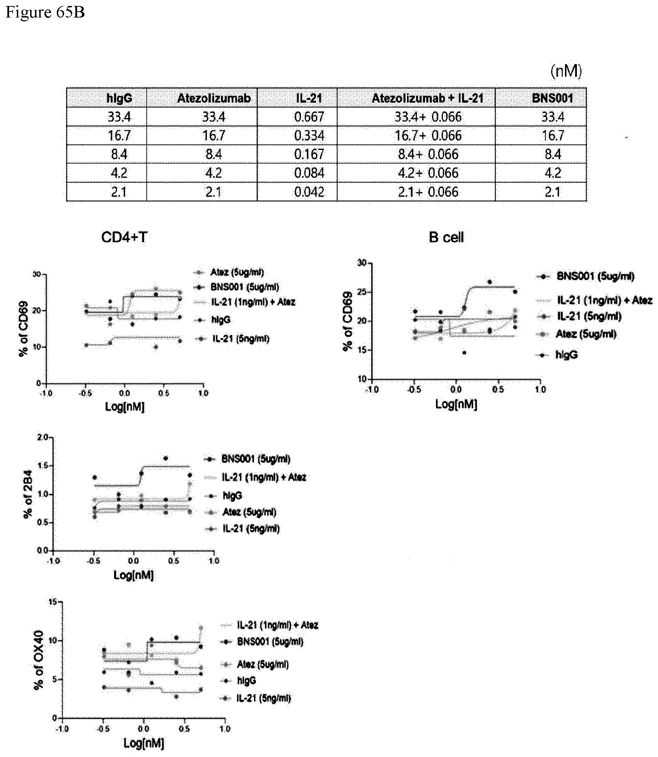

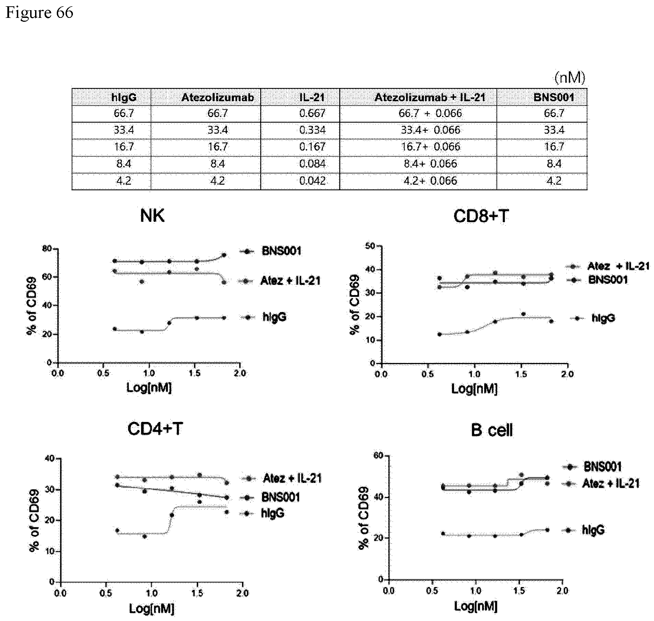

In addition, human PBMCs obtained from donor 1, donor 2, and donor 3 and stimulated with PHA were treated with BNS001, hIgG, atezolizumab, and IL-21 alone or in combination at various concentrations, and changes in the activity of immune cell populations in PBMCs was measured. The analysis results are shown in Figures 64 to 66.

Specifically, it was identified that activation of NK cells, CD8+ T cells, CD4+ T cells, and B cells in PBMCs was induced obtained from three donors when treated with BNS001 compared to when treated with atezolizumab (anti-PD-L1) and IL-21 alone or in combination at various concentrations. In particular, it was identified that activation of NK cells among the immune cell populations was strongly induced.

### Experimental Example 34. Measurement of immunoreactivity of CD8+ T cells by BNS001 fusion protein

Human PBMC stimulation assay was performed to measure the immunoreactivity of CD8+ T cells. The immunoreactivity of CD8+ T cells was identified by measuring the proliferation using CellTrace Violet (CTV) staining. At this time, hIgG, atezolizumab (anti-PD-L1 (Tecentriq, Roche)), IL-21, and atezolizumab + IL-21 were used as comparative controls.

Specifically, human PBMC frozen vials were thawed and then rested in an incubator at 37°C for 4 hours. The rested human PBMCs were stained with CellTrace Violet (CTV). CTV-stained PBMC were seeded at 1×10⁵/well in a 96 well round bottom plate. PHA-P was diluted in RPMI complete medium to a final concentration of 1.5 µg/ml and added to a 96 well round bottom plate. The reagents were serially diluted for each condition in RPMI complete medium and added to a 96 well round bottom plate. After 3 days, the proliferation of CD8+ T cells was analyzed by FACS based on CTV staining.

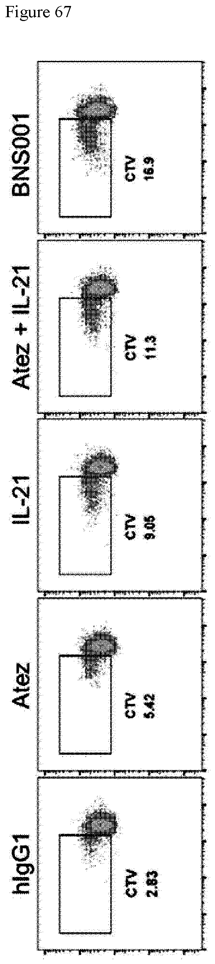

As a result, it was identified that the proliferation of CD8+ T cells was activated in PBMCs treated with the BNS001 fusion protein compared to the cells treated with atezolizumab and IL-21 alone or in combination (Figure 67).

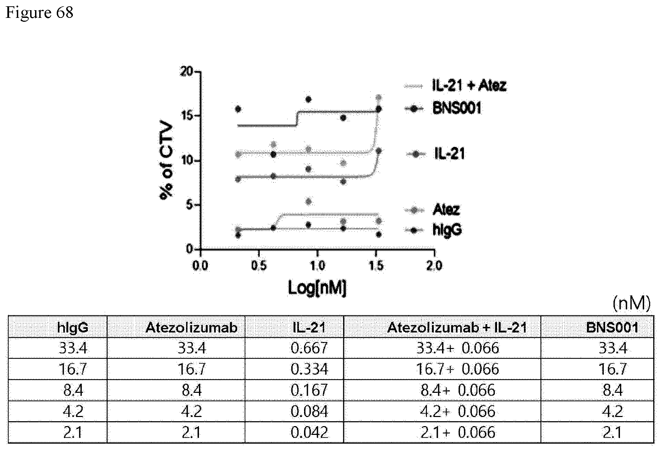

Specifically, it was identified that the proliferation of CD8+ T cells was activated in PBMCs treated with BNS001 compared to the cells treated with atezolizumab (anti-PD-L1) and IL-21 alone or in combination at various concentrations (Figure 68).

### XI. Evaluation of anti-cancer efficacy of BNS001 fusion protein on humanized mouse tumor model

### Experimental Example 35. Identification of anti-cancer effect of BNS001 fusion protein in humanized mice transplanted with human-derived colorectal cancer cells (HCT116) and human peripheral blood mononuclear cells

### Experimental Example 35.1. Identification of anti-cancer effect of BNS001 fusion protein by dosage

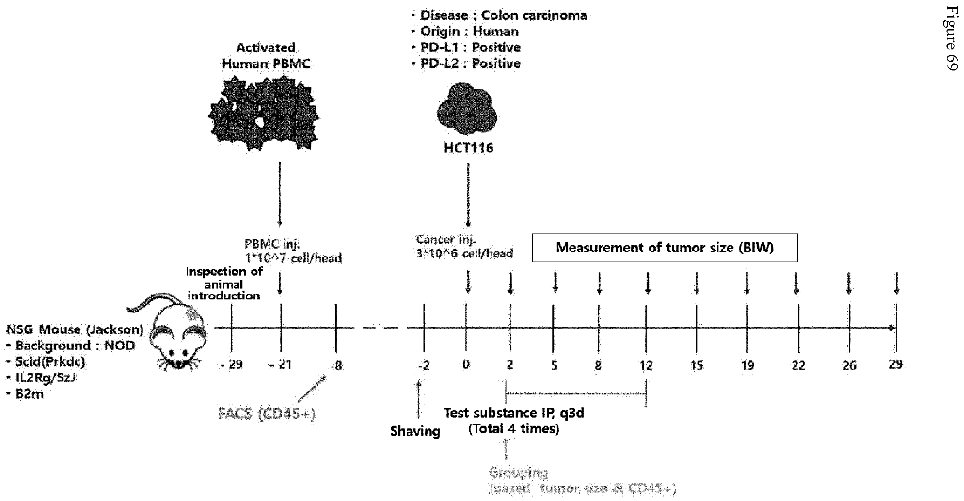

The cell line was prepared in the same manner as in Experimental Example 28., except that HCT116 cell line, a colon carcinoma cell line, was used as a frozen human tumor cell line. In addition, peripheral blood mononuclear cells (PBMC) isolated from humans were actively cultured for one week as in Experimental Example 18.1. After checking the viability using a microscope, the cell lines were prepared by diluting them in medium to a concentration of 2.5×10⁸ cells/mL. The used mouse was a 6-week-old male NSG mouse (NOD.Cg-B2mtm1UncPrkdcscidIl2rgtm1Wjl/SzJ) (ORIENT BIO INC.), and for construction of a humanized mouse, human peripheral blood mononuclear cells were administered subcutaneously at a dosage volume of 1×10⁷ cells (hPBMC)/0.1 mL/head into the tail vein. Humanized mice were identified by measuring the expression of hCD45+ in the blood through FACS analysis 14 days after administration of human peripheral blood mononuclear cells. After identifying the humanized mouse, the cell line HCT116 was transplanted and administered subcutaneously at a dosage volume of 3 × 10⁶ cells. Two days after transplanting the cancer cells, the mice were anesthetized with an inhalation anesthetic (2% isoflurane, Hana Pharm Co., Ltd.), and then the BNS001 fusion protein (PD-1 D-Fc-IL-21) was administered intraperitoneally at a dose of 5, 10, or 20 mg/kg. After the first administration, a total of 4 administrations were performed once every 3 to 4 days. At this time, saline (JW Pharmaceutical) was administered as a negative control. In order to identify the anti-cancer effect, the tumor size was measured twice a week (Figure 69).

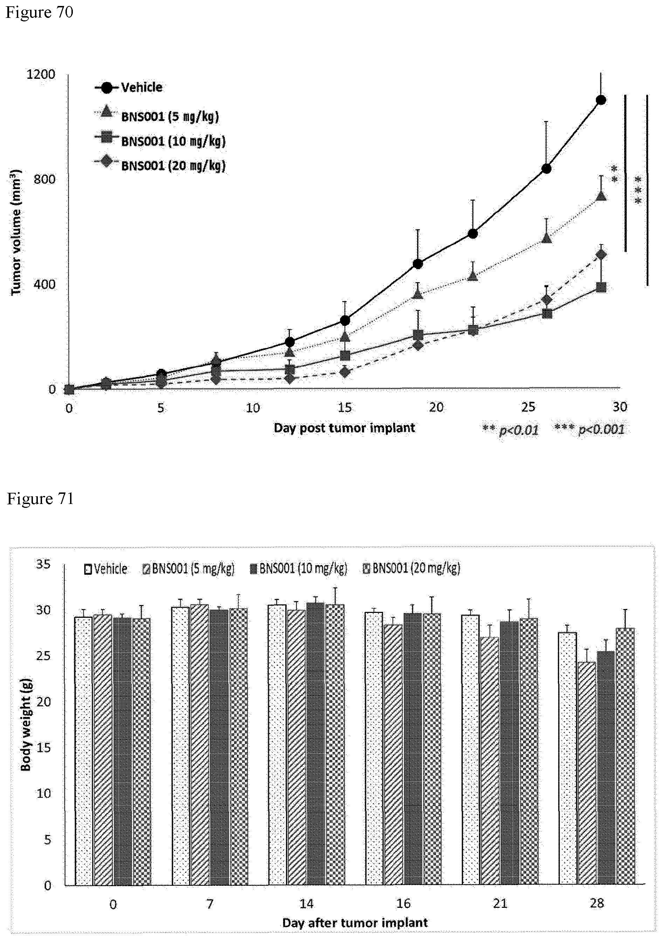

As a result, it was identified that in animal experiments of humanized mice transplanted with HCT116 cancer cell line treated with the BNS001 fusion protein, the tumor size was statistically significantly reduced in a concentration-dependent manner compared to the negative control (vehicle treatment). In particular, the anti-cancer effect was the best when administered at a dose of 10 mg/kg (Figure 70).

Meanwhile, it was identified that there was no effect on body weight change by treatment with the BNS001 fusion protein (Figure 71).

### Experimental Example 35.2. Identification of anti-cancer effect between BNS001 fusion protein and control drug

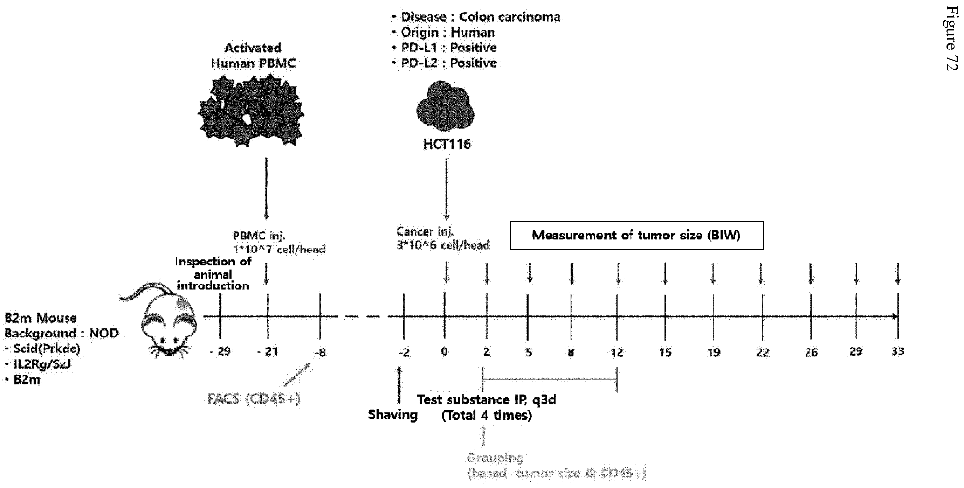

Humanized mice were produced by preparing HCT116 cell line and PBMC in the same manner as in Experimental Example 35.1. Two days after transplanting the humanized mouse with HCT116 cancer cells, the mice were anesthetized with an inhalation anesthetic (2% isoflurane, Hana Pharm Co., Ltd.). Thereafter, the BNS001 fusion protein (PD-1 D-Fc-IL-21) was administered intraperitoneally at a dose of 10 mg/kg. After the first administration, a total of 4 administrations were performed once every 3 to 4 days. At this time, saline (JW Pharmaceutical) was administered as a negative control, and the BNS001D (PD-1 D-Fc) fusion protein and the BNS001I (Fc-IL-21) fusion protein were administered alone or in combination as comparative controls. In addition, the commercially available antibodies atezolizumab (anti-PD-L1 (Tecentriq, Roche)), avelumab (anti-PD-L1 (Bavencio, Merck)), and pembrolizumab (anti-PD-1 (Keytruda, MSD)) were administered as positive controls. In order to identify the anti-cancer effect of the administered substance, the tumor size and body weight were measured twice a week (Figure 72).

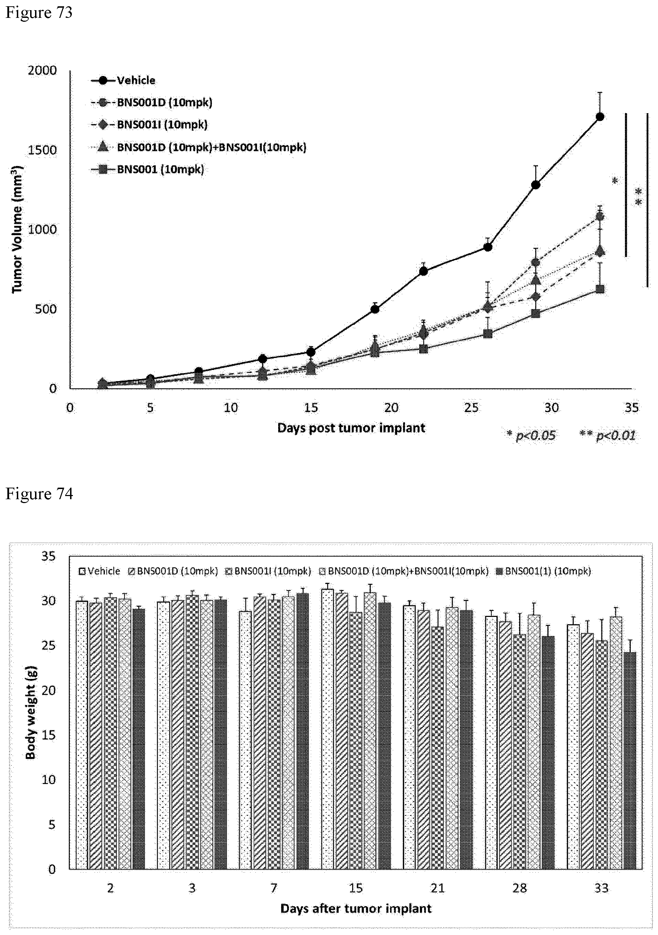

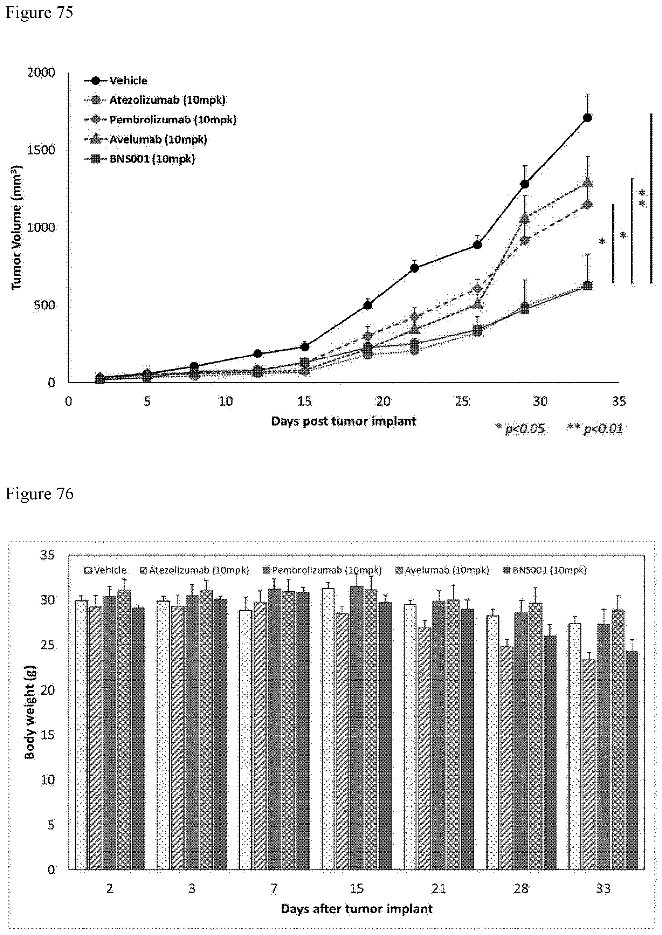

As a result, it was identified that in animal experiments of humanized mice transplanted with HCT116 cancer cell line treated with the BNS001 fusion protein (PD-1 D-Fc-IL-21), the tumor size was statistically significantly reduced compared to the negative control (Figures 73 and 75).

It was identified that the tumor size was reduced in the group administered with the BNS001 fusion protein compared to the group administered with BNS001D (PD-1 D-Fc) and BNS001I (Fc-IL-21) as a comparative control. In addition, it was identified that the tumor size was reduced in the group administered with the BNS001 fusion protein compared to the group administered with BNS001D (PD-1 D-Fc)+BNS001I (Fc-IL-21) in combination (Figure 73).

In addition, it was identified that the tumor size was statistically significantly reduced in the group administered with the BNS001 fusion protein compared to the group administered with the commercially available antibodies pembrolizumab (anti-PD-1 (Keytruda, MSD)) and avelumab (anti-PD-L1 (Bavencio, Merck)) used as the positive control. It was identified that the tumor size was reduced at an equivalent level in the group administered with the BNS001 fusion protein compared to the group administered with atezolizumab (anti-PD-L1 (Tecentriq, Roche)) (Figure 75).

Meanwhile, it was identified that there was no effect on body weight change by treatment with the BNS001 fusion protein (Figures 74 and 76).

### Experimental Example 36. Identification of anti-cancer effect between BNS001 fusion protein and control drug in humanized mice transplanted with human-derived lung cancer cells (A549) and human peripheral blood mononuclear cells

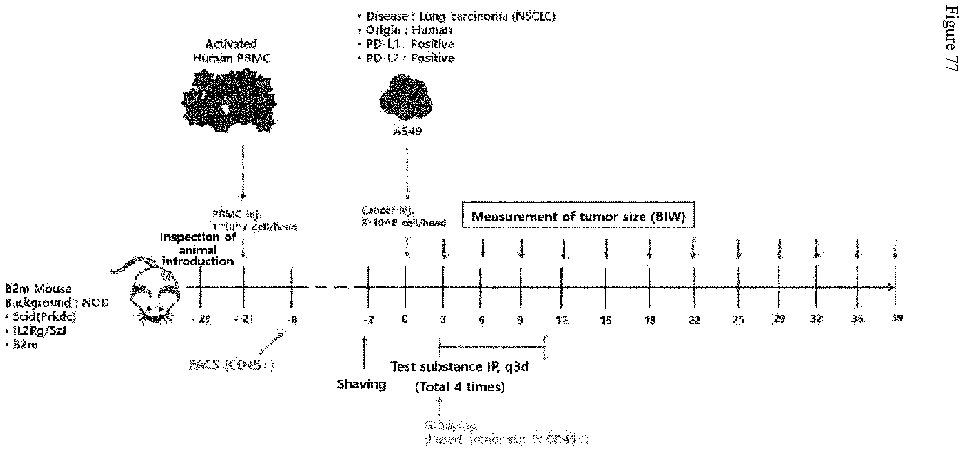

Humanized mice were produced by preparing A549 cell line and PBMC in the same manner as in Experimental Example 35.1., except that A549 cell line, a lung carcinoma (NSCLC) cell line, was used as a frozen human tumor cell line. Two days after transplanting the humanized mouse with A549 cancer cells, the mice were anesthetized with an inhalation anesthetic (2% isoflurane, Hana Pharm Co., Ltd.). Thereafter, the BNS001 fusion protein (PD-1 D-Fc-IL-21) was administered intraperitoneally at a dose of 10 mg/kg. After the first administration, a total of 4 administrations were performed once every 3 to 4 days. At this time, saline (JW Pharmaceutical) was administered as a negative control, and the commercially available antibodies atezolizumab (anti-PD-L1 (Tecentriq, Roche)), avelumab (anti-PD-L1 (Bavencio, Merck)), and pembrolizumab (anti-PD-1 (Keytruda, MSD)) were administered as positive controls. In order to identify the anti-cancer effect of the administered substance, the tumor size and body weight were measured twice a week (Figure 77).

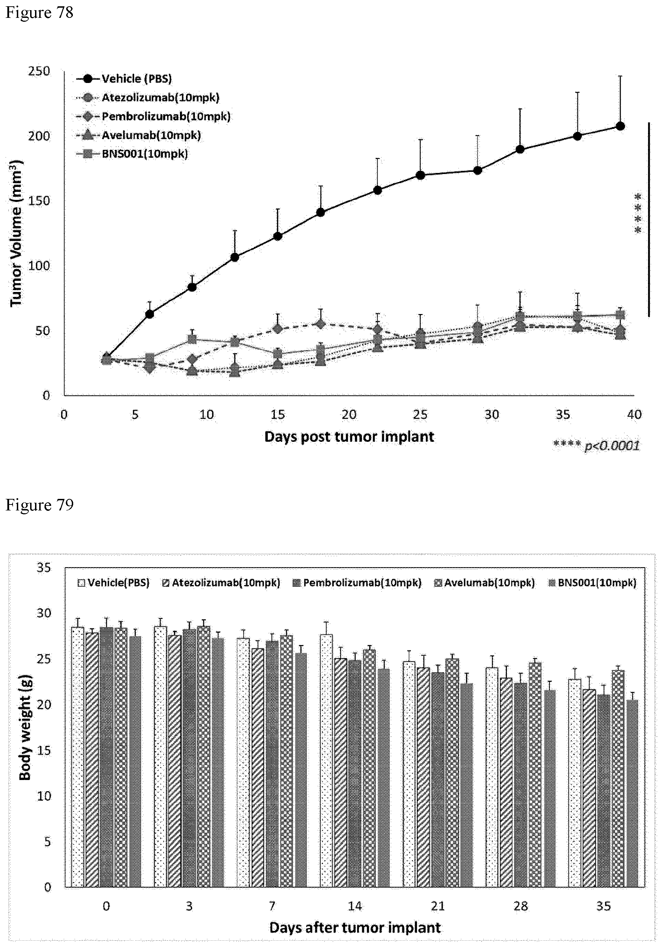

As a result, it was identified that in animal experiments of humanized mice transplanted with A549 cancer cell line treated with the BNS001 fusion protein (PD-1 D-Fc-IL-21), the tumor size was statistically significantly reduced compared to the negative control. In addition, it was identified that the tumor size was reduced at an equivalent level in the group administered with the BNS001 fusion protein compared to the group administered with the commercially available antibodies atezolizumab (anti-PD-L1 (Tecentriq, Roche)), pembrolizumab (anti-PD-1 (Keytruda, MSD)), and avelumab (anti-PD-L1 (Bavencio, Merck)) used as positive controls (Figure 78).

Meanwhile, it was identified that there was no effect on body weight change by treatment with the BNS001 fusion protein (Figure 79).

### Experimental Example 37. Identification of anti-cancer effect between BNS001 fusion protein and control drug in humanized mice transplanted with human-derived breast cancer cells (MDA-MB-231) and human peripheral blood mononuclear cells

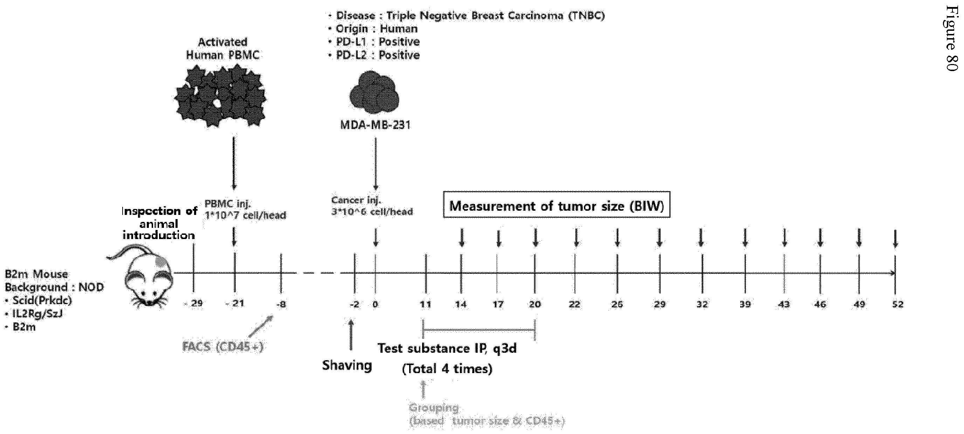

Humanized mice were produced by preparing MDA-MB-231 cell line and PBMC in the same manner as in Experimental Example 35.1., except that MDA-MB-231 cell line, a breast carcinoma (TNBC) cell line, was used as a frozen human tumor cell line. Two days after transplanting the humanized mouse with MDA-MB-231 cancer cells, the mice were anesthetized with an inhalation anesthetic (2% isoflurane, Hana Pharm Co., Ltd.). Thereafter, the BNS001 fusion protein (PD-1 D-Fc-IL-21) was administered intraperitoneally at a dose of 10 mg/kg. After the first administration, a total of 4 administrations were performed once every 3 to 4 days. At this time, saline (JW Pharmaceutical) was administered as a negative control, and the commercially available antibodies atezolizumab (anti-PD-L1 (Tecentriq, Roche)), avelumab (anti-PD-L1 (Bavencio, Merck)), and pembrolizumab (anti-PD-1 (Keytruda, MSD)) were administered as positive controls. In order to identify the anti-cancer effect of the administered substance, the tumor size and body weight were measured twice a week (Figure 80).

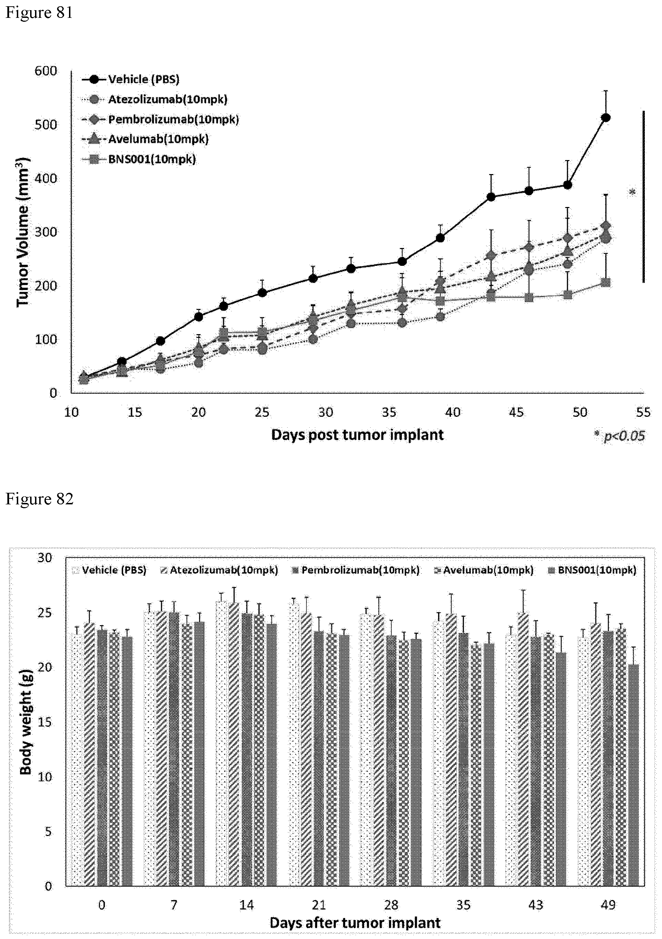

As a result, it was identified that in animal experiments of humanized mice transplanted with MDA-MB-231 cancer cell line treated with the BNS001 fusion protein (PD-1 D-Fc-IL-21), the tumor size was statistically significantly reduced compared to the negative control. In addition, it was identified that the tumor size was reduced in the group administered with the BNS001 fusion protein compared to the group administered with the commercially available antibodies atezolizumab (anti-PD-L1 (Tecentriq, Roche)), pembrolizumab (anti-PD-1 (Keytruda, MSD)), and avelumab (anti-PD-L1 (Bavencio, Merck)) used as the positive control (Figure 81).

Meanwhile, it was identified that there was no effect on body weight change by treatment with the BNS001 fusion protein (Figure 82).

### Experimental Example 38. Identification of anti-cancer effect between BNS001 fusion protein and control drug in humanized mice transplanted with human-derived lung cancer cells (NCI-H1975) and human peripheral blood mononuclear cells

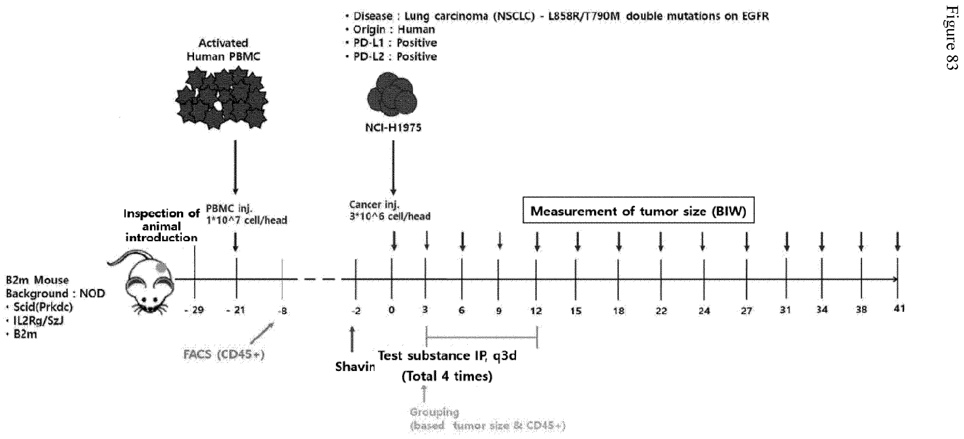

Humanized mice were produced by preparing NCI-H1975 cell line and PBMC in the same manner as in Experimental Example 35.1., except that NCI-H1975 (L858R/T790M double mutations on EGFR) cell line, a lung carcinoma (NSCLC) cell line, was used as a frozen human tumor cell line. Two days after transplanting the humanized mouse with NCI-H1975 cancer cells, the mice were anesthetized with an inhalation anesthetic (2% isoflurane, Hana Pharm Co., Ltd.). Thereafter, the BNS001 fusion protein (PD-1 D-Fc-IL-21) was administered intraperitoneally at a dose of 10 mg/kg. After the first administration, a total of 4 administrations were performed once every 3 to 4 days. At this time, saline (JW Pharmaceutical) was administered as a negative control, and the commercially available antibodies bevacizumab (anti-VEGF (Avastin, Genentech)), ramucirumab (anti-VEGFR2 (Cyramza, Lilly)), cetuximab (anti-EGFR (Erbitux, BMS)) and amivantamab (anti-EGFR/anti-MET (Rybrevant, Jassen)) were administered as positive controls. In order to identify the anti-cancer effect of the administered substance, the tumor size and body weight were measured twice a week (Figure 83).

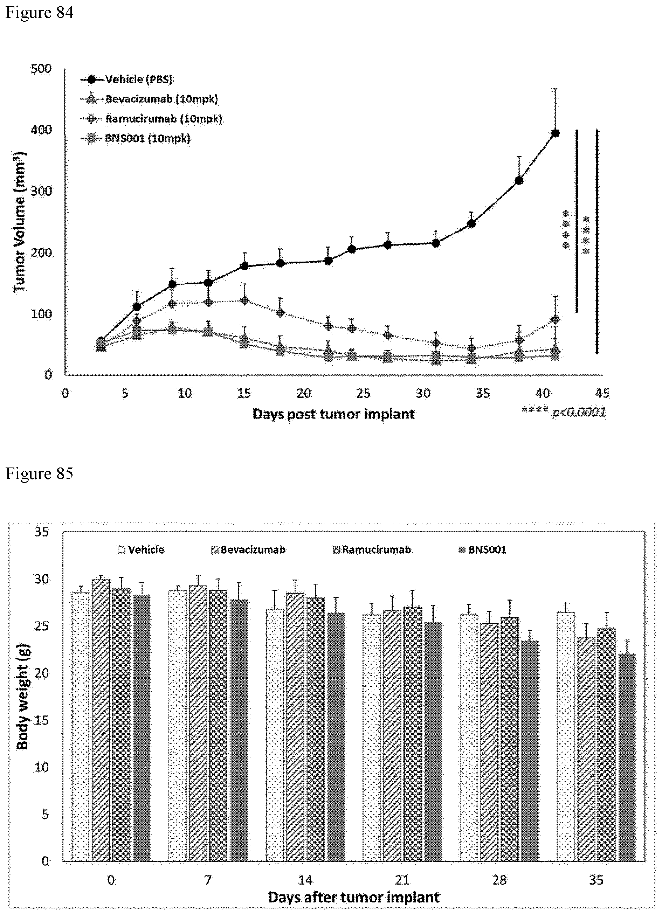

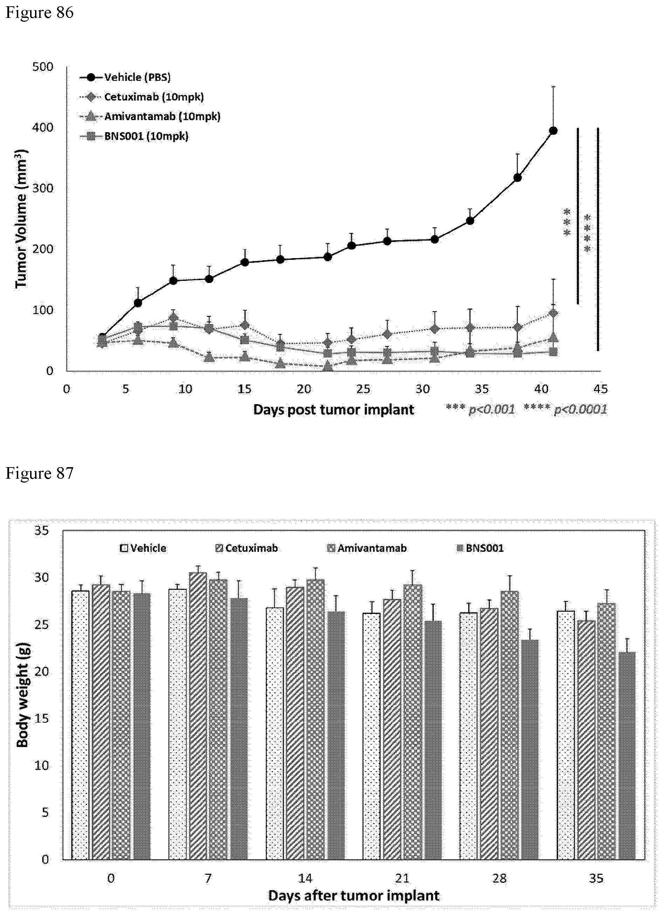

As a result, it was identified that in animal experiments of humanized mice transplanted with NCI-H1975 cancer cell line treated with the BNS001 fusion protein (PD-1 D-Fc-IL-21), the tumor size was statistically significantly reduced compared to the negative control (Figures 84 and 86).

In addition, it was identified that the tumor size was reduced in the group administered with the BNS001 fusion protein compared to the group administered with the commercially available antibodies ramucirumab (anti-VEGFR2 (Cyramza, Lilly)) and cetuximab (anti-EGFR (Erbitux, BMS)) used as the positive control. In addition, it was identified that the tumor size was reduced at an equivalent level in the group administered with the BNS001 fusion protein compared to the group administered with bevacizumab (anti-VEGF (Avastin, Genentech)) and amivantamab (anti-EGFR/anti-MET (Rybrevant, Jassen)) (Figures 84 and 86).

Meanwhile, it was identified that there was no effect on body weight change by treatment with the BNS001 fusion protein (Figures 85 and 87).

## Claims

1. A fusion protein comprising a PD-1 protein and an IL-21 protein.

2. The fusion protein according to claim 1, wherein the PD-1 protein and the IL-21 protein are linked via a linker.

3. The fusion protein according to claim 1, wherein the PD-1 protein has the amino acid sequence of SEQ ID NO: 2, 21, 34, or 103.

4. The fusion protein according to claim 1, wherein the IL-21 protein has the amino acid sequence of SEQ ID NO: 6 or 22.

5. The fusion protein according to claim 2, wherein the linker is an Fc domain of an immunoglobulin.

6. The fusion protein according to claim 5, wherein the Fc domain is wild type Fc domain or variant thereof.

7. The fusion protein according to claim 6, wherein the wild type Fc domain has the amino acid sequence of SEQ ID NO: 14.

8. The fusion protein according to claim 6, wherein the variant of the Fc domain has a substitution of Q81R, M198L, L79G, T136W, Q81R/M198L, L79G/M198L or Q81R/T136W/M198L in the amino acid sequence of SEQ ID NO: 14.

9. The fusion protein according to claim 8, wherein the variant of the Fc domain has the amino acid sequence of SEQ ID NO: 4, 10, 50, 87, 95, 99, 104, 108, 112, or 116.

10. The fusion protein according to claim 6, wherein the variant of the Fc domain has an increased half-life compared to the wild type Fc domain.

11. The fusion protein according to claim 1, wherein the fusion protein consists of the following structural formula I or II:
N'-A-[Li]n-Fc domain-[L₂]m-B-C' I
N'-B-[L₁]n-Fc domain-[L₂]m-A-C' II
wherein, in the structural formulas I and II,
N' is the N-terminus of the fusion protein,
C' is the C-terminus of the fusion protein,
A is the PD-1 protein or a fragment thereof,
B is the IL-21 protein or a variant thereof,
L₁ and L₂ are peptide linkers, and
n and m are each independently 0 or 1.

12. The fusion protein according to claim 11, wherein Li is a peptide linker consisting of the amino acid sequence of SEQ ID NO: 3 or 38.

13. The fusion protein according to claim 11, wherein L₂ is a peptide linker consisting of the amino acid sequence of SEQ ID NO: 5, 39, or 57.

14. The fusion protein according to claim 11, wherein the fusion protein consists of the structural formula I.

15. The fusion protein according to claim 1, wherein the fusion protein has 85% or more sequence identity to the amino acid sequence of SEQ ID NO: 8, 12, 16, 24, 27, 32, 36, 41, 44, 52, 55, 76, 79, 82, 89, 92, 97, 101, 106, 110, 114, or 118.

16. A fusion protein dimer in which the two fusion proteins according to any one of claims 1 to 15 are attached to each other.

17. The fusion protein dimer according to claim 16, wherein the fusion protein dimer is a homodimer.

18. A polynucleotide encoding the fusion protein according to any one of claims 1 to 15.

19. The polynucleotide according to claim 18, wherein the polynucleotide has 85% or more sequence identity to the nucleotide sequence of SEQ ID NO: 9, 13, 17, 25, 28, 33, 37, 42, 45, 53, 56, 77, 80, 83, 90, 93, 98, 102, 107, 111, 115, or 119.

20. A vector comprising the polynucleotide according to claim 18.

21. A host cell transformed with the vector according to claim 20.

22. A pharmaceutical composition for preventing or treating cancer, comprising the fusion protein according to any one of claims 1 to 15; or the fusion protein dimer according to claim 16 or 17 as an active ingredient.

23. The pharmaceutical composition according to claim 22, wherein the pharmaceutical composition further comprises a pharmaceutically acceptable carrier.

24. The pharmaceutical composition according to claim 23, wherein the cancer is any one selected from the group consisting of colorectal cancer, melanoma, gastric cancer, liver cancer, lung cancer, breast cancer, prostate cancer, ovarian cancer, pancreatic cancer, cervical cancer, bladder cancer, kidney cancer, gallbladder cancer, thyroid cancer, laryngeal cancer, acute myeloid leukemia, brain tumor, neuroblastoma, retinoblastoma, head and neck cancer, salivary gland cancer, and lymphoma.

25. A use of the fusion protein dimer according to claim 16 or 17 for manufacture of a medicament for preventing or treating cancer.

26. A use of the fusion protein dimer according to claim 16 or 17 for preventing or treating cancer.

27. A method for preventing or treating cancer, comprising administering the fusion protein dimer according to claim 16 or 17 to a subject.
